Europäisches Patentamt

European Patent Office

Office européen des brevets

(19)

(11) **EP 0 475 842 B1**

(12) **FASCICULE DE BREVET EUROPEEN**

(45) Date de publication et mention
de la délivrance du brevet:
**11.02.1998 Bulletin 1998/07**

(51) Int Cl.⁶: **C12N 15/80**, C12N 1/15,
C12N 9/58

(21) Numéro de dépôt: **91402419.5**

(22) Date de dépôt: **11.09.1991**

(54) **Cassette d'expression d'un précurseur de l'endothiapepsine dans Cryphonectria parasitica**

Expressionskassette eines Vorläufers von Endothiapepsine in Cryphonectria parasitica

Expression cassette of a precursor of endothiapepsin in Cryhonectria parasitica

(84) Etats contractants désignés:
**AT BE CH DE DK ES FR GB GR IT LI LU NL SE**

(30) Priorité: **11.09.1990 FR 9011230**

(43) Date de publication de la demande:
**18.03.1992 Bulletin 1992/12**

(83) **Déclaration conformément à la règle 28(4) CBE
(solution de l'expert)**

(73) Titulaire: **SYSTEMS BIO-INDUSTRIES
92641 Boulogne-Billancourt Cédex (FR)**

(72) Inventeurs:
- **Jara, Patrick
  F-31320 Castanet-Tolosan (FR)**
- **Legoux, Richard
  F-31460 Caraman (FR)**
- **Loison, Gérard
  F-31300 Toulouse (FR)**
- **Razanamparany, Voahangy
  F-31400 Toulouse (FR)**

(74) Mandataire: **Gillard, Marie-Louise et al
Cabinet Beau de Loménie
158, rue de l'Université
75340 Paris Cédex 07 (FR)**

(56) Documents cités:
**WO-A-89/01969**          **US-A- 3 275 453**

- **PHYTOPATHOLOGY vol. 80, no. 10, 1990, page
  1062; G.H. CHOI ET AL.: "Identification and
  characterization of genomic clones encoding
  endothiapepsin of Cryphonectria parasitica"
  ANNUAL MEETING OF THE AMERICAN
  PHYTOPATHOLOGY SOCIETY AND THE
  CANADIAN PHYTOPATHOLOGY SOCIETY,
  Grands Rapids, Michigan, 4-8 aoUt 1990**
- **GENE vol. 69, 1988, AMSTERDAM NL pages
  49-57; P.J.PUNT ET AL.: "Isolation and
  characterization of the
  glyceraldehyde-3-phosphate dehydrogenase
  gene of Aspergillus nidulans"**
- **CURRENT GENETICS vol. 17, no. 1, Janvier 1990,
  pages 25-31; A.C.L. CHURCHILL ET AL.:
  "Transformation of the fungal pathogen
  Cryphonectria parasitica with a variety of
  heterologous plasmids"**
- **CELL. vol. 52, 12 Février 1988, CAMBRIDGE, NA,
  US, pages 303-305; L. GUARENTE: "UASs and
  Enhancers: Common mechanism of
  transcriptional activation in yeast and
  mammals"**
- **MOL. GEN. GENET. vol. 221, Avril 1990, BERLIN,
  pages 148-154; G.D. FREDERICK ET J.A.
  KINSEY: "Nucleotide sequence and nuclear
  protein binding of the two regulatory sequences
  upstream of the am (GDH) gene in Neurospora"**
- **EUR. J. BIOCHEM. vol. 167, no. 2, Septembre
  1987, pages 327-338; V. BARKHOLT: "Amino
  acid sequence of endothiapepsin"**

**Description**

La présente invention concerne une nouvelle cassette d'expression d'un précurseur de l'endothiapepsine dans Cryphonectria parasitica, une souche de cette espèce transformée par cette cassette, un procédé pour préparer de l'endothiapepsine à l'aide de cette souche, ainsi qu'un procédé pour préparer une telle souche dépourvue de marqueur de sélection dominant.

Le champignon filamenteux Cryphonectria parasitica, également appelé Endothia parasitica, qui appartient au groupe des ascomycètes, sécrète naturellement une aspartylprotéase : l'endothiapepsine, capable de faire coaguler le lait en hydrolysant spécifiquement les micelles de caséine et donc utile dans la fabrication de certains fromages, en particulier les fromages à pâte cuite de type Emmental. Cette enzyme, dont La séquence de 330 acides aminés a été décrite par Barkolt V., 1987, Eur. J. Biochem. 167, 327-338, remplace alors la chymosine.

On produit actuellement cette enzyme à l'échelle industrielle par des procédés de fermentation de souches Cryphonectria parasitica choisies pour leur bon niveau d'expression de l'endothiapepsine.

Ces procédés présentent des inconvénients et notamment celui d'une immobilisation d'un volume de réacteur important car la quantité d'endothiapepsine par unité de biomasse, donc la quantité d'endothiapepsine par unité de volume du réacteur pour une quantité de biomasse donnée, sont faibles.

Les techniques classiques de mutation-sélection ne permettent d'augmenter que d'environ 30 % la quantité d'endothiapepsine produite par unité de biomasse.

Il existe donc un besoin pour des outils de génie génétique permettant d'obtenir des souches Cryohonectria parasitica surproductrices d'endothiapepsine avec un facteur de surproduction important. Celui-ci est ici défini comme le rapport entre la quantité d'endothiapepsine produite par la souche transformée surproductrice et la quantité de cette protéine produite par la souche témoin non transformée, pour une même quantité de biomasse.

Cryphonectria parasitica est un champignon filamenteux dont la génétique est encore très peu connue, contrairement à celle d'autres champignons filamenteux du groupe des ascomycètes tels ceux du genre Aspergillus, Neurospora ou Trichoderma. Ce n'est que très récemment qu'ont été décrites les premières transformations de Cryphonectria par Churchill et al, 1990, Curr. Gen, 17, 25-31. Ces auteurs ont transformé des souches de laboratoire de Cryphonectria en utilisant des plasmides porteurs de marqueurs de résistance à l'hygromycine, le bénomyl ou le G 418. Aucune protéine d'intérêt commercial n'a jamais jusqu'à présent été produite en utilisant une souche de Cryphonectria recombinée par génie génétique.

Récemment au Congrès : "Annual Meeting of the American Phytopathological Society and the Canadian Phytopathological Society" du 4 au 8 août 1990, G.H. Choi et al ont - semble-t'il (d'après l'abstract publié de leur communication) - présenté la séquence codante de l'endothiapepsine, une séquence prépro supposée de 88 acides aminés et une partie du promoteur de l'endothiapepsine comprenant la boîte TATA mais pas les régions activatrices nécessaires pour que le promoteur puisse fonctionner. Un tel fragment d'ADN génomique, mais comportant une séquence prépro de 89 acides aminés, avait déjà été isolé et séquencé par la Demanderesse en 1989, qui avait constaté qu'il ne permet pas l'expression de l'endothiapepsine (cf. sections 1 et 6 ci-après).

La présente invention concerne une nouvelle cassette d'expression d'un précurseur de l'endothiapepsine dans Cryphonectria parasitica, caractérisée en ce qu'elle comprend un promoteur fonctionnel en amont d'une séquence codant pour le précurseur de l'endothiapepsine, laquelle a la séquence (P1) suivante:

```
Ser Thr Gly Ser Ala Thr Thr Thr Pro Ile Asp Ser Leu Asp Asp Ala Tyr
Ile Thr Pro Val Gln Ile Gly Thr Pro Ala Gln Thr Leu Asn Leu Asp Phe
Asp Thr Gly Ser Ser Asp Leu Trp Val Phe Ser Ser Glu Thr Thr Ala Ser
Glu Val Asp Gly Gln Thr Ile Tyr Thr Pro Ser Lys Ser Thr Thr Ala Lys
Leu Leu Ser Gly Ala Thr Trp Ser Ile Ser Tyr Gly Asp Gly Ser Ser Ser
Ser Gly Asp Val Tyr Thr Asp Thr Val Ser Val Gly Gly Leu Thr Val Thr
Gly Gln Ala Val Glu Ser Ala Lys Lys Val Ser Ser Ser Phe Thr Glu Asp
Ser Thr Ile Asp Gly Leu Leu Gly Leu Ala Phe Ser Thr Leu Asn Thr Val
Ser Pro Thr Gln Gln Lys Thr Phe Phe Asp Asn Ala Lys Ala Ser Leu Asp
Ser Pro Val Phe Thr Ala Asp Leu Gly Tyr His Ala Pro Gly Thr Tyr Asn
Phe Gly Phe Ile Asp Thr Thr Ala Tyr Thr Gly Ser Ile Thr Tyr Thr Ala
Val Ser Thr Lys Gln Gly Phe Trp Glu Trp Thr Ser Thr Gly Tyr Ala Val
Gly Ser Gly Thr Phe Lys Ser Thr Ser Ile Asp Gly Ile Ala Asp Thr Gly
Thr Thr Leu Leu Tyr Leu Pro Ala Thr Val Val Ser Ala Tyr Trp Ala Gln
Val Ser Gly Ala Lys Ser Ser Ser Ser Val Gly Gly Tyr Val Phe Pro Cys
Ser Ala Thr Leu Pro Ser Phe Thr Phe Gly Val Gly Ser Ala Arg Ile Val
Ile Pro Gly Asp Tyr Ile Asp Phe Gly Pro Ile Ser Thr Gly Ser Ser Ser
Cys Phe Gly Gly Ile Gln Ser Ser Ala Gly Ile Gly Ile Asn Ile Phe Gly
Asp Val Ala Leu Lys Ala Ala Phe Val Val Phe Asn Gly Ala Thr Thr Pro
Thr Leu Gly Phe Ala Ser Lys ;
```

Une cassette d'expression d'un précurseur de l'endothiapepsine désigne ici une séquence d'ADN comprenant la séquence codant pour ce précurseur, flanquée de signaux permettant la transcription et la traduction de cette séquence codante dans Cryphonectria parasitica.

Par précurseur de l'endothiapepsine, on entend une protéine susceptible d'être sécrétée et de générer, après une ou plusieurs étapes de maturation, de l'endothiapepsine dans le milieu de culture.

On utilisera de préférence le précurseur naturel de l'endothiapepsine, appelé préproendothiapepsine, de séquence (P4) suivante :

```
Met Ser Ser Pro Leu Lys Asn Ala Leu Val Thr Ala Met Leu Ala Gly
Gly Ala Leu Ser Ser Pro Thr Lys Gln His Val Gly Ile Pro Val Asn
Ala Ser Pro Glu Val Gly Pro Gly Lys Tyr Ser Phe Lys Gln Val Arg
Asn Pro Asn Tyr Lys Phe Asn Gly Pro Leu Ser Val Lys Lys Thr Tyr
Leu Lys Tyr Gly Val Pro Ile Pro Ala Trp Leu Glu Asp Ala Val Gln
Asn Ser Thr Ser Gly Leu Ala Glu Arg Ser Thr Gly Ser Ala Thr Thr
Thr Pro Ile Asp Ser Leu Asp Asp Ala Tyr Ile Thr Pro Val Gln Ile
Gly Thr Pro Ala Gln Thr Leu Asn Leu Asp Phe Asp Thr Gly Ser Ser
Asp Leu Trp Val Phe Ser Ser Glu Thr Thr Ala Ser Glu Val Asp Gly
Gln Thr Ile Tyr Thr Pro Ser Lys Ser Thr Thr Ala Lys Leu Leu Ser
Gly Ala Thr Trp Ser Ile Ser Tyr Gly Asp Gly Ser Ser Ser Ser Gly
Asp Val Tyr Thr Asp Thr Val Ser Val Gly Gly Leu Thr Val Thr Gly
Gln Ala Val Glu Ser Ala Lys Lys Val Ser Ser Ser Phe Thr Glu Asp
Ser Thr Ile Asp Gly Leu Leu Gly Leu Ala Phe Ser Thr Leu Asn Thr
Val Ser Pro Thr Gln Gln Lys Thr Phe Phe Asp Asn Ala Lys Ala Ser
Leu Asp Ser Pro Val Phe Thr Ala Asp Leu Gly Tyr His Ala Pro Gly
Thr Tyr Asn Phe Gly Phe Ile Asp Thr Thr Ala Tyr Thr Gly Ser Ile
Thr Tyr Thr Ala Val Ser Thr Lys Gln Gly Phe Trp Glu Trp Thr Ser
Thr Gly Tyr Ala Val Gly Ser Gly Thr Phe Lys Ser Thr Ser Ile Asp
Gly Ile Ala Asp Thr Gly Thr Thr Leu Leu Tyr Leu Pro Ala Thr Val
Val Ser Ala Tyr Trp Ala Gln Val Ser Gly Ala Lys Ser Ser Ser Ser
Val Gly Gly Tyr Val Phe Pro Cys Ser Ala Thr Leu Pro Ser Phe Thr
Phe Gly Val Gly Ser Ala Arg Ile Val Ile Pro Gly Asp Tyr Ile Asp
Phe Gly Pro Ile Ser Thr Gly Ser Ser Ser Cys Phe Gly Gly Ile Gln
Ser Ser Ala Gly Ile Gly Ile Asn Ile Phe Gly Asp Val Ala Leu Lys
Ala Ala Phe Val Val Phe Asn Gly Ala Thr Thr Pro Thr Leu Gly Phe
Ala Ser Lys
```

Par analogie avec ce qui est connu pour d'autres aspartylprotéases telles que la chymosine de veau (Foltmann, 1970, Methods in Enzymol., 19, 421-436), le pepsinogène de porc (James et Sielecki, 1986, Nature, 319, 33-38) et la protéase A de S. cerevisiae (Woolford et al, 1986 Mol. Cel. Biol. 6, 2500-2510) on peut supposer que le précurseur naturel de l'endothiapepsine génère une forme sécrétée inactive, appelée proendothiapepsine, laquelle s'autoactive en endothiapepsine mature.

A cause de la dégénérescence du code génétique, il existe un grand nombre de séquences d'ADN codant pour une protéine dont la séquence (P4) répond à la formule donnée ci-dessus. Parmi celles-ci une séquence adaptée est celle qui comprend la séquence (N4a) suivante :

EP 0 475 842 B1

```
          ATGTCTT CCCCTCTCAA GAACGCCTTG GTGACCGCCA TGTTGGCTGG
          TGGTGCTCTC AGCTCGCCTA CAAAGCAACA CGTTGGAATT CCCGTCAACG
          CCTCTCCTGA AGTTGGCCCC GGAAAGTACT CGTTCAAGCA AGTCCGGAAC
          CCCAACTACA AGTTCAACGG GCCTCTGTCG GTCAAGAAGA CGTACCTCAA
          GTACGGCGTG CCGATCCCAG CCTGGCTGGA GGATGCTGTC CAGAACTCTA
          CCTCGGGCCT GGCTGAGCGC TCGACCGGTT CTGCGACCAC AACTCCCATC
          GACAGCCTCG ATGATGCTTA CATCACTCCG GTTCAGATCG GCACCCCTGC
          GCAGACTCTG AACCTGGACT TTGACACTGG ATCTTCGGAT CTGTGGGTCT
          TCAGCAGCGA GACTACAGCC AGCGAGGTCG ATGGGCAGAC CATCTACACC
          CCCAGCAAGA GCACCACCGC CAAGCTGCTG TCGGCGCTAC CTGGTCCATC
          TCCTACGGAG ACGGTAGCTC TTCCAGCGGC GATGTCTACA CTGACACCGT
          CTCGGTTGGA GGCCTTACCG TGACGGGCCA GGCTGTCGAG TCGGCCAAGA
          AGGTTTCTTC CAGCTTCACC GAGGACTCGA CCATTGACGG TCTCCTGGGC
          CTGGCCTTCA GCACCCTGAA CACTGTGTCG CCTACCCAGC AAAAGACTTT
          CTTCGACAAT GCGAAGGCGT CCTTGGACTC GCCTGTGTTC ACGGCTGATC
          TTGGCTACCA TGCCCCTGGT ACCTACAACT TCGGCTTCAT CGATACCACT
          GCCTACACGG GCTCCATCAC CTACACCGCT GTCTCGACCA AGCAAGGGTT
          CTGGGAGTGG ACTTCGACCG GCTACGCCGT CGGCTCCGGC ACCTTCAAGT
          CGACTTCCAT CGACGGCATC GCTGACACTG GCACGACCCT CCTGTACCTC
          CCTGCCACCG TCGTGTCGGC CTACTGGGCC CAGGTCTCGG GCGCCAAGTC
          CAGCTCTTCC GTCGGCGGCT ACGTCTTCCC CTGCAGCGCG ACCCTGCCTT
          CCTTCACCTT CGGCGTTGGC TCAGCTCGCA TTGTGATTCC TGGCGACTAC
          ATTGATTTCG GCCCCATCTC CACTGGAAGC TCGTCTTGCT TTGGCGGCAT
          CCAGTCCAGC GCTGGTATCG GCATCAACAT CTTCGGTGAT GTCGCTCTGA
          AGGCTTTGTC GTCTTCAACG GGGCTACAAC TCCCACTCTT GGCTTTGCTT
          CCAAG
```

Il est préférable que la séquence codant pour le précurseur de l'endothiapepsine soit interrompue par au moins un intron. On sait en effet que la présence d'introns dans la partie codante d'un gène peut dans certains cas augmenter l'expression de celui-ci (voir par exemple les travaux de J. Callis et al, 1987, Genes and Development, 1, 1183-1200).

Une séquence codant pour la préproendothiapepsine intéressante est donc celle qui comprend la séquence (N4a) interrompue par au moins un intron. Une séquence particulièrement appréciée de ce type est celle qui comprend la séquence (N4b) suivante :

```
                                            AT  GTCTTCCCCT  CTCAAGAACG
         CCTTGGTGAC  CGCCATGTTG  GCTGGTGGTG  CTCTCAGCTC  GCCTACAAAG
         CAACACGTTG  GAATTCCCGT  CAACGCCTCT  CCTGAAGTTG  GCCCCGGAAA
         GTACTCGTTC  AAGCAAGGTG  AGTAGAGCTG  CTTCTGTGTG  TTGCAACAGA
         AGACCAACGC  AAAAAGAAGA  GGTCAAGGCA  AGACGGATAT  TTTACTGACA
         ATTATACTTT  TGAAGTCCGG  AACCCCAACT  ACAAGTTCAA  CGGGCCTCTG
         TCGGTCAAGA  AGACGTACCT  CAAGTACGGC  GTGCCGATCC  CAGCCTGGCT
         GGAGGATGCT  GTCCAGAACT  CTACCTCGGG  CCTGGCTGAG  CGCTCGACCG
         GTTCTGCGAC  CACAACTCCC  ATCGACAGCC  TCGATGATGC  TTACATCACT
         CCGGTTCAGA  TCGGCACCCC  TGCGCAGACT  CTGAACCTGG  ACTTTGACAC
         TGGATCTTCG  GATCTGTGGG  TCTTCAGCAG  CGAGACTACA  GCCAGCGAGG
         TTGGTCAACC  CTCGCCCGCA  TTTTATTGCA  TACATTTTTA  GTTTTTTTGG
         TAATCAGAAT  ACTAACATTG  GGAATTTCCC  AACTGTAGGT  CGATGGGCAG
         ACCATCTACA  CCCCCAGCAA  GAGCACCACC  GCCAAGCTGC  TGTCGGGCGC
         TACCTGGTCC  ATCTCCTACG  GAGACGGTAG  CTCTTCCAGC  GGCGATGTCT
         ACACTGACAC  CGTCTCGGTT  GGAGGCCTTA  CCGTGACGGG  CCAGGCTGTC
         GAGTCGGCCA  AGAAGGTTTC  TTCCAGCTTC  ACCGAGGACT  CGACCATTGA
         CGGTCTCCTG  GGCCTGGCCT  TCAGCACCCT  GAACACTGTG  TCGCCTACCC
         AGCAAAAGAC  TTTCTTCGAC  AATGCGAAGG  CGTCCTTGGA  CTCGCCTGTG
         TTCACGGCTG  ATCTTGGCTA  CCATGCCCGT  GAGTGACCCC  TCTTGATACA
         TATACTTTTT  GATGAATCTT  GTTGGAGAAG  CATTCCCCAC  TAATATGGAA
         ATTGTTTGTA  TCTACAGCTG  GTACCTACAA  CTTCGGCTTC  ATCGATACCA
         CTGCCTACAC  GGGCTCCATC  ACCTACACCG  CTGTCTCGAC  CAAGCAAGGG
         TTCTGGGAGT  GGACTTCGAC  CGGCTACGCC  GTCGGCTCCG  GCACCTTCAA
         GTCGACTTCC  ATCGACGGCA  TCGCTGACAC  TGGCACGACC  CTCCTGTACC
         TCCCTGCCAC  CGTCGTGTCG  GCCTACTGGG  CCCAGGTCTC  GGGCGCCAAG
         TCCAGCTCTT  CCGTCGGCGG  CTACGTCTTC  CCCTGCAGCG  CGACCCTGCC
         TTCCTTCACC  TTCGGCGTTG  GCTCAGCTCG  CATTGTGATT  CCTGGCGACT
         ACATTGATTT  CGGCCCCATC  TCCACTGGAA  GCTCGTCTTG  CTTTGGCGGC
         ATCCAGTCCA  GCGCTGGTAT  CGGCATCAAC  ATCTTCGGTG  ATGTCGCTCT
         GAAGGCCGCC  TTTGTCGTCT  TCAACGGGGC  TACAACTCCC  ACTCTTGGCT
         TTGCTTCCAA  G
```

Un promoteur fonctionnel signifie ici un promoteur, constitutif ou régulable, susceptible de provoquer dans <u>Cryphonectria parasitica</u> la transcription de la séquence codant pour le précurseur de l'endothiapepsine. Ce promoteur comporte un élément TATA situé dans une zone riche en AT, une région d'initiation de la transcription en aval de celui-ci et en amont de ce dernier, des séquences, dites séquences d'activation amont UAS (Upstream Activating Sequence) ou de répression amont URS (Upstream Repressing Sequence), qui règlent la force du promoteur sous l'effet de protéines régulatrices.

Pour déterminer la fonctionnalité d'une séquence d'ADN comme promoteur, on utilisera commodément la méthode décrite dans la section 11. Celle-ci consiste à tansformer une souche de Cryphonectria parasitica rendue déficiente pour la production d'endothiapepsine par une mutation du gène de structure de cette protéine, par la cassette d'expression portant la séquence à tester et un marqueur de sélection, puis à repérer parmi les tansformants ceux qui sont producteurs d'endothiapepsine à l'aide du test de sélection sur milieu gélosé contenant de la caséine décrit dans la section 7.

Un promoteur apprécié est le promoteur du gène codant pour la préproendothiapepsine ou une partie fonctionnelle de ce promoteur. Celle-ci comprend, par exemple, une partie portant la boite TATA de la séquence (N5) suivante :

```
AAGCTTATCC GCCGCCGGCG GGGGAATTCT ATTGAACTTG TTCGAATCAT

TGGTCCGTGG TCTTTTCGTC CATGCGGGCT CCGCTGGCGG ATGAATGACC

TTCTGGCTTC TAGCCTGGCG AAGCGATGTT ACTCTGTTGT CTATACTATA

CGATATGGTC AAGAGAGCAC ATGTGCCGCC AGATGAAGAC ATGTATATAA

AAGGAGTGGC CTCGACGGTT GCTCAACCAT CTTCTGTCTG TCCCAACGCC

ATCGACTCTT CAACTTCTCC TTCGTGTTCC ACCACCATCA CCTTGCTCCA

GACTTAGGAC TTTCAGCAAC CTTCAAAG
```

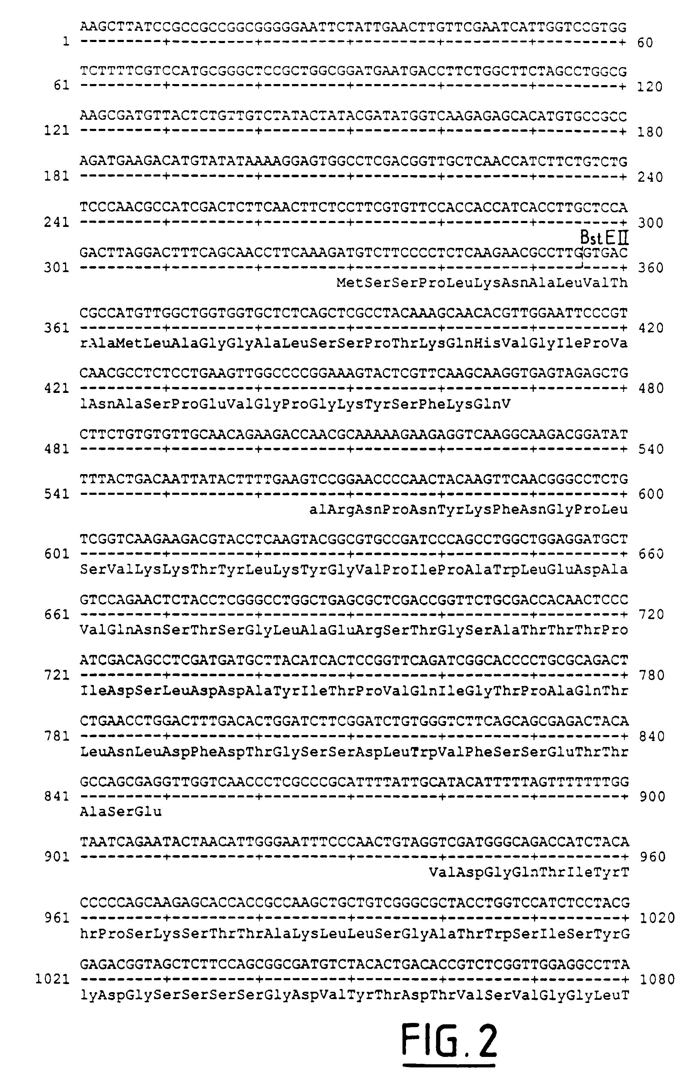

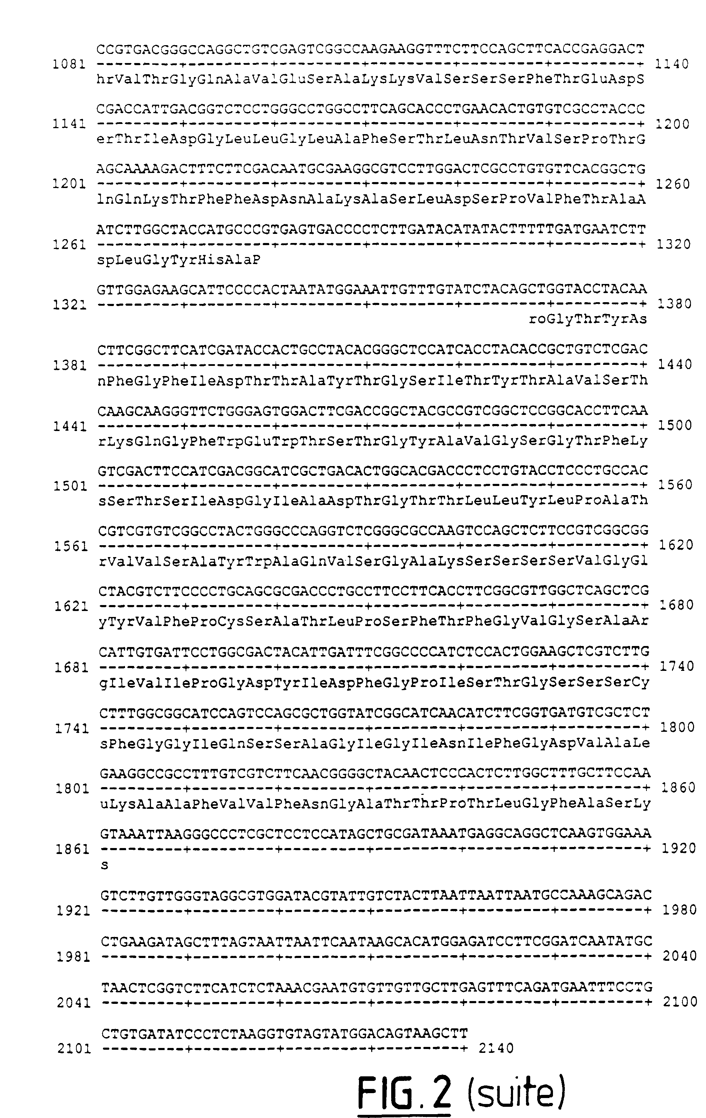

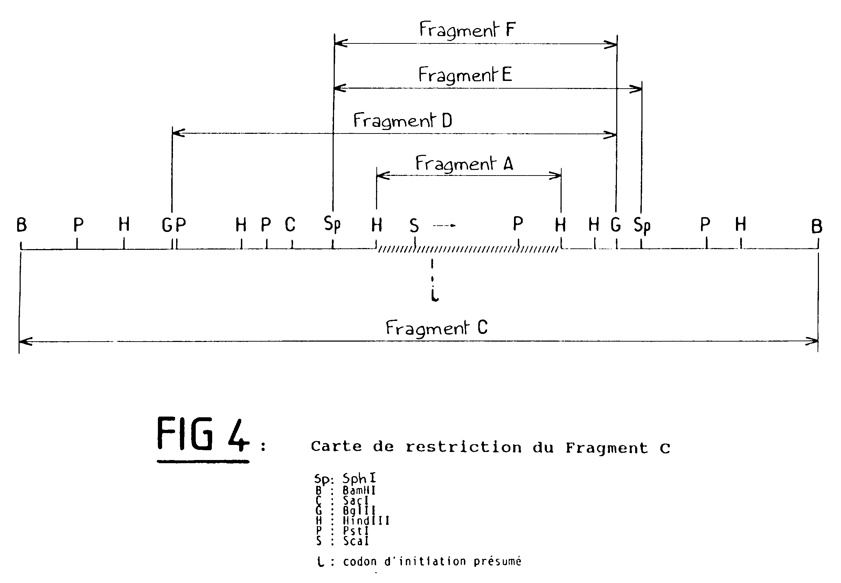

et en amont de la séquence (N5), un segment X du fragment C compris entre l'extrémité 5' du fragment A et l'extrémité 5' du fragment C, choisi de façon que le segment X contienne une région activatrice. Le fragment C est une partie de l'ADN génomique de Cryphonectria parasitica contenue dans la souche d'E. coli déposée à la CNCM le 31.08.1990 sous le N° I-998. Sa carte de restriction, ainsi que celle du fragment A contenu dans le fragment C, sont représentées sur la figure 4. La séquence nucléotidique du fragment A, qui comprend l'ADN génomique de Cryphonectria parasitica codant pour la préproendothiapepsine, est représentée sur la figure 2.

Une localisation plus précise de cette région activatrice pourra être déterminée par l'obtention d'une série de segments du fragment C (préparés par exemple par digestion à l'aide d'endonucléases ou d'exonucléases) comprenant le fragment A flanqué en 5' de segments de différentes tailles de la partie du fragment C comprise entre l'extrémité 5' du fragment A et l'extrémité 5' du fragment C, et détermination de la fonctionnalité du promoteur obtenu à l'aide de la méthode mentionnée ci-dessus.

Un exemple de segment X du fragment C comportant une région activatrice est le fragment de séquence ci-après :

```
GCATGCTTGG CTCTTTAACG TCCTGCCCAT TCAGGGCCTT CAGCCGGCAC

TGGTCCTTCA TCAAGGGGGA CCTCATGACC ATGAACTAAT CTGTGATATC

TGATATATTC TAGAAGGCTT GGCTCCTCAA AGTTCCAGC TAATGAATCA

GCGGCCCGCC GCCCTTAAAC CGCATCAGGC AAGTCGTTTG GTGTTGCCAG

GCGATGGCGA CAGGAGAGTG GTGTTGATGG GACAAGGGGA GGGAGGCTTA

GCCGACTTCA TCCATAGCAC CCACCTGCTT GGCGCCGATA AGTCTGACGA

TCCGCTTGAG CTGCAAAACG GCTCCTTGAC CTTTGTTTGG TCGACCGAGG

GAAATAGTCT CTTTTTGCGT GATCGTGCGC GCTTCGTATA GCAATAGCAG

CCAGCACCAG CAGGACGGGC CGTTGTCACG GTCACATCGT TCGCAACATG

CCGAGCGTAG GGATGAACGA ATGACTCGAG CCTTGCCTGA CAGTCTGGCA

ATCAATCTAT GGTCACGCAC GATCACAAGC CAATCGCTGT GACTGCGTTA

CTAGCCCAAT AATCCCTTGT TCGATCAGAG TGTTCTACAG ACTTCAAGTG

AGGTTCAC
```

Des exemples de parties fonctionnelles du promoteur du gène codant pour la préproendothiapepsine sont les segments BglII-ScaI et BamHI-ScaI du fragment C (cf. figure 4).

Il peut être intéressant aussi d'utiliser un promoteur provenant d'un autre gène connu pour être exprimé dans Cryphonectria parasitica ou dans un autre champignon filamenteux du groupe des ascomycètes, par exemple le promoteur du gène codant pour la glycéraldéhyde-3 phosphate déshydrogénase de Cryphonectria parasitica décrit par Choi et al, 1990, Nucleic Acids Research, 18, 18, Oxford University Press ou celui du gène codant pour la glycéraldéhyde-3-phosphate déshydrogénase d'Aspergillus nidulans décrit par Mullaney et al, 1985, Mol. Gen. Genet., 199, 37-45.

La cassette d'expression de l'invention est introduite dans Cryphonectria parasitica de préférence par cotransformation avec un vecteur portant le gène de sélection. La cassette d'expression est elle-même portée par un vecteur ou, de préférence, sous la forme d'un fragment linéaire. La cassette d'expression est, de préférence, maintenue à l'état intégré dans le chromosome. Le vecteur portant le gène de sélection est maintenu, soit par intégration dans le chromosome, soit sous forme linéaire extrachromosomique à l'aide de séquences du type séquences télomériques. Après sporulation, on sélectionne, de préférence, les transformés ayant perdu le marqueur de sélection.

L'invention concerne donc aussi une souche de Cryphonectria parasitica productrice d'endothiapepsine, caractérisée en ce qu'elle est transformée par la cassette définie précédemment et elle surproduit l'endothiapepsine par rapport à la souche non transformée c'est-à-dire qu'elle sécrète davantage d'endothiapepsine que cette dernière.

Pour une utilisation de cette souche dans l'industrie agroalimentaire, il est avantageux que cette souche soit dépourvue de marqueur de sélection dominant, tel que par exemple un gène de résistance à un antibiotique.

De préférence la souche hôte est la souche SEBR103, obtenue à l'issue d'un procédé classique de mutation-sélection et déposée à la CNCM le 31.08.1990 sous le N° I-997. On apprécie alors que la souche tansformée surproduise l'endothiapepsine par rapport à la souche SEBR103 avec un rapport de surproduction au moins égal à deux.

L'invention a également trait à un procédé de préparation de l'endothiapepsine, caractérisé en ce qu'il comprend une étape de culture de la souche définie précédemment, suivie d'une étape d'isolement et de purification de cette protéine. Ce procédé remplacera avantageusement les procédés actuels de production de l'endothiapepsine.

L'invention concerne également un procédé pour obtenir une souche Cryphonectria parasitica surproductrice d'endothiapepsine, transformée par la cassette définie précédemment et dépourvue de marqueur de sélection dominant, caractérisé en ce qu'il comprend au moins un cycle comportant une étape de cotransformation par une cassette d'expression telle que définie précédemment et un marqueur de sélection dominant, suivie d'une étape de purification par sporulation permettant d'éliminer le marqueur de sélection dominant. De préférence ce procédé comprend au moins deux cycles de ce type.

L'invention sera mieux comprise à l'aide de l'exposé ci-après, divisé en sections, qui comprend des résultats expérimentaux et une discussion de ceux-ci. Certaines de ces sections concernent des expériences effectuées dans le but de réaliser l'invention, d'autres des exemples de réalisation de l'invention, donnés bien sûr à titre purement illustratif.

Une grande partie de l'ensemble des techniques ci-après, bien connues de l'homme de l'art, est exposée en détail dans l'ouvrage de Sambrook et Maniatis : "Molecular cloning : a Laboratory manual" publié en 1989 par les éditions Cold Spring Harbor Press à New York (2e édition) :

La description ci-après sera mieux comprise en se rapportant aux figures 1 à 12.

La figure 1 représente la séquence d'acides aminés de l'endothiapepsine.

La figure 2 représente la séquence nucléotidique du fragment A, le site BstEII utilisé dans la section 10 étant représenté par des pointillés verticaux, ainsi que la séquence d'acides aminés traduite.

La figure 3 représente la séquence d'acides aminés de la préproendothiapepsine.

La figure 4 représente une carte de restriction du fragment C, les sites BamHI, HindIII, PstI, SphI, SacI, BglII et ScaI étant symbolisés par les lettres B, H, P, Sp, C, G et S, ainsi que les fragments A, D, E et F contenus dans le fragment C.

La figure 5 représente la séquence de l'ADN génomique codant pour la préproendothiapepsine, interrompue par trois introns, qui sont soulignés.

La figure 6 représente une carte de restriction du plasmide p163,1. Les différents segments de restriction sont marqués de manière arbitraire selon la légende ci-dessous :

= Segment d'ADN issu du plasmide pBR322

= Localisation de l'origine de réplication (ORI)

= Segment d'ADN contenant la séquence codant pour un précurseur naturel de l'hGH

= Segment d'ADN du phage fd contenant un terminateur de transcription

= Segment d'ADN contenant un promoteur-opérateur hybride tryptophane-lactose UV5

= Segment d'ADN codant pour la β-lactamase (ApR : résistance à l'ampicilline).

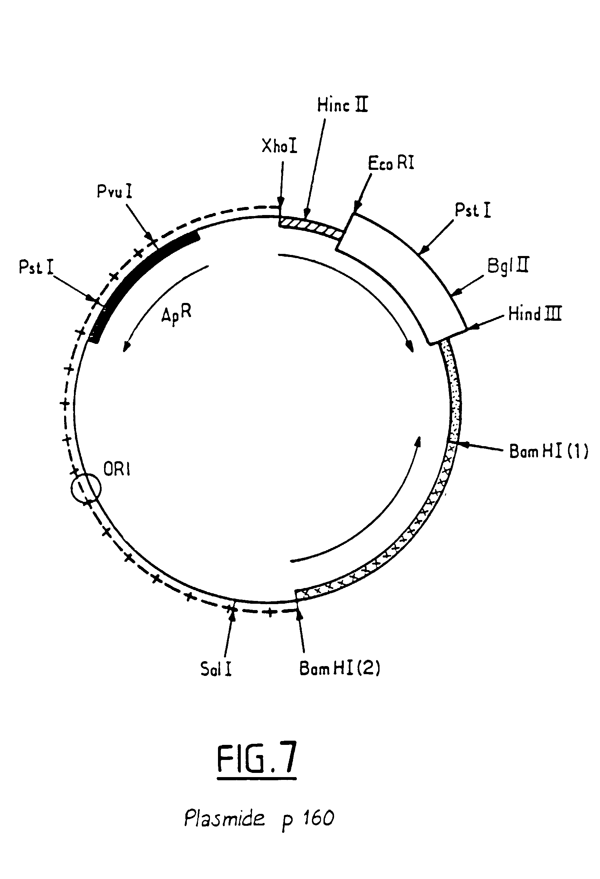

La figure 7 représente la carte de restriction d'un plasmide p160 dont les fragments PvuI-XhoI-BamHI(1) et PvuI-ORI-BamHI(2) proviennent respectivement des plasmides p163,1 et pBR327 et dont la petit fragment BamHI(2)-BamHI(1) est le fragment 3 décrit ci-après.

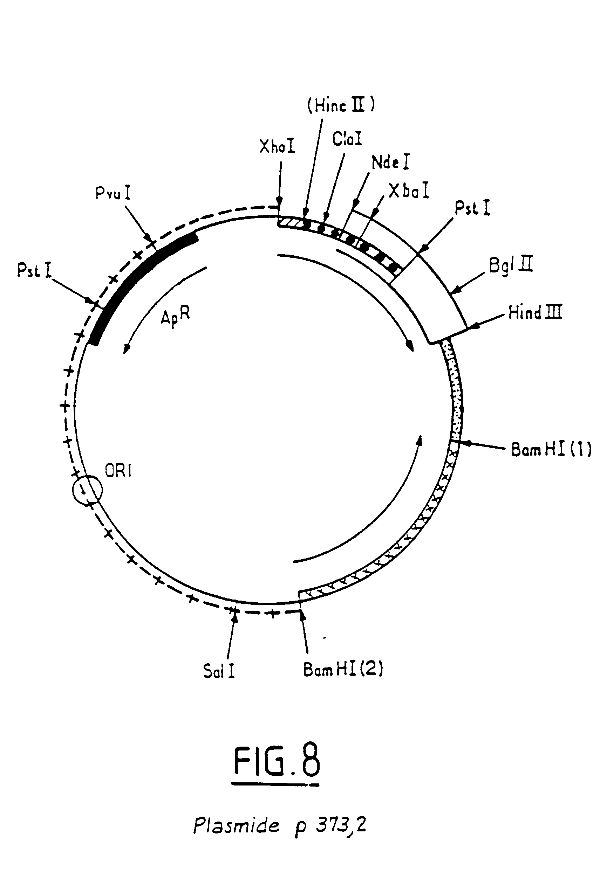

La figure 8 représente la carte de restriction du plasmide p373,2. Les différents segments de restriction sont marqués de manière arbitraire selon la légende ci-dessous :

```
 _____
 -+-+-+-+-+-+-   = Séquence PvuI-BamHI issue du plasmide pBR327
```

```
 _____
 _ __ __ __ __ __  = Séquence PvuI-XhoI issue du plasmide p163,1
```

```
 ///////////////  = Séquence XhoI-HincII issue du plasmide p163,1
```

```
          ClaI    NdeI    PstI
 (HincII)   |       |       |
 _____|_____|_____|___
 ●●●●●●●●●●●●●●         Fragment 4 décrit ci-après
```

```
 XXXXXXXXXXXXX   = Fragment 3 décrit ci-après
```

```
 ▓▓▓▓▓▓▓▓▓▓▓▓▓   = Segment d'ADN du phage fd contenant un terminateur
                    de transcription
```

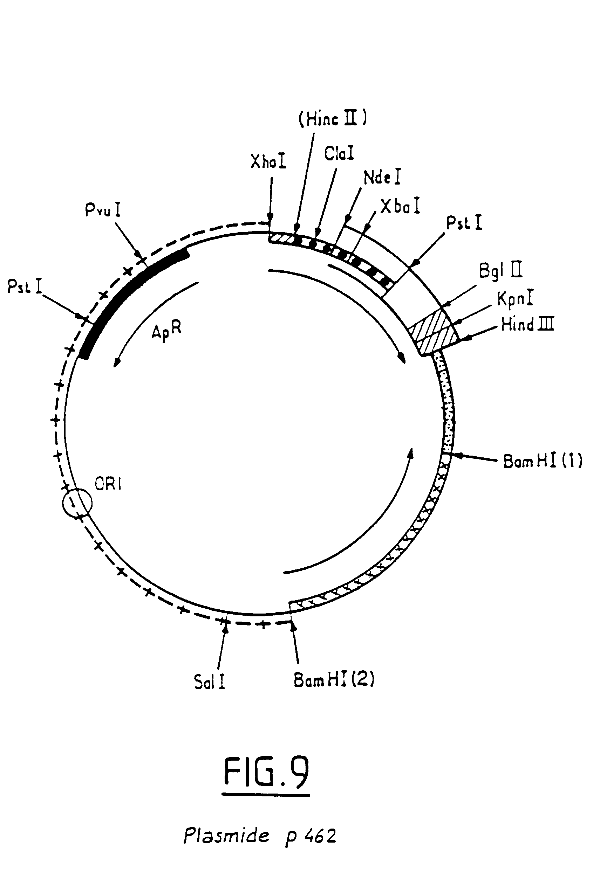

La figure 9 représente une carte de restriction du plasmide p462, le fragment synthétique BglII-HindIII défini ci-après étant représenté par : ▨

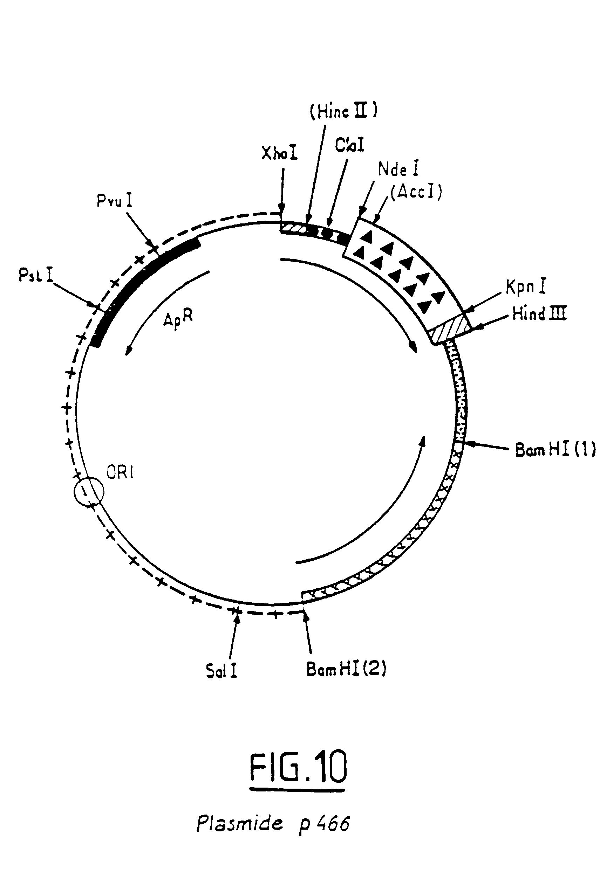

La figure 10 représente une carte de restriction du plasmide p466, le fragment NdeI-KpnI comprenant le gène codant pour l'urate oxydase étant représenté par : ▨

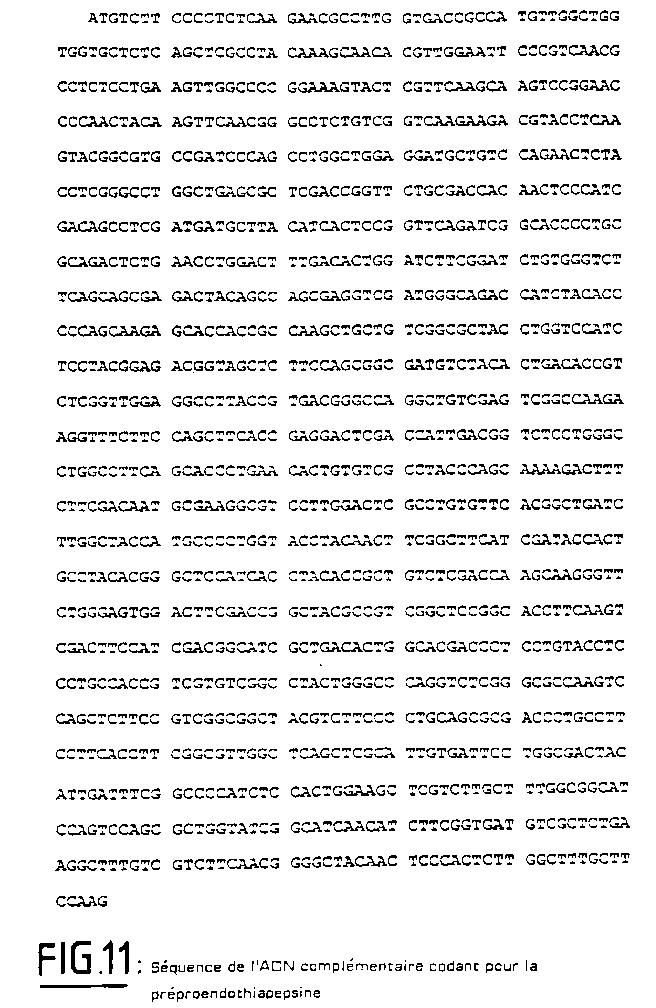

La figure 11 représente la séquence de l'ADN complémentaire codant pour la préproendothiapepsine.

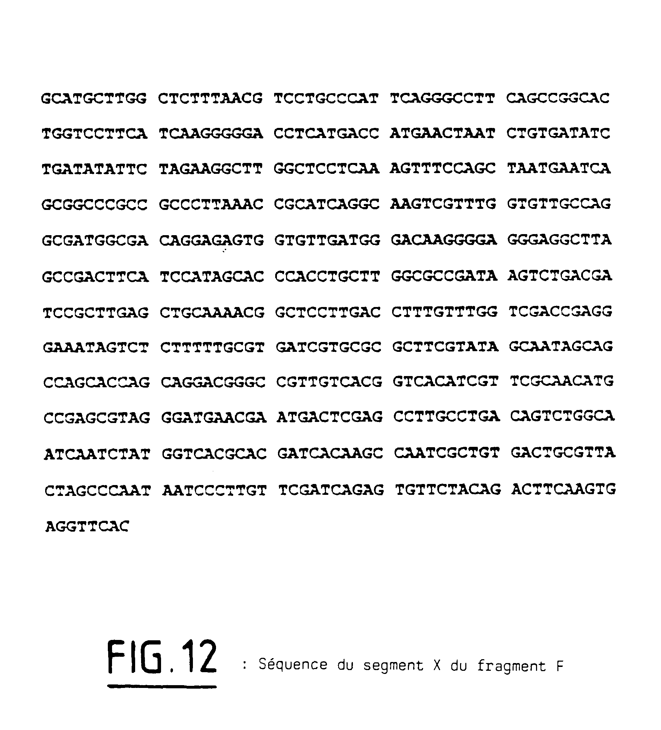

La figure 12 représente la séquence du segment SphI-HindIII du fragment F.

Section 1 : Isolement du fragment A, fragment d'ADN génomique d'environ 2,1 kb, contenant la séquence codante du précurseur de l'endothiapepsine.

1) Préparation de l'ADN génomique

La souche appelée SEBR 103 a été identifiée par le Centraal Bureau Voor Schimellcultures comme appartenant à l'espèce Cryphonectria parasitica et déposée à la Collection Nationale de Culture de Microorganismes C.N.C.M. sous le n° I-997.

A partir de conidiospores de la souche Cryphonectria parasitica SEBR 103, récoltées à la surface d'une boîte de Petri contenant un milieu gélosé, appelé milieu G et dont la composition est précisée dans le tableau 3 ci-après, préalablement inoculé par un implant mycélien ou par une suspension de conidiospores de cette même souche, puis incubé pendant 4 semaines à température ambiante et la lumière, on ensemence par étalement d'une goutte de suspension de conidiospores une boite de Petri contenant 25 ml d'un milieu gélosé, appelé milieu A, dont la composition est donnée dans le tableau 1 ci-après. Après 3 jours d'incubation à 30°C, le mycélium obtenu est utilisé pour ensemencer une fiole de 1 l contenant 100 ml d'un milieu liquide, appelé milieu B, dont la composition est précisée dans le tableau 2 ci-après. Au bout de 24 h d'incubation à 28°C sous agitation à 220 tr/min, 25 ml du bouillon de culture servent à ensemencer 250 ml de milieu B neuf contenu dans une fiole de 1 l. Le lendemain, après incubation dans les conditions déjà définies, la totalité du bouillon de culture (250 ml) est centrifugée à 5 000 g pendant 5 min. Le culot est remis en suspension dans une solution d'acétate de sodium 0,15 M contenant 1 mM d'EDTA. Le mycélium est récupéré par filtration sur papier Whatman 3MM, congelé dans l'azote liquide et réduit en poudre au pilon dans un mortier.

On reprend 10 g de poudre de mycélium dans 40 ml de solution d'acétate de sodium 0,15 M contenant 4 % de sodium lauroyl-sarcosine de pH 5. On ajoute, après 30 min d'agitation douce à température ambiante, du chlorure de sodium à 0,1 M final. Au bout de 30 min d'agitation douce, à température ambiante, on extrait les protéines avec une solution de phénol (49 % v/v) contenant de l'alcool isoamylique (2 % v/v) et du chloroforme (49 % v/v). Après 3 extractions avec ce mélange, on extrait avec une solution de chloroforme contenant de l'alcool isoamylique (96 % de chloroforme - 4 % d'alcool isoamylique). Après 2 extractions de ce type, on soumet la phase aqueuse à l'addition de 2 volumes d'éthanol. Les 2 solutions sont mélangées doucement par retournement, ce qui provoque l'apparition d'un filament d'ADN précipité. Ce filament est prélevé à l'aide d'une pipette Pasteur et déposé dans un tube Eppendorf. On centrifuge ce tube pendant 5 min à 100 g. Le culot est lavé dans une solution d'éthanol à 70 % puis séché sous vide. Le culot est repris dans 1 ml du tampon, appelé tampon TE, de composition [Tris (HCl) 10 mM, pH 8 ; EDTA 1 mM].

TABLEAU 1

| Composition du milieu A | |
|---|---|
| Glucose anhydre | 50 g/l |
| Farine de soja (Soyoptim de Société Industrielle des Oléagineux) | 20 g/l |
| Nitrate de calcium | 9 g/l |
| Agar (Bacto-agar de Difco) | 15 g/l |
| Solution saline 1 (composition précisée ci-après) | 0,5 g/l |
| Vérifier ou ajuster le pH à 6,0 à l'aide de HCl 1N ou NaOH 1N | |
| | |
| Composition de la solution saline 1 | |
| $H_3BO_3$ | 30 ml |
| $MnCl_2, 4H_2O$ | 79 ml |
| $ZnCl_2$ | 200 ml |
| $Na_2MoO_4, 2H_2O$ | 20 ml |
| $FeCl_3$ anhydre | 50 ml |
| $CuSO_4, 5H_2O$ | 200 ml |
| eau distillée qsp | 500 ml |
| (Cette suspension est conservée au maximum un mois à + 4°C) | |

TABLEAU 2

| Composition du milieu B | |
|---|---|
| Glucose | 10 g/l |
| Thiamine | 2 mg/l |
| Solution saline 2 (composition donnée ci-après) | 62,5 ml/l |
| Extrait de levure (Difco) | 2,5 g/l |
| Extrait de malt (Difco) | 7,5 g/l |
| Vérifier ou ajuster le pH à 6,0 à l'aide de HCl 1N ou NaOH 1N. | |

TABLEAU 2   (suite)

| Composition du milieu B | |
| --- | --- |
| Stériliser 30 min à 110°C. | |
| | |
| Composition de la solution saline 2 | |
| $NH_4NO_3$ | 24 g/l |
| $KH_2PO_4$ | 16 g/l |
| $NaH_2SO_4$ | 4 g/l |
| KCl | 8 g/l |
| $MgSO_4, 7H_2O$ | 2 g/l |
| $CaCl_2$ | 1 g/l |
| solution saline 1 (composition précisée dans le tableau 1) | 8 ml/l |

TABLEAU 3

| Composition du milieu G | |
| --- | --- |
| Partie A | |
| Extrait de malt | 20 g |
| Extrait de levure | 2 g |
| agar | 16 g |
| Eau | 1 l |
| Ajuster le pH à 5,5 | |
| Autoclaver 20 min à 120°C | |
| Partie B | |
| Acide aspartique | 1 000 mg |
| Biotine | 10 mg |
| Eau | 1 l |
| Diluer au 1/10 la partie B, la répartir en filtrant à 0,2 M par fractions de 1 ml et la conserver à -20°C. | |
| Au moment de l'emploi, ajouter 1 ml de la partie B diluée à 1 l de la partie A en surfusion à 45°C puis répartir l'ensemble en boîte de Petri. | |

2) <u>Préparation de la sonde 1 et de la sonde 2</u>

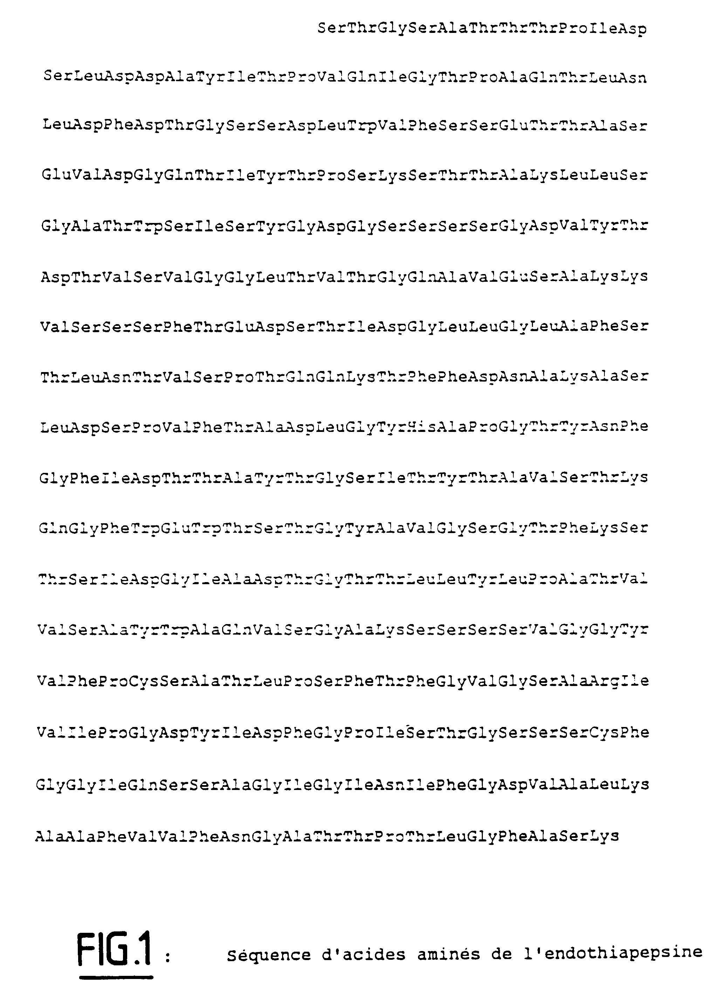

Ces sondes sont des pools d'oligonucléotides de synthèse, comprenant l'ensemble des séquences codantes pour deux peptides choisis dans la séquence d'acides aminés de l'endothiapepsine mature [SEQ ID NO : 1] exportée dans le milieu de culture de <u>C</u>. <u>parasitica</u>, décrite par V. BARKHOLT, 1987, Eur. J. Biochem., 167, 327-338, représentée sur la figure 1. Les peptides choisis dans cette séquence appelés peptide 1 et peptide 2 et correspondant respectivement à la sonde 1 et à lasonde 2 sont les suivants:

```
Peptide 1 : Val-Asp-Gly-Gln-Thr
```

```
Peptide 2 : Gly-Phe-Trp-Glu-Trp-Thr
```

A ces peptides correspondent respectivement 256 ($4^3$ x $2^2$) et 64 ($4^2$ x $2^2$) oligonucléotides codant pour ceux-ci, représentés par les formules ci-après.

```
              N   Y   N   R   N
              A       A       A
              C   C   C   G   C
             GT  GA  GG  CA  AC        sonde 1
              G   T   G   A   G
              T       T       T




              N               N
                  Y   R
              A               A
              C   C   A       C
             GG  TT  TGGGA  TGGAC      sonde 2
              G   T   G       G
              T               T
```

3) <u>Marquage de la sonde 1 et de la sonde 2</u>

Les sondes sont marquées à la terminale désoxynucléotide transférase (TdT) (commercialisée par Stratagene, réf. : 600 132).

La réaction s'effectue sur 100 ng d'un mélange d'oligonucléotides en solution dans du tampon de réaction "Cobalt" (fourni 10 fois concentré par IBI inc.) : 1,4 M de cocodylate de potassium-pH 7,2, 300 mM de dithiothréitol, 1 µl d'enzyme désoxynucléotidylterminal-transférase (Stratagene) et 50 µCi de désoxycytidyltri-phosphate dCTP marqué au $^{32}$P.

La réaction se fait à 37°C pendant 10 min puis est arrêtée en ajoutant 1 µl d'EDTA 0,5 M.

On extrait au phénol et on dialyse sur colonne de polyacrylamide Biogel P 10 (Biorad : 150-1050).

On obtient ainsi la sonde 1 radiomarquée et la sonde 2 radiomarquée.

4) <u>Hydrolyse de l'ADN génomique de Chryphonectria parasitica</u>

L'ADN génomique obtenu à l'issue de 1) a été soumis séparément à une digestion par chacune des enzymes de restriction suivantes : EcoRI, HindIII et BamHI. Dans chaque cas, 10 µg du produit de digestion ont été déposés sur gel d'agarose 0,8 % et soumis à une électrophorèse en présence d'une gamme de tailles radiomarquée (Amersham réf. SJ5000). L'ADN a ensuite été transféré sur un filtre de nitrocellulose (Biorad, réf. 162-0117) suivant la technique bien connue de l'homme de l'art sous le nom de Southern Blot, décrite dans Maniatis, op. cit., cette opération étant répétée de façon à obtenir deux filtres de nitrocellulose destinés à être hybridés l'un avec la sonde 1, l'autre avec la sonde 2.

5) <u>Hybridation</u>

Chaque filtre de nitrocellulose traité selon les techniques habituelles (Maniatis et al., op. cit.) a tout d'abord été lavé dans une solution de préhybridation contenant 6 x SSC ; 10 x Denhardt's et 100 µg/ml d'ADN de sperme de saumon soniqué et dénaturé (Sigma D9156) pendant quelques heures à 42°C, puis incubé dans la même solution et dans les mêmes conditions que celles indiquées précédemment en présence de l'une des sondes marquées 1 et 2. L'hybridation dure une nuit. La solution 6 x SSC s'obtient par dilution d'une solution 20 x SSC. La préparation du tampon 20 x SSC est décrite dans Maniatis, op. cit. En résumé, ce tampon contient 175,3 g/l de NaCl ; 88,2 g/l de citrate de sodium et est ajusté à pH 7 par quelques gouttes de NaOH 1N. La solution 10 x Denhardt's contient 1 g de Ficoll, 1 g de polyvinylpyrrolidone, 1 g de sérum-albumine bovine pour 500 ml de volume final.

Après l'hybridation, chacun des filtres est lavé individuellement dans une solution contenant 0,5 SSC à 42°C. Les filtres sont ensuite exposés à un film photographique (Kodak XAR5) pendant la nuit. L'analyse du film révélé montre, dans le cas de l'hydrolysat obtenu avec l'enzyme HindIII, qu'une bande dont le poids moléculaire correspond approximativement à un fragment de taille légèrement supérieure à 2,1 kb répond positivement avec les deux sondes radiomarquées.

6) Clonage d'un fragment d'ADN HindIII-HindIII, d'environ 2,1 kb, s'hybridant avec les sondes radiomarquées 1 et 2 :

a) Constitution d'une banque d'ADN génomique.

100 µg de l'ADN de Cryphonectria parasitica sont hydrolysés par l'enzyme HindIII et les fragments sont séparés par électrophorèse sur gel d'agarose 0,8 %. La région qui correspond aux fragments de taille légèrement supérieure à 2,1 kb est découpée et l'ADN est purifié par adsorption sur lit de silice (Geneclean Tm, Biorad P.O.Box 2284, La Jolla, California 92038-2284). Ces fragments sont ligués à l'aide de la ligase d'ADN du phage T4 (Gibco-BRL) dans pBR322 hydrolysé par HindIII et déphosphorylé (Biolabs Réf ; 321). Des cellules compétentes (c'est-à-dire capables d'être transformées) (cellules RRI, Gibco BRL, Réf. 520-8261 SA) sont transformées, comme indiqué par le fournisseur, par la solution de ligation et étalées sur milieu gélosé, appelé milieu gélosé LB (Maniatis, op. cit.) de composition précisée dans le tableau 4 ci-après. L'ensemble des colonies obtenues est la banque d'ADN génomique.

TABLEAU 4

| Composition du milieu LB gélosé | |
|---|---|
| Hydrolysat de caséine Bactotryptone (Difco) | 10 g/l |
| Extrait de levure Bacto (Difco) | 5 g/l |
| NaCl | 10 g/l |
| Ajuster le pH à 7,0 à l'aide de NaOH 5N | |
| Agar Bacto (Difco) | 15 g/l |
| Autoclaver 20 min à 120°C | |

b) Sélection des clones qui portent le fragment HindIII-HindIII s'hybridant avec les sondes radiomarquées 1 et 2, appelé fragment A.

Les colonies obtenues après transformation sont absorbées sur un filtre de nitrocellulose (Schleicher et Schull, Réf. 40117) et répliquées sur deux autres filtres de nitrocellulose. Une première série de filtres est hybridée avec la sonde 1 radiomarquée et une deuxième série de filtres est hybridée avec la sonde 2 radiomarquée. Les conditions de préhybridation, hybridation et lavage sont les mêmes que celles utilisées précédemment (cf. 5 ci-dessus).

Les clones qui répondent positivement avec les deux sondes sont purifiés. L'ADN plasmidique de l'un de ces clones est isolé. Le plasmide correspondant est appelé p472. Après s'être assuré qu'il s'hybridait bien avec chacune des deux sondes radiomarquées 1 et 2, le fragment HindIII-HindIII de 2,1 kb porté sur ce plasmide a été isolé, subcloné dans M13mp19 (Pharmacia). Ce fragment est appelé fragment A. Il a été séquencé par la technique du cyclone ("Cyclone I Biosystem" de IBI).

La séquence nucléotidique du fragment A est représentée sur la figure 2, laquelle indique également la numérotation des nucléotides, choisie arbitrairement, de façon à attribuer le n° 1 au nucléotide de l'extrémité 5' du fragment A, ainsi que la séquence d'acides aminés traduite.

7) Description de la séquence du fragment A (cf. figure 2)) :

Le fragment A porte la séquence nucléotidique codant pour la séquence peptidique de l'endothiapepsine mature exportée dans le milieu de culture décrite par Barkolt (cf. figure 1), laquelle commence au nucléotide 694 et se termine au nucléotide 1861 et est interrompue par deux introns situés aux nucléotides 850-938 et 1279-1367. En amont du nucléotide 694, la phase ouverte continue jusqu'au nucléotide 566 où se trouve un signal caractéristique de la fin d'un intron : AG. Aux nucléotides 468-469 se trouve un signal caractéristique du début d'un intron : GT. On remarquera que la séquence à cheval sur le début de l'intron et la fin de l'exon : AAGGTGAGT, correspond à la séquence consensus 5' de la jonction d'épissage décrite par Mount S.M., 1982, Nucl. Ac. Res., 10, 459-472. En amont du nucléotide 468, il n'y a qu'une seule phase ouverte (phase non interrompue par un codon stop) comportant au moins un ATG. Celle-ci comporte un ATG à la position 365-367 (dont l'environnement nucléotidique est compatible avec M. Kozak, 1984, Nucl. Ac. Res. 12, p. 2) et un ATG à la position 329-331, cette phase de lecture étant interrompue par le codon stop TAG à la position 305-307. Le logiciel U.W.G.C.G. de l'Université de Wisconsin : Devereux et al, 1984, Nucl. Ac. Res., 12, 8711-8721-option : Recherche de peptide-signal d'après la méthode de G. von Heijne, 1986, Nucl. ; Ac. Res., 14, 483-490, prévoit dans cette phase ouverte une seule séquence codant pour un peptide signal, la séquence ci-après, appelée séquence nucléotidique pré (commençant au nucléotide 329) :

```
     ATGTCTT CCCCTCTCAA GAACGCCTTG GTGACCGCCA TGTTGGCTGG

     TGGTGCTCTC AGC
```

codant pour le peptide signal de 20 acides aminés de séquence suivante, appelée séquence peptidique pré:

```
     Met Ser Ser Pro Leu Lys Asn Ala Leu Val Thr Ala Met Leu Ala Gly Gly

     Ala Leu Ser
```

Un peptide-signal est attendu par l'homme de l'art car l'endothiapepsine est une protéine sécrétée, ce qui exige la présence d'un peptide-signal.

Entre la séquence codant pour le peptide-signal ci-dessus et celle codant pour la protéine mature, il y a la séquence nucléotidique, suivante, appelée séquence nucléotidique pro (commençant au nucléotide 389) :

```
                    TCGCCTA CAAAGCAACA CGTTGGAATT CCCGTCAACG

          CCTCTCCTGA AGTTGGCCCC GGAAAGTACT CGTTCAAGCA AGTCCGGAAC

          CCCAACTACA AGTTCAACGG GCCTCTGTCG GTCAAGAAGA CGTACCTCAA

          GTACGGCGTG CCGATCCCAG CCTGGCTGGA GGATGCTGTC CAGAACTCTA

          CCTCGGGCCT GGCTGAGCGC
```

codant pour la séquence peptidique suivante, appelée séquence peptidique pro :

```
     Ser Pro Thr Lys Gln His Val Gly Ile Pro Val Asn Ala Ser Pro Glu Val

     Gly Pro Gly Lys Tyr Ser Phe Lys Gln Val Arg Asn Pro Asn Tyr Lys Phe

     Asn Gly Pro Leu Ser Val Lys Lys Thr Tyr Leu Lys Tyr Gly Val Pro Ile

     Pro Ala Trp Leu Glu Asp Ala Val Gln Asn Ser Thr Ser Gly Leu Ala Glu

     Arg
```

Une séquence peptidique (389-693) pro est également attendue par l'homme de l'art car elle a pour fonction d'inhiber l'endothiapepsine, probablement toxique pour C. parasitica, avant son exportation vers le milieu extérieur à la suite de laquelle cette séquence est clivée.

Il y a donc en amont de la séquence codant pour l'endothiapepsine mature la séquence codant pour la séquence peptidique prépro suivante :

```
Met Ser Ser Pro Leu Lys Asn Ala Leu Val Thr Ala Met Leu Ala Gly Gly

Ala Leu Ser Ser Pro Thr Lys Gln His Val Gly Ile Pro Val Asn Ala Ser

Pro Glu Val Gly Pro Gly Lys Tyr Ser Phe Lys Gln Val Arg Asn Pro Asn

Tyr Lys Phe Asn Gly Pro Leu Ser Val Lys Lys Thr Tyr Leu Lys Tyr Gly

Val Pro Ile Pro Ala Trp Leu Glu Asp Ala Val Gln Asn Ser Thr Ser Gly

Leu Ala Glu Arg
```

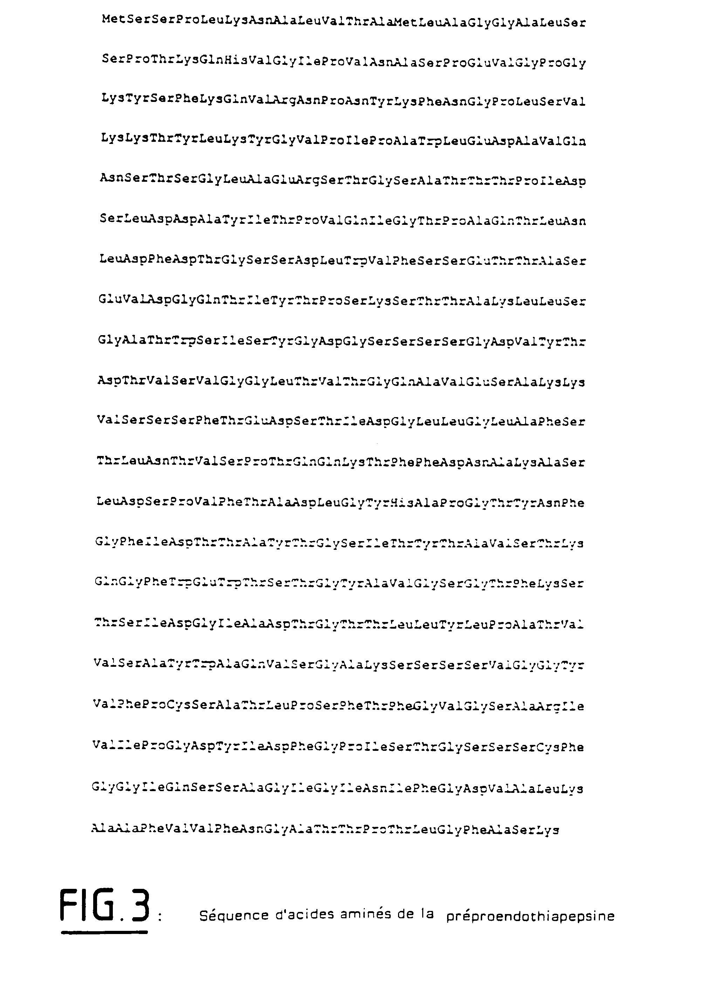

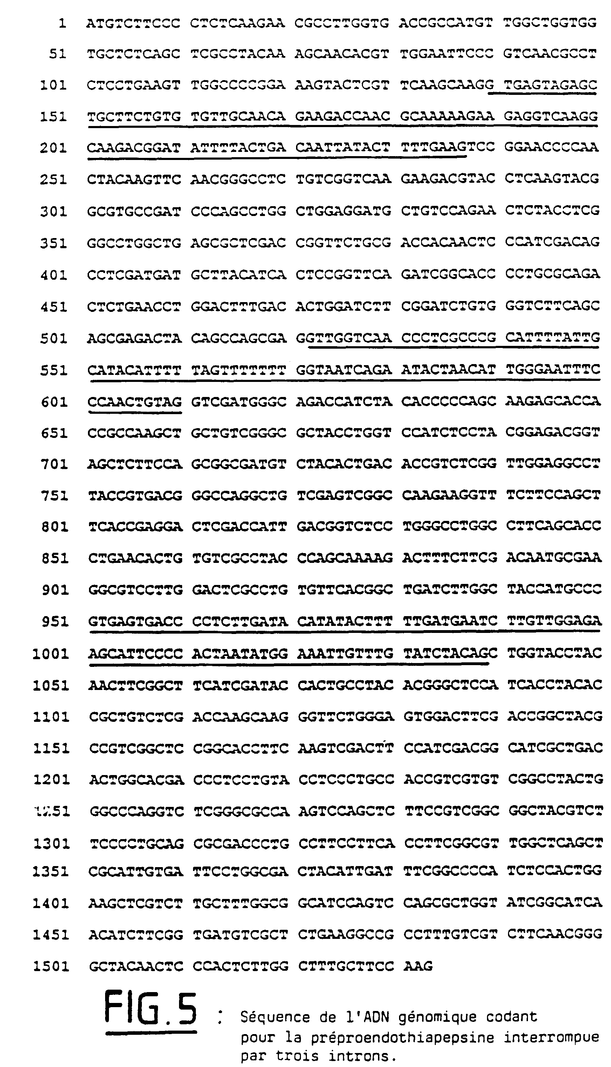

La séquence nucléotidique commençant au nucléotide 329 (figure 2) et se terminant au nucléotide 1861 (figure 2), interrompue par trois introns, est représentée sur la figure 5, les introns étant soulignés, code donc pour la préproendothiapsine, dont la séquence d'acides aminés est représentée sur la figure 3.

Le fragment A comprend de plus une séquence de 328 nucléotides en 5' de l'ATG (329-331) d'initiation et une séquence de 275 nucléotides en 3' du codon stop TAA (1862-1864), qui comporte plusieurs sites potentiels de polyadénylation.

La partie du fragment A en 5' de l'ATG d'initiation comprend d'une part l'extrémité 5' non traduite de l'ARN messager et d'autre part en amont de celle-ci une séquence TATAA (187-191), habituellement appelée boite TATA, séquence consensus présente dans la plupart des promoteurs des eucaryotes (Ballance D.J., 1986, Yeast, 2, 229-236). Par contre, cette partie ne comprend apparemment pas de séquence de type de celles habituellement appelées séquences d'activation amont (UAS : Upstream Activating Sequence) ou séquences de régulation amont (URS : Upstream Régulatory Sequence), qui sont présentes par exemple chez Saccharomyces (Guarente L., 1988, all, 52, 303-305) et chez Neurospora (Frederick G.D., 1990, Mol. Gen. Gent., 221, 148-154). Il n'y a donc pas dans le fragment A de région d'activation du promoteur en amont de la boîte TATA. Le promoteur n'est donc pas fonctionnel comme cela sera montré dans la section 6.

Section 2 : Isolement du fragment B, fragment d'ADN génomique d'environ 32,6 kb contenant la séquence codante du précurseur de l'endothiapepsine.

1) Préparation de l'ADN de C. parasitica.

L'ADN génomique de C. parasitica SEBR 103 a été préparé selon un protocole proche de celui décrit par B. TURCQ (Thèse d'Université : spécialité Sciences de la vie, soutenue le 6 janvier 1989 à l'Université de BORDEAUX II), résumé ci-après.

Préparation de protoplastes

Le mycélium provenant de 250 ml du bouillon de culture de la souche Cryphonectria parasitica SEBR 103, préparé comme décrit dans la section 1, est filtré sur de la gaze, puis rincé avec 50 ml de $MgSO_4$ 1M. Après incubation pendant 30 min à 37°C, on filtre à nouveau sur de la gaze et on reprend le mycélium dans 20 ml de $MgSO_4$ 1M. On ajoute alors 20 ml de $MgSO_4$ 1M contenant 10 mg/l du mélange enzymatique CAYLASE C3 (Société CAYLA) constitué de chiti-nases, de $\beta1,3$, $\beta1,6$, d'$\alpha1,3$ et $\alpha1,4$ glucanases ainsi que d'autres polysaccharidases, et on incube pendant 1 h 30 à 37°C sous faible agitation. Après filtration du mélange, le filtrat est centrifugé pendant 10 min à 3 000 g, puis le culot de protoplastes obtenu est repris dans 20 ml d'un tampon, appelé tampon ST, de composition : sorbitol 0,8 M, Tris HCl 100 mM, pH : 7,5.

Extraction de l'ADN génomique des protoplastes

Après une nouvelle centrifugation pendant 10 min à 3 000 g, le culot de protoplastes est repris avec 14 ml de tampon de lyse (Tris HCl 100 mM pH 9, EDTA 35 mM pH 8 ; SDS 4 % (poids/ volume) ; protéinase K (Sigma) 600 µg/ ml, puis le mélange est incubé pendant 1 h à 50°C. Après une centrifugation pendant 10 min à 12 000 g, le volume de surnageant est ajusté à 15,5 ml avec du tampon TE de composition Tris HCl 10 mM, pH 8 EDTA 1 mM, puis additionné de 19,53 g de CsCl. Après une ultracentrifugation (16 h à 50 000 tr/min dans un rotor vertical), le gradient est collecté par fractions qui sont dialysées contre du tampon de composition : Tris HCl 10 mM, pH 8 EDTA 1 mM, et analysées sur gel d'agarose 0,8 %. La fraction présentant un rapport spectrométrique entre l'absorption à 260 nm et l'absorption à 280 nm voisin de 1,8 a été conservée.

Construction de la banque cosmidique

Environ 10 µg d'ADN génomique de la fraction ci-dessus ont été soumis à une digestion partielle par l'enzyme de restriction MboI et ligués à l'aide de la ligase T4 au cosmide pHC79-ura 5 vecteur cosmidique d'environ 8 kb, construit en insérant le fragment EcoRI-EcoRI contenant le gène ura 5, (Begueret et al, 1984, Gene, 32 487-492) au site EcoRI du cosmide pHC79 disponible commercialement (commercialisé par BRL et construit par HOHN B. et al., 1980, Gene, 11, 291-298), le cosmide pHC79-ura 5 ayant été au préalable linéarisé par l'endonucléase BamHI et déphosphorylé à la phosphatase alcaline (Promega réf. CIP).

Le mélange de ligation a été empaqueté dans les particules phagiques à l'aide du kit "Gigapack plus" de Stratagene, et a servi à transformer la souche réceptrice E. coli LE 392 (Murray et al, 1977, Mol. gen. Genet. 150, p.53), disponible dans le commerce et distribuée par Genofit. Après étalement sur milieu gélosé LB (cf. tableau 4) additionné de 100 mg/l d'ampicilline.

Environ 4 500 clones résistants à l'ampicilline ainsi obtenus ont été repiqués individuellement sur des plaques de microtitration contenant du milieu liquide LB de composition précisée dans le tableau 4, mais sans agar, et conservés à -80°C. On a montré, par digestion de cosmides extraits de quelques 12 clones pris au hasard, que la taille moyenne des inserts de la banque était d'environ 37 kb.

2) Criblage de la banque par hybridation avec le fragment A

Le fragment A contenant le gène de structure de l'endothiapepsine a été utilisé comme sonde pour des expériences d'hybridation. Dans un premier temps, les clones contenus dans les plaques de microtitration ont été repiqués dans des boîtes de Petri contenant du milieu LB gélosé (cf. tableau 4) additionné d'ampicilline puis transférés sur des membranes de Nylon (Hybond N⁺, Amersham). Les bactéries ont alors été lysées à l'aide d'une solution contenant 1,5 M NaCl et 0,5 M NaOH. Après traitement avec une solution de protéinase K (Sigma) pendant 30 min à 37°C, les filtres sont lavés avec du 2 x SSC (NaCl 17,5 g/l, citrate de sodium 8,82 g/l pH 7) et préhybridés à 42°C pendant 20 min. Les filtres sont hybridés à 42°C pendant une nuit avec le fragment A isolé à l'exemple 1 marqué à la peroxydase de raifort (Amersham) et révélés avec le kit à sondes chemiluminescentes ECL "Système de détection de gène" (RPN 2101, Amersham). Les signaux d'hybridation obtenus sont visualisés sur un film approprié. Sur les 4 500 clones de la banque, 2 clones ont donné un signal positif. Ces deux clones, dénommés ci-après 8H12 et 41H7 et contenant respectivement les cosmides appelés p8H12 et p41H7, ont été repiqués sur milieu liquide LB contenant 100 mg/l d'ampicilline. Après une culture de nuit à 37°C, les cosmides sont extraits par la méthode de lyse en milieu alcalin, purifiés par ultracentrifugation au chlorure de césium et bromure d'éthidium selon les techniques décrites dans Maniatis, op. cit. Les cosmides ainsi purifiés ont été digérés par l'enzyme HindIII et les fragments obtenus ont été soumis à une électrophorèse sur gel d'agarose 0,8 %. Un Southern Blot sur une membrane en Nylon a été effectué et le filtre a été hybridé avec le fragment A à l'aide de la technique décrite précédemment. La présence de ce fragment dans chacun des cosmides p8H12 et p41H7 a ainsi été confirmée.

3) Analyse physique des cosmides positifs p8H12 et p41H7

On a constaté que les profils de restriction des cosmides p8H12 et p41H7, effectués à l'aide des enzymes NotI, SmaI, SfiI, XbaI, BamHI et PvuI, étaient identiques, ce qui indique que cette région de l'ADN génomique contenant le gène codant pour l'endothiapepsine de C. parasitica a été clonée sans réarrangement pour ces deux clones. Le profil de restriction pour le cosmide p8H12, cosmide retenu, pour la suite de l'étude, est donné dans le tableau 5 ci-après.

TABLEAU 5

| Profil de restriction du cosmide p8H12 | | | |
|---|---|---|---|
| Enzyme de restriction | Nombre de sites de coupure | Taille en kb | TOTAL en kb |
| NotI | 1 | non déterminée | - |
| SmaI | 2 | non déterminée | - |
| SfiI | 3 | 20 ; 16 ; 4,1 | 40,1 |
| XbaI | 4 | 23 ; 14 ; 2,9 ; 1,2 | 41,1 |
| BamHI | 5 | 15 ; 9 ; 7 ; 7 ; 2,6 | 40,6 |
| PvuI | 5 | 23 ; 7,4 ; 6,4 ; 2,1 ; 1,5 | 40,4 |
| | | Moyenne | 40,6 |

Ce profil permet de calculer la taille moyenne du cosmide p8H12, égale à environ 40,6 kb, donc celle de l'insert génomique égale à environ 40,6 - 8,0 = 32,6 kb. Cet insert génomique est appelé fragment B.

On a montré d'autre part, par Southern Blot sur membrane de Nylon, que le fragment BamHI-BamHI d'environ 9 kb (cf. tableau 5), appelé ci-après fragment C, était le seul fragment BamHI-BamHI à s'hybrider avec le fragment A utilisé comme sonde, donc qu'il contenait en totalité ce fragment.

Section 3 : Clonage du fragment C, fragment d'ADN génomique d'environ 9 kb contenant la séquence codante du précurseur de l'endothiapepsine.

10 µg du cosmide p8H12 ont été digérés par l'endonucléase BamHI et les différents fragments ont été séparés sur gel d'agarose 0,8 %.

Le produit de la digestion par l'enzyme BamHI contenant le fragment C a été ligué à l'aide de la ligase d'ADN T4 (Gibco-BRL) au plasmide pBR322 ouvert au site BamHI et déphosphorylé (commercialisé par Biolabs - réf. 320). Le produit de la ligation a servi à transformer des cellules compétentes de la souche E. coli K12 RR1 (Gibco-BRL- réf. 520-8261A). Après étalement du mélange de transformation sur des boîtes de Petri contenant du milieu LB gélosé additionné d'ampicilline (100 µg/ml) et incubation des boites à 37°C pendant 24 h, les colonies sont répliquées sur des membranes de Nylon ; les bactéries sont alors lysées puis les membranes sont hybridées avec le fragment A, comme décrit précédemment à la section 2.2). 18 colonies contenant de l'ADN s'hybridant avec le fragment A ont ainsi été mises en évidence. Leur contenu en ADN plasmidique a été extrait et analysé sur gel d'agarose 0,8 % après digestion par l'endonucléase BamHI. On a ainsi vérifié que toutes ces colonies contenaient un plasmide dérivé de pBR322 ayant inséré au site BamHI un fragment de 9 kb environ. On a choisi pour la suite de l'étude un clone appelé Ep1, contenant le plasmide dénommé pEp1. Le clone Ep1 a été déposé à la C.N.C.M. sous le n° I-998.

On a soumis le plasmide pEp1 à des digestions simples et/ou multiples à l'aide des enzymes BamHI, HindIII, PstI, SacI, SphI, BglII et ScaI. La carte de restriction obtenue est représentée sur la figure 4, les symboles B, H, P, Sp, C, G et S représentant respectivement BamHI, HindIII, PstI, SphI, SacI, BglII ainsi que ScaI, le codon d'initiation du gène de l'endothiapepsine de C. parasitica étant indiqué par un i et la flèche indiquant le sens de la transcription du gène de l'endothiapepsine. Le fragment A décrit dans la section 2, ainsi que les fragments D, E et F décrits dans les sections 4 et 5 sont également représentés sur cette figure.

Il apparaît que le site BamHI (B) qui forme l'extrémité en 5' du fragment C est situé à environ 4 kb du codon d'initiation et que le site BglII (G) en 5' est situé à environ 3 kb du codon d'initiation. Il semble donc intéressant d'isoler et de cloner le fragment BglII-BglII d'environ 5,1 kb contenant la totalité du fragment A, appelé fragment D, et qui contient vraisemblablement l'information nécessaire à l'expression du précurseur de l'endothiapepsine.

Section 4 : Clonage du fragment D, fragment d'ADN génomique d'environ 5,2 kb contenant la séquence codante du précurseur de l'endothiapepsine

20 µg du plasmide pEp1 ont été digérés par l'enzyme BglII (cf. figure 4) et les produits de la digestion ont été séparés sur gel d'agarose à bas point de fusion (Sigma - Réf. A9414) à 0,8 %. Après coloration du gel d'agarose au bromure d'éthidium, la bande d'agarose contenant le fragment D d'environ 5,2 kb est découpée au scalpel sous les ultraviolets à 310 nm. L'ADN est alors extrait suivant les instructions du kit NACS. 52PREPAC (Gibco-BRL) puis dilué dans 10 µl de tampon TE, de composition Tris-HCl 10 mM pH 8, EDTA 1 mM ; 1 µl de la suspension obtenue a été ligué à l'aide de la ligase d'ADN T4 (Gibco-BRL) au plasmide pBT6, dérivé du plasmide pBT3 par l'insertion d'un linker BglII au site SmaI du polylinker de pUC12. Le plasmide pBT3 décrit par Orbach M.J. et al, 1986, Mol. Cell. Biol., 6, 2452-2461, porte un gène muté de la β-tubuline de Neurospora crassa (champignon filamenteux ascomycète) conférant la résistance au bénomyl (marqueur de sélection dominant). Avant la ligation, le plasmide pBT6 a été linéarisé par l'endonucléase BglII et déphosphorylé à la phosphatase alcaline (Promega, réf. CIP-M204).

Le produit de la ligation a servi à transformer des cellules compétentes de la souche E. coli K12 RR1 (Gibco-BRL -Réf. 530-8261SA). Après étalement du mélange de transformation sur des boîtes de Petri contenant du milieu LB gélosé additionné d'ampicilline (100 µg/ml) et incubation des boîtes à 37°C pendant 24 h, les colonies sont répliquées sur des membranes de Nylon. Les bactéries sont alors lysées, puis les membranes sont hybridées avec le fragment A, comme décrit dans la Section 2.2. Deux colonies contenant de l'ADN s'hybridant avec le fragment A ont ainsi été mises en évidence. Leur contenu en ADN plasmidique a été extrait et analysé sur gel d'agarose 0,8 % après digestion par l'endonucléase BglII. On a ainsi vérifié que ces deux colonies contenaient un plasmide dérivé de pBT6 ayant inséré dans les deux orientations possibles au site BglII le fragment D de 5,2 kb environ. On a choisi pour la suite de l'étude un clone contenant ce plasmide, dénommé plasmide pEp2.

Section 5 : Clonage du fragment F, fragment d'ADN génomique d'environ 3,5 kb contenant la séquence codante du précurseur de l'endothiapepsine.

Le clonage a été fait en deux étapes. D'abord, le fragment E d'environ 3,7 kb contenant la totalité du fragment F a été cloné dans le plasmide pUC18 au site SphI du polylinker. Le plasmide pEp3 ainsi obtenu a permis de purifier le fragment F qui a alors été sous-cloné à son tour dans le plasmide pUC18 au site BamHI du polylinker. On a ainsi obtenu le plasmide pEp4.

**1/ Construction du plasmide pEp3**

1 μg du plasmide pEp1 a été digéré par l'enzyme SphI (cf. figure 4) puis l'ADN a été purifié avec 0,1 volume d'acétate de sodium 3M et 2 volumes d'éthanol. On a ainsi obtenu un fragment SphI d'environ 3,7 kb, appelé fragment E. L'ADN a alors été dissous dans 40 μl de tampon TE de composition (Tris-HCl 10 mM pH 8, EDTA 1 mM) puis dialysé sur colonne P10 (Pharmacia). On a alors ligué à l'aide de la ligase d'ADN T4 (Gibco-BRL) 1 μl du mélange obtenu ci-dessus avec environ 25 ng du plasmide pUC18 préalablement linéarisé par l'endonucléase SphI et déphosphorylé à la phosphatase alcaline (Promega, réf CIP-M204). Le produit de la ligation a servi à tansformer des cellules compétentes de la souche E. coli DH5α (Gibco-BR1, Réf. 530-8263 SA). Après étalement du mélange de transformation sur des boîtes de Petri, contenant du milieu LB gélosé additionné d'ampicilline (100 μg/ml) de Xgal (40 μg/ml) et d'IPTG (2 μg/ml) et incubation des boîtes à 37°C pendant 24 h, 350 colonies blanches ont été repiquées sur le même milieu. Les colonies ont alors été répliquées sur des membranes de Nylon puis les bactéries ont alors été lysées et enfin les membranes ont été hybridées avec le fragment A, comme décrit précédemment dans la section 2.2. 37 colonies contenant de l'ADN s'hybridant avec le fragment A ont été ainsi mises en évidence.

L'ADN plasmidique de 30 clones a été extrait et analysé sur gel d'agarose 0,8 %, après digestion par l'endonucléase SphI. Deux colonies contenant un plasmide dérivé de pUC18 ayant inséré (dans les deux orientations possibles) au site SphI le fragment E de 3,7 kb ont été conservées. Ces plasmides ont été appelés pEp3 (a) et pEp3 (b). Dans le plasmide pEp3 (b), le site bglIII du fragment E est distant d'environ 3,5 kb du site BamHI situé sur le polylinker de pUC18.

**2/ Construction du plasmide pEp4**

10 μg du plasmide pEp3 (b) ont été soumis à trois digestions successives par les endonucléases BglII, BamHI et PvuI et les produits de la digestion ont été séparés sur gel d'agarose à 0,8 %. Après coloration du gel au bromure d'éthidium, la bande d'agarose contenant le fragment SphI d'environ 3,5 kb, dénommé fragment E, additionné d'un fragment SphI-BamHI du polylinker de pUC18, a été découpée au scalpel sous les ultraviolets à 310 nm. L'ADN est alors extrait puis dissous dans 20 μl de tampon TE de composition (Tris-HCl 10 mM pH 8, EDTA 1 mM). 5 μl de la suspension obtenue ont été ligués à environ 750 ng du plasmide pUC18 préalablement linéarisé par l'endonucléase BamHI et déphosphorylés à la phosphatase alcaline. Le produit de la ligation a servi à transformer des cellules compétentes de la souche E. coli DH5α suivant le protocole décrit ci-dessus. L'ADN plasmidique de 30 colonies blanches a été extrait et analysé sur gel d'agarose 0,8 % après digestion par les endonucléases BamHI, BglII ou SmaI. Une colonie a été retenue dans laquelle est contenu un plasmide dérivé de pUC18 ayant inséré au site BamHI le fragment F d'environ 3,5 kb additionné d'un fragment SphI-BamHI du polylinker de pUC18. Ce plasmide a été dénommé pEp4.

**3/ Détermination de la séquence du segment SphI-HindIII du segment F**

Cette séquence, déterminée comme précédemment (cf. section 1), est représentée dans la figure 12. Elle contient des signaux d'activation du promoteur du gène codant pour la préproendothiapepsine, comme il sera montré dans la section 10.

Section 6 : Transformation de C. parasitica par chacun des vecteurs contenant l'un des fragments A, B et C

Préparation de protoplastes

Le mycélium provenant de 250 ml du bouillon de culture de la souche C. parasitica SEBR 103, préparé comme décrit à l'exemple 1, est rincé avec 50 ml de MgSO$_4$ 1M. Après incubation pendant 30 min à 37°C, on filtre sur de la gaze et on reprend le mycélium dans 20 ml de MgSO$_4$ 1M. On ajoute alors 20 ml de MgSO$_4$ 1M contenant 10 mg/l du mélange enzymatique CAYLASE C3 (Société CAYLA) constitué de chitinases, de β1,3, β1,6, d'α1,3 et α1,4 glucanases ainsi que d'autres polysaccharidases, et on incube pendant 1 h 30 à 37°C sous faible agitation. Après filtration du mélange, le filtrat est centrifugé pendant 10 min à 3 000 g, puis le culot de protoplastes obtenu est repris dans 15 ml du tampon, appelé tampon ST, de composition : sorbitol 0,8 M, Tris HCl 100 mM, pH : 7,5. Après une nouvelle centrifugation pendant 10 min à 3 000 g, le culot est repris dans 10 ml d'un tampon, appelé tampon STC10, de composition : sorbitol 0,8 M, Tris ; HCl 100 mM pH : 7,5, CaCl$_2$ 10 mM. Les protoplastes sont alors dénombrés à l'aide d'une cellule Malassez de façon à ajuster leur concentration à 10$^8$/ml après centrifugation pendant 10 min à 3 000 g et reprise du culot dans un tampon, appelé tampon STC50, de composition : sorbitol 0,8 M, Tris HCl 100 mM pH : 7,5, CaCl$_2$ 50 mM.

Cotransformation des protoplastes par le cosmide p8H12 et le plasmide pBT3

Le cosmide p8H12 qui contient le fragment B (cf. section 2) ne porte pas de marqueur de sélection dominant (utilisable pour la sélection directe). Le plasmide pBT3 décrit par Orbach M.J. et al, 1986, Mol. Cell. Biol., 6, 2452-2461, qui porte un gène muté de la β-tubuline de Neurospora crassa (champignon filamenteux ascomycète) conférant la résistance au bénomyl (marqueur de sélection dominant) a donc été utilisé conjointement avec le cosmide p8H12 (méthode de cotransformation : voir les travaux sur la cotransformation d'Aspergillus niger de Wernars K. et al, 1987, Mol. Gen. Genet., 209, 71-77).

Un mélange composé de 1 µg du plasmide pBT3, préalablement purifié par ultracentrifugation dans un tampon contenant du chlorure de césium et du bromure d'éthidium selon les techniques décrites dans Maniatis, op. cit., et de 4 µg du cosmide p8H12, purifié de la même façon, dans 10 ml de tampon TE, de composition (Tris HCl 10 mM pH8, EDTA 1 mM), est incubé pendant 20 min à 0°C avec 100 ml de la préparation de protoplastes préparée ci-dessus (soit 10$^7$ protoplastes). Après addition de 1 ml d'une solution constituée de 60 % (poids/volume) de PEG 4000 (polyéthylèneglycol de masse moléculaire 4000) et du tampon de composition : Tris-HCl 20 mM pH 7,5 ; CaCl$_2$ 100 mM, et incubation du mélange pendant 10 min à température ambiante, on mélange avec ce dernier 1 ml du tampon STC10 (défini précédemment). Les protoplastes ainsi traités sont inclus dans 60 ml de milieu gélosé contenant 1 mg/l de Benlate (antifongique commercialisé par Dupont Nemours), appelé milieu D, dont la composition est précisée dans le tableau 6 ci-après, maintenu en surfusion à 45°C. Le mélange en surfusion est étalé sur des boîtes de Petri contenant un milieu gélosé additionné de 1 mg/l de Benlate (antifongique commercialisé par Dupont Nemours qui contient 50 % de bénomyl), appelé milieu C et dont la composition est précisée dans le tableau 7 ci-après. Les boîtes de Petri sont incubées à 28°C pendant le temps nécessaire à l'apparition de protoplastes régénérés résistants au bénomyl. Les transformants régénérés ainsi obtenus sont appelés 29Pn, n désignant le numéro du clone considéré. La résistance au bénomyl des clones est confirmée par repiquage d'implants mycéliens de chaque clone sur le milieu B rendu gélosé par l'addition de 20 g/l d'agar et additionné de 1 mg/l de Benlate.

Seuls les clones se développant sur ce milieu sont mis en sporulation suivant la méthode décrite à l'exemple 1) 1. Les conidiospores obtenues étant récoltées en masse dans un tampon contenant au moins 15 % du glycérol et conservées à -80°C.

TABLEAU 6

| Composition du milieu D | |
|---|---|
| Saccharose | 250 g/l |
| Glucose | 20 g/l |
| Thiamine | 2 g/l |
| Asparagine | 100 mg/l |
| Extrait de malt | 0,2 g/l |
| Agar | 20 g/l |
| Solution saline 1 (composition précisée dans le tableau 2) | 62,5 ml |
| Ajuster le pH à 6,0 à l'aide de HCl 1N ou NaOH 1N, Autoclaver 30 min à 110°C, puis Ajouter 1 mg/l de Benlate au milieu refroidi à 60 °C | |

TABLEAU 7

| Composition du milieu C | |
|---|---|
| Milieu D | 750 ml/l |
| Tampon STC10 (de composition : sorbitol 0,8 M, tris HCl 100 mM pH 7,5, CaCl$_2$ 10 mM) | 250 ml/l |

TABLEAU 7   (suite)

| Composition du milieu C |
| --- |
| Ajuster le pH à 6,0 |
| Ajouter 1 mg/l de Benlate au milieu autoclavé à 60 °C |

<u>Cotransformation des protoplastes par le plasmide p472 et le plasmide pBT3 d'une part, ainsi que par le plasmide pEp1 et le plasmide pBT3 d'autre part</u> :

Le plasmide p472 qui contient le fragment A (cf. section 1) et le plasmide pEp1 qui contient le fragment C (cf. section 3), ne portent pas non plus de marqueur de sélection dominant.

La souche <u>C. parasitica</u> SEBR 103 a été cotransformée selon un protocole identique à celui décrit dans le paragraphe précédent, avec les mélanges de plasmides suivants : 4 µg de pEp1 et 1 µg de pBT3, 4 µg de p472 et 1 µg de pBT3. Les transformants ainsi obtenus sont appelés 30Pn pour la cotransformation par les plasmides pEp1 et pBT3, et 31Pn pour la cotransformation par les plasmides p472 et pBT3, n désignant le numéro du clone considéré.

Section 7 : Sélection de souches transformées surproductrices d'endothiapepsine.

1) <u>Méthode générale</u>

a) Sélection sur milieu gélosé contenant de la caséine.

Des implants mycéliens d'environ 100 colonies résistantes au bénomyl ont été repiqués sur un milieu gélosé contenant de la caséine, appelé milieu E, dont la composition est précisée dans le tableau 8 ci-après. Sur ce milieu, les colonies de <u>Cryphonectria parasitica</u> qui produisent la protéase donnent naissance à un halo de précipitation dont la surface est proportionnelle à la quantité d'endothiapepsine sécrétée.

Les souches surproductrices sont retenues sur la base d'un rapport entre le diamètre de halo de précipitation et le diamètre de la colonie, significativement plus élevé que celui de la souche témoin non transformée. Une préparation de conidiospores de ces souches surproductrices est effectuée selon la méthode utilisée dans la section 1.1). De plus, on a vérifié par l'addition au milieu E de 5 µg/ml de pepstatine, substance inhibitrice spécifique des aspartyl-protéases, que l'accroissement des halos observé chez les souches surproductrices était réduit. Ce résultat indique qu'il s'agit bien d'une surproduction d'une aspartyl-protéase.

b) Sélection en milieu liquide.

α) <u>Etude en fioles</u> :

Pour confirmer ce résultat, des essais de production en fioles ont été réalisés de la façon suivante : ensemencement des fioles de 250 ml contenant 40 ml de milieu F dont la composition est précisée ci-après. On incube ensuite les fioles à 28°C sur un agitateur rotatif excentrique réglé à 220 tr/min pendant 48 h. Pour chaque souche, les cultures ont été réalisées dans 3 fioles différentes et la moyenne des résultats de dosage d'activité coagulante pour les 3 fioles a été calculée. Le témoin est constitué par la souche <u>Cryphonectria parasitica</u> SEBR 103 non transformée. Le dosage de l'activité coagulante est réalisé selon la méthode officielle de détermination de la teneur en enzyme des solutions coagulantes publiée au Journal Officiel de la République Française du 20 mars 1981 (paragraphe C), résumée ci-après :

- 1 ml de surnageant de culture dilué avec de l'eau de façon à avoir un temps de coagulation compris entre 5 et 10 min est ajouté à 10 ml de lait standardisé (fourni par l'INRA - Station Expérimentale Laitière - 39800 POLIGNY), placé dans un flacon approprié ;

- on mesure le temps de coagulation repéré par l'apparition d'une floculation du lait sur la paroi du flacon en rotation dans un bain-marie à 30°C ;

- l'activité coagulante appelée AC, exprimée en mg/l, est donnée par la formule :

$$AC = \frac{K}{T\text{-}a} \times \alpha$$

avec K et a facteurs dépendant du lait et de l'enzyme considérés (exprimés respectivement en mgs/l et en s).

T : temps de coagulation (exprimé en secondes)
α : facteur de dilution.

β) Etude en fermenteur :

La production d'endothiapepsine a été évaluée en fermenteur de 2 l (Biolaffite) contenant 1,2 l d'un milieu de culture obtenu par concentration du milieu F, stérilisé par autoclavage pendant 45 min à 120°C. Les conditions de culture sont les suivantes : agitation à 800 tr/min ; aération : 2 vvm (vvm : volume d'air par volume de milieu par min). Le fermenteur est ensemencé à 5 % (v/v) avec une préculture en fiole telle que décrite ci-dessus (en α). La température est maintenue à 28°C. La mesure de l'activité coagulante est effectuée après environ 90 h de fermentation suivant la méthode de dosage précédemment décrite.

On a vérifié par détermination du poids sec de la culture que la quantité de biomasse produite par les transformants surproducteurs ne diffère pas significativement de celle produite par la souche témoin.

TABLEAU 8

| Composition du milieu E | |
|---|---|
| $KH_2PO_4$ | 0,36 g/l |
| $Na_2HPO_4$, $2H_2O$ | 0,71 g/l |
| $MgSO_4$, $7H_2O$ | 0,50 g/l |
| NaCl | 0,10 g/l |
| Hydrolysat de caséine (casamino-acids de Difco) | 0,05 g/l |
| Caséine (Hammarsten) | 6,0 g/l |
| $CaCl_2$ | 0,06 g/l |
| Solution saline 2 | 10 ml/l |
| (composition précisée dans le tableau 2 de l'exemple 2) | |
| Laisser sous agitation 15 min de façon à éviter la formation de mousse. | |
| Ajouter 15 g d'Agar (Bacto-agar de Difco). | |
| Ajuster le pH à 6,2 à l'aide de HCl 1N ou NaOH 1N. | |
| Autoclaver 20 min à 120°C. | |

TABLEAU 9

| Composition du milieu F | |
|---|---|
| Farine de germes de coton | 10 g/l |
| Glucose | 35 g/l |
| $Ca(NO_3)_2$ | 3,5 g/l |
| $CaCO_3$ | 0,75 g/l |
| Huile de lin | 2,5 ml/l |
| Oléate de sodium | 1,5 g/l |
| Ajuster le pH à 6,20 à l'aide de HCl 1N ou NaOH 1N. | |
| Autoclaver 45 min à 120°C. | |

Analyse de l'enzyme sécrétée par les transformants :

Le moût de fermentation obtenu après culture (soit en fioles, soit en fermenteurs) des transformants surproducteurs et de la souche témoin C. parasitica SEBR 103 non transformée a été soumis à une centrifugation de façon à éliminer la masse mycélienne. Après dénaturation des protéines du surnageant en présence de SDS pendant 5 min à 100°C, on a effectué une électrophorèse sur gel de polyacrylamide en présence de SDS. On observe, après coloration au bleu de Coomassie, une bande majoritaire de masse moléculaire voisine de 36 kDa, qui correspond à la masse moléculaire de l'endothiapepsine mature déduite de sa séquence (cf. figure 1), de coloration plus intense pour les transformants surproducteurs que pour la souche témoin ; et des bandes minoritaires identiques pour les transformants surproducteurs et la souche témoin.

On a vérifié, d'autre part, par réaction antigène-anti-corps (kit Rennetest, France Biochem) sur les surnageants de culture des transformants surproducteurs et de la souche témoin non transformée, que l'enzyme sécrétée est identique à celle de Cryphonectria parasitica conformément à la méthode d'identification décrite dans le Journal Officiel de la République Française du 20 mars 1981.

De plus, on a évalué le rapport entre l'activité coagulante et l'activité protéolytique de l'enzyme sécrétée. L'activité coagulante exprimée en g/l est mesurée suivant la méthode décrite précédemment au paragraphe b) α) ; l'activité protéolytique exprimée en milliéquivalent glycine par litre est mesurée à l'aide du réactif TNBS, décrit par R. Fields, Biochem. J. (1971) 124 : 581-590, par dosage des groupements aminés apparus après prétéolyse de la diméthylcaséine.

Ce rapport est compris entre 0,045 et 0,050 pour les transformants surproducteurs, ce qui est très proche de celui obtenu avec la souche témoin non transformée. On peut conclure de ces trois études que c'est bien la production d'endothiapepsine qui a été spécifiquement augmentée chez les transformants surproducteurs.

c) Vérification de l'intégration du fragment A, B ou C par Southern Blot.

L'ADN génomique des transformants sélectionnés est préparé selon un protocole proche de celui décrit par Raeder et Broda, 1965, Letters in Applied Microbiology 1, 17-20, résumé ci-après :

Le mycélium provenant d'une culture en fioles, effectuée comme décrit dans la section 1.1), est lyophilisé et filtré sur gaze. Après rinçage dans une solution 20 mM EDTA pH 8, le mycélium est filtré à nouveau puis congelé à -80°C et lyophilisé. 50 mg du lyophilisat obtenu sont alors broyés puis remis en suspension dans 500 µl du tampon d'extraction suivant : Tris HCl 200 mM, pH 8,5, NaCl 250 mM, EDTA 25 mM, SDS 0,5 %, protéinase K (Sigma) 200 µg/ml. Le tout est incubé pendant 45 min à 45°C, puis l'ADN est extrait au phénol chloroforme, puis précipité à l'isopropanol. Le culot est alors repris dans 100 µl de TE (Tris HCl 10 mM pH 8, EDTA 1 mM).

L'ADN est ensuite digéré par les endonucléases appropriées et les fragments obtenus sont séparés par électrophorèse sur gel d'agarose 0,8 %. Les fragments sont transférés par capillarité sur membrane de Nylon (Hybond N+ - Amersham) selon la méthode préconisée par le fabricant. Les filtres sont alors hybridés à 42°C pendant une nuit avec successivement le fragment A et le fragment HindIII-HindIII contenant le gène muté de la β tubuline de Neurospora du plasmide pBT3, marqués à la peroxydase de raifort (Amersham et révélés avec le kit à sondes chemiluminescentes ECL "Système de détection de gène" (RPN 2101, Amersham). Les signaux d'hybridation obtenus sont visualisés sur un film approprié.

2) Sélection de surproducteurs parmi les transformants 29Pn (contenant le cosmide p8H12 qui porte le fragment B - cf. section 6)

164 colonies résistantes au bénomyl ont été obtenues lors de la cotransformation par le cosmide p8H12 et le plasmide pBT3. 127 clones ont été choisis au hasard parmi ces dernières pour le test de sélection sur milieu gélosé contenant de la caséine, lequel a permis de retenir 14 clones. Parmi ces derniers, 7 choisis au hasard ont été soumis au test de sélection en milieu liquide. On a ainsi retenu 3 clones ; les clones 29P1, 29P2 et 29P3 présentant après culture en fioles une activité coagulante respectivement de 0,9, 1,07 et 0,94 g/l alors que la souche témoin non transformée présente une activité de 0,62 g/l (le facteur de surproduction : rapport entre l'activité coagulante de la souche surproductrice et l'activité coagulante de la souche témoin est donc respectivement 1,45, 1,73 et 1,54). Les clones 29P2 et 29P3 ont alors été testés en fermenteur 2 l et on produit respectivement une activité coagulante de 1,76 et 2,19 g/l, la souche témoin ne produisant que 1,20 g/l (le facteur de surproduction est donc de + 1,47 et + 1,83).

Le clone 29P3 et la souche témoin C. parasitica SEBR 103 ont été cultivés dans un fermenteur de 20 l dans des conditions expérimentales moins limitantes en particulier en ce qui concerne l'agitation, l'aération et le transfert de masse, et proches des conditions utilisées dans le procédé industriel.

Cet essai a permis d'obtenir une quantité d'endothiapepsine égale à environ 2 fois la quantité produite par la souche témoin.

Les 7 clones précédemment choisis au hasard ont été analysés par Southern Blot. Leur ADN génomique et celui de la souche témoin C. parasitica SEBR 103 non transformée ont été digérés par l'enzyme Smal qui génère seulement deux sites de coupure dans le cosmide p8H12 et aucun dans le fragment A. Après hybridation avec ce dernier, on a observé deux bandes d'hybridation avec les clones 29P1, 29P2, 29P3 et une seule bande d'hybridation, de taille identique à l'une des deux bandes précédemment citées, avec la souche témoin et les autres clones non retenus après le test de sélection en milieu liquide. Les trois clones surproducteurs 29P1, 29P2, 29P3 ont donc intégré une copie du cosmide p8H12 à un locus différent de leur génome car les bandes surnuméraires observées sont de taille différente.

EP 0 475 842 B1

Enfin, le Southern Blot obtenu ci-dessus a été hybridé avec le fragment HindIII du plasmide pBT3 qui confère la résistance au bénomyl, ce qui a permis de constater que les 7 clones résistants au bénomyl ont tous reçu au moins une copie du plasmide pBT3, car ils présentent plusieurs bandes d'hybridation supplémentaires par rapport à la souche témoin, celle-ci ne présentant qu'une bande correspondant au gène de la β tubuline endogène (la β tubuline est une protéine structurale présente chez les champignons filamenteux, en particulier Neurospora crassa et C. parasitica).

Ces résultats montrent que le fragment B contient donc les signaux nécessaires à l'expression (et la sécrétion) de l'endothiapepsine. Il contient donc un gène fonctionnel de l'endothiapepsine c'est-à-dire une séquence codant pour un précurseur de l'endothiapepsine encadrée par un promoteur et un terminateur fonctionnels. Ce promoteur fonctionnel comprend donc une région activatrice située en amont de la boîte TATA localisée dans le fragment A (cf. section 1).

L'addition d'une copie supplémentaire de ce fragment au génome de la souche C. parasitica SEBR 103 par transformation permet d'obtenir des souches transformées qui surproduisent l'endothiapepsine avec un facteur voisin de 2.

3) Sélection de surproducteurs parmi les transformants 30Pn (contenant le plasmide pEp1 qui porte le fragment C : cf. section 6)

663 colonies résistantes au bénomyl ont été obtenues lors de la cotransformation par le plasmide pEp1 et le plasmide pBT3. 108 clones ont été choisis au hasard parmi ces dernières pour le test de sélection en milieu gélosé contenant de la caséine, lequel a permis de retenir 32 clones. On remarquera que ce test a permis de retenir un taux de surproducteurs parmi les transformants 30Pn nettement plus élevé (32/108) que celui obtenu pour les transformants 29Pn (14/127). Ceci indique que la fréquence de cotransformation obtenue avec le plasmide pEp1 d'environ 13,4 kb est plus grande que celle obtenue avec le cosmide p8H12 d'environ 40,6 kb.

Parmi ces 32 clones, 12 clones choisis au hasard ont été soumis au test de sélection en milieu liquide. On a ainsi retenu 7 clones : les clones $30P_1$, $30P_2$, $30P_3$, $30P_4$, $30P_5$, $30P_6$, $30P_7$ présentant après culture en fioles une activité coagulante respectivement de 1,12 ; 1,06 ; 0,96 ; 1,12 ; 1,05 ; 0,88 et 0,90 g/l, alors que la souche témoin non transformée présente une activité de 0,62 g/l (le facteur de surproduction est donc compris entre + 1,42 et + 1,81).

Les clones $30P_1$, $30P_2$, $30P_5$ ont alors été testés dans un fermenteur de 2 l et ont produit une activité coagulante respectivement de 3,05, 3,0 et 3,04 g/l, la souche témoin ne produisant que 1,20 g/l (le facteur de surproduction est donc respectivement de + 2,54, + 2,50 et + 2,53).

Il est probable (cf. 2 ci-dessus) que, dans les conditions expérimentales non limitantes du fermenteur de 20 l, il serait possible d'atteindre un facteur de surproduction d'environ 3.

Les 7 clones précédemment choisis au hasard ont été analysés par Southern Blot. Leurs ADN génomiques et celui de la souche témoin : C. parasitica SEBR 103 non transformée, ont été digérés par l'enzyme SacI, choisie car elle ne génère qu'un seul site de coupure dans le plasmide pEp1, site localisé dans le fragment C en dehors du fragment A (cf. figure 4). Après hybridation pour chacun des 7 clones avec le fragment A, on a observé au moins deux bandes d'hybridation (2 à selon le clone), dont la bande du gène endogène de l'endothiapepsine présente également pour la souche témoin, le profil étant différent pour chacun des clones. Pour les clones $30P_1$, $30P_2$, $30P_5$, on observe parmi celles-ci une bande d'hybridation de taille identique à celle du plasmide pEp1 linéarisé, ce qui indique l'intégration en tandem probable d'au moins deux copies supplémentaires de ce plasmide.

Il est en effet bien connu de l'homme de l'art que l'intégration en tandem de plusieurs copies d'un plasmide après transformation est un événement fréquent chez les champignons filamenteux (Fincham J.R.S., mars 1989, Microbiological Reviews, 148-170) et que la digestion de l'ADN génomique dans lequel le plasmide s'est ainsi intégré, par une endonucléase ne générant qu'un seul site de coupure dans ce plasmide, libère ce plasmide.

Ces résultats montrent que le fragment C contient tous les signaux nécessaires à l'expression (et la sécrétion) de l'endothiapepsine. Il contient donc un gène fonctionnel de l'endothiapepsine, donc un promoteur complet fonctionnel. La région d'activation du promoteur est donc située dans le fragment C.

L'addition d'au moins deux copies supplémentaires de ce fragment au génome de la souche C. parasitica SEBR 103 permet d'obtenir des souches transformées qui surproduisent l'endothiapepsine avec un facteur voisin de 3.

4) Sélection de surproducteurs parmi les transformants 31Pn (contenant le plasmide p472 qui porte le fragment A : cf. section 6)

840 colonies résistantes au bénomyl ont été obtenues lors de la cotransformation par le plasmide p472 et le plasmide pBT3. 108 clones ont été choisis au hasard pour le test de sélection sur milieu gélosé contenant de la caséine. Contrairement aux résultats obtenus précédemment avec les cotransformants 29Pn et 30Pn, aucun surproducteur n'a pu être mis en évidence. Néanmoins, 5 clones $30P_1$, $30P_2$, $30P_3$, $30P_4$, $30P_5$, choisis parmi ceux donnant un halo d'hydrolyse de grande taille mais non significativement supérieure à celle du halo de la souche témoin non transformée, ont été soumis au test de sélection en milieu liquide. Les clones précités présentent respectivement, après culture en fioles, une activité coagulante de 0,38 ; 0,66 ; 0,66 ; 0,56 et 0,65 g/l alors que la souche témoin présente une activité de 0,62 g/l (la différence constatée est donc de -38, +6, +6, -22 et +4 %). Aucune surproduction significative n'est mise en évidence par ce test en milieu liquide, ce qui confirme le résultat négatif du test de sélection en milieu gélosé caséine.

L'analyse par Southern Blot a montré que les 5 clones ont intégré au moins une copie du plasmide pBT3 et 3 clones sur 5 sont réellement cotransformés par le plasmide pBT3 et le plasmide p472. L'addition d'une copie supplémentaire du fragment A contenu dans le plasmide p472 ne conduit donc pas à une surproduction d'endothiapepsine, ce qui indique que des séquences d'ADN essentielles pour l'expression de l'endothiapepsine font défaut sur ce fragment. Le fragment A ne contient donc pas de gène fonctionnel de l'endothiapepsine, ce qui confirme qu'il manque au fragment A une région d'activation amont du promoteur nécessaire pour rendre celui-ci fonctionnel (cf. sections 1-7).

La région d'activation amont du promoteur est donc située dans le fragment C entre son extrémité 5' (site BamHI) et celle 5' (site HindIII) du fragment A qu'il contient.

Une localisation plus précise de cette région activatrice pourra être déterminée par l'obtention d'une série de sous-fragments du fragment C (préparés par exemple par digestion à l'aide d'endonucléases ou d'exonucléases) comprenant le fragment A flanqué en 5' de segments de différentes tailles de la partie du fragment C comprise entre l'extrémité 5' du fragment A et un nucléotide situé entre l'extrémité 5' du fragment A et l'extrémité 5' du fragment C, transformation de Cryphonectria parasitica SEBR 103 par ces sous-fragments et sélection des transformés exprimant la protéase recombinante. Des exemples de tels sous-fragments C sont les fragments D et F, dont la préparation est décrite dans les sections 4 et 5.

Section 8 : Méthode de purification (élimination du marqueur de sélection) d'un transformant surproducteur en endothiapepsine et d'amplification du gène codant pour l'endothiapepsine par transformations successives

1) Généralités :

Il est connu (cf. notamment Fincham J.R.S., mars 1989, Microbiological Reviews, 148-170) que les cellules fongiques comprennent plusieurs noyaux, contenant en général le même matériel génétique. Les protoplastes obtenus après digestion enzymatique de leurs parois peuvent être anucléés (incapables de se régénérer), uninucléés ou polynucléés. Après transformation de ces derniers, on peut donc obtenir des cellules transformées de type hétérocaryons, contenant des noyaux transformés, éventuellement de différentes sortes (selon le mode d'intégration et la nature du matériel intégré, qui peuvent varier) et des noyaux non transformés. Dans le cas de C. parasitica les conidiospores sont uninucléées (Puhalla J.E. et al, Phytopathology, 1971, 61, 169-173).

Les travaux décrits ci-après utilisent ces caractéristiques des cellules fongiques pour construire des souches contenant uniquement l'ADN recombinant d'intérêt et non le marqueur de sélection.

2) Purification du transformant 29P3 en vue de la recherche de surproducteurs bénomyl-sensibles :

Une préparation de conidiospores du transformant initial surproducteur d'endothiapepsine 29P3 (cf. section 1.1) et section 7.2)), suffisamment diluée pour obtenir des colonies isolées, a servi à ensemencer des boîtes de Petri contenant du milieu B (cf. tableau 2 ci-dessus) rendu gélosé par l'addition de 20 g/l d'agar. Après incubation pendant 5 jours à 30°C, des implants mycéliens de 50 colonies ont été repiqués en parallèle sur le même milieu B gélosé et sur ce dernier additionné de 1 μg/ml de Benlate (contenant 50 % de bénomyl). Après incubation pendant 5 jours à 30°C, 6 clones présentent une croissance normale sur le milieu B gélosé et une croissance nulle sur le milieu B gélosé additionné de Benlate. Ces 6 clones bénomyl-sensibles, issus chacun de la germination d'une spore, sont de type homocaryon, donc purs.

On a soumis les 6 clones et le transformant initial 29P3 au test de sélection de surproduction d'endothiapepsine sur milieu gélosé contenant de la caséine (cf. section 7.1) a)), ce qui a permis de sélectionner un clone bénomyl-sensible appelé 29P3 ben[s] qui présente une surproduction non significativement différente de celle du transformant initial. On a soumis le clone 29P3 ben[s], le clone 29P3 et la souche témoin C. parasitica SEBR 103, après sporulation, au test de sélection en milieu liquide. Les clones 29P3 et 29P3 ben[s] produisent une activité coagulante identique (compte tenu de la marge d'erreur expérimentale), mais nettement supérieure à celle de la souche témoin. De plus, un Southern Blot, effectué sur ces deux clones et la souche témoin, après digestion de leur ADN génomique par l'endonucléase SmaI, montre pour chacun des deux clones le même profil d'hybridation avec le fragment A comme sonce, lequel comporte une bande surnuméraire par rapport au témoin (cf. section 7.2)), ce qui indique que dans les deux clones l'intégration du gène fonctionnel de l'endothiapepsine est identique, et un profil d'hybridation différent avec le fragment HindIII-HindIII du plasmide pBT3 qui confère la résistance au bénomyl (cf. section 6), le transformant initial 29P3 présentant plusieurs bandes d'hybridation avec ce fragment et le clone 29P3 ben[s] ainsi que la souche témoin présentant une seule bande d'hybridation de taille identique (correspondant au gène endogène de la β turbuline).

Ces résultats montrent que la méthode de purification a permis d'obtenir un transformant pur (en ce qui concerne son génotype), dépourvu du marqueur de sélection (gène conférant la résistance au bénomyl) et que le caractère surproducteur d'endothiapepsine est intégré de façon stable dans le clone 29P3 ben⁵ car il est issu de la germination d'une conidiospore uninucléée et toutes les cellules mycéliennes possèdent des noyaux ayant intégré le cosmide p8H12 dans leur ADN.

3) Amplification :

Des protoplastes préparés à partir du clone 29P3 ben⁵ ont été transformés par le plasmide pBT3, selon le protocole décrit dans la section 6. On a obtenu 400 clones bénomyl-résistants pour 1 µg d'ADN plasmidique.

Il est donc possible d'obtenir, après cotransformation par un cosmide contenant le gène fonctionnel de l'endothiapepsine et d'un plasmide contenant un marqueur de sélection, un transformant surproducteur d'endothiapepsine dépourvu du marqueur de sélection, capable d'être transformé à nouveau. On peut donc, en effectuant plusieurs cycles successifs comprenant une étape de cotransformation à l'aide des deux vecteurs ci-dessus, suivie d'une étape de purification permettant d'éliminer le marqueur de sélection, amplifier sélectivement dans C. parasitica le gène codant pour le précurseur de l'endothiapepsine.

Section 9 : Recherche de souches déficientes dans la production d'endothiapepsine après transformation de la C. parasitica SEBR 103.

Une analyse exhaustive des clones bénomyl-résistants obtenus après la cotransformation (cf. section 6) de la souche C. parasitica SEBR 103 avec le cosmide p8H12 et le plasmide pBT3 a permis d'obtenir un clone ne produisant pas de halo d'hydrolyse sur le milieu E gélosé contenant de la caséine. Ce clone a été purifié suivant la méthode décrite dans la section 8 et de nombreux clones bénomyl-sensibles et ne produisant pas de halo d'hydrolyse ont été obtenus. Un clone, dénommé 29P(end-), a été choisi au hasard parmi ces clones bénomyl-sensibles. Après culture en fiole, le clone 29P(end-) présente une activité coagulante inférieure à 0,01 g/l alors que la souche témoin Cryphonectria parasitica SEBR 103 non transformée présente une activité coagulante de 0,62 g/l (la baisse de production constatée est donc de plus de 98 %). On a de plus observé que les caractéristiques morphologiques et physiologiques de ce clone sont modifiées par rapport à celles de la souche de C. parasitica SEBR 103.

L'analyse par Southern Blot de l'ADN génomique du clone 29P(end-) et de la souche témoin SEBR 103, après hybridation avec le fragment A d'une part et le fragment contenant le gène de résistance au bénomyl du plasmide pBT3 d'autre part, n'ont pas montré de différences.

Ces résultats montrent que le clone 29P(end-) sélectionné et purifié a été rendu déficient pour la production d'endothiapepsine après cotransformation de la souche C. parasitica avec le cosmide de p8H12 et le plasmide pBT3. Il apparaît évident pour l'homme de l'art que des tranformants déficients dans la production d'endothiapepsine pourraient être obtenus par cotransformation de C. parasitica SEBR 103 avec un marqueur de sélection, tel que le plasmide pBT3, et un ADN comportant le gène de la préproendothiapepsine rendu non fonctionnel, par exemple par délétion d'une partie de la séquence codante, puis par sélection des transformants bénomyl résistants ne produisant pas de halo d'hydrolyse de la caséine. Un tel ADN portant un gène non fonctionnel peut être obtenu facilement par linéarisation de l'ADN du plasmide pEp1, par exemple en effectuant une double digestion Scal et Sfil puis en purifiant le plus grand fragment par électrophorèse sur gel d'agarose 0,8 % et enfin en traitant les extrémités de ce fragment à la polymérase klenow en présence des quatre désoxyribonucléotides triphosphates dNTP pour permettre une religation du vecteur et obtenir ainsi un plasmide portant un gène non fonctionnel de l'endothiapepsine.

Section 10 : Construction de la souche SEBR 3700, déficiente dans la production d'endothiapepsine et dépourvue de marqueur de sélection dominant

1) Construction d'un fragment, appelé fragment EM, dans lequel la séquence codant pour l'endothiapepsine est interrompue par deux codons d'arrêt de la traduction.

On a introduit au début de la séquence codant pour le précurseur de l'endothiapepsine des mutations entraînant un arrêt de la traduction, ce qui fait que l'endothiapepsine n'est plus produite à partir de l'ARN messager portant ces mutations. Le fragment A de l'endothiapepsine comprend un fragment HindIII -BstEII d'environ 360 pb (cf. figure 2), le site BstEII étant localisé à 25 pb en aval du début de la séquence codante. La séquence naturelle en question est la suivante :

EP 0 475 842 B1

```
                                                    BstEII
                                                      |
                                                      |
       5'-GATGTCTTCC CCTCTCAAGA ACGCCTTGGT GACC
```

Le triplet ATG souligné représente le codon ouvrant la phase codante. Le site BstEII est indiqué par une barre verticale.

La séquence mutée désirée est

```
                                                      BstEII
                                                        |
                                                        |
        5'-GATGTCTTCC CCTCTCTAAT GAACGCCTTG GTGACC
```

Cette séquence diffère de la séquence naturelle par l'introduction de 2T, l'un entre les 5ème et 6ème codon, l'autre à l'intérieur du 6ème codon (entre la 2ème et la 3ème base). Ces introductions résultent dans la création de 2 codons stops arrêtant la phase de lecture du gène de l'endothiapepsine.

Un fragment HindIII - BstEII ne différant de la séquence de type sauvage que par ces 2 modifications a été obtenu par la technique d'amplification par PCR décrite dans la section 15 en utilisant pour une des amorces un oligonucléotide portant les mutations désirées.

L'oligonucléotide 1 a la séquence suivante :

```
                        HindIII
                          |
        5' - GCTAAAGCTT ATCCGCCGCC GGCGGGGGAA TTC
```

Cette séquence se retrouve à l'extrémité HindIII du fragment HindIII-BstEII. Le site HindIII est figuré par une barre verticale.

L'oligonucléotide 2 a comme séquence:

```
                        BamHI   BstEII
                          |       |
        5' - CAATGGATCC GGTCACCAAG GCGTTCATTA GAGAGGGGAA GA
    CATC
```

Cet olignonucléotide est complémentaire de la séquence mutée désirée, on y a adjoint un site BamHI flanquant le site naturel BstEII. Les nucléotides soulignés correspondent aux additions créant les codons stops sur le brin complémentaire.

L'ADN servant de matrice est l'ADN du plasmide p472 décrit dans la Section 1. Le mélange d'amplification comprend :

300 ng (soit 3 µl) de l'ADN de p472
100 ng (soit 1 µl) de chaque oligonucléotide
5 µl de tampon bipv2 concentré 10 fois
40 µl d'eau
2 unités (soit 0,5 µl) d'enzyme : polymérase Taq.

Le tampon bipv2 10 fois concentré a la composition suivante : Tris-HCl 670 mM (pH 8,8, sulfate d'ammonium, 165 mM ; 2 mercaptoéthanol, 10 mM ; gélatine, 2 mg/ml ; Triton - X100 1,5 % ; EDTA, 67 µM ; MgC12, 20 mM ; dATP, 2

27

mM ; dCTP, 2 mM ; dGTP, 2 mM, DTTP, 2 mM.

Trois essais d'amplification sont réalisés en parallèle. On effectue 15 cycles d'amplification, chaque cycle se décomposant en 1 min de dénaturation à 92°C, 1 min d'hybridation à 55°C, 1 min d'élongation à 72°C. Après l'amplification par PCR, les 3 tubes sont rassemblés dans 1 tube Eppendorf et précipités avec 2 volumes d'éthanol absolu contenant 0,3 M d'acétate d'ammonium (pendant 20 min à 0°C) puis centrifugés à 10 000 g (pendant 20 min). Le culot est lavé à l'éthanol 70 %, puis séché sous vide pendant 10 min.

L'ADN est repris dans 60 µl de solution de TE (Tris-HCl 10 mM pH 7,5 EDTA 1 mM) et analysé sur gel d'agarose. La bande correspondant au fragment de 360 bp est éluée du gel, purifiée et clonée dans la forme réplicative d'un phage M13 (M13mp19) entre les sites HindIII et BamHI après action de ces enzymes.

Le fragment ainsi cloné a été séquencé à partir du fragment simple brin, et on a vérifié que la séquence correspondait bien au fragment muté aux endroits attendus.

Le fragment A d'environ 2,1 kb décrit dans la section 1 est délimité par 2 sites HindIII et présente un site unique BstEII localisé à 360 paires de bases d'une extrémité. Le site unique BstEII délimite donc 2 segments du fragment A, le plus court correspond à la séquence soumise à l'amplification par PCR. En préparant le grand segment BstEII-HindIII du fragment A et le petit segment HindIII-BstEII muté, et en ligant l'ensemble dans pBR322 coupé par HindIII, on reconstitue ainsi un fragment A porteur des 2 mutations, appelé fragment A - M.

Le fragment A-M présente un site PstI qui est unique pour ce fragment. Ce site PstI délimite donc 2 segments du fragment A-M dont un segment HindIII-PstI d'environ 1,67 kb qui porte la double mutation.

Le plasmide pEp3 décrit dans la section 5 dérive d'un plasmide pUC dans lequel a été cloné un fragment SphI d'environ 3,7 kb appelé fragment E. Ce fragment de 3,7 kb comprend comme sous-fragment le fragment A. Ce fragment A a été remplacé par le fragment A-M de la façon suivante :

Le plasmide pEp3b a été digéré à la fois par les enzymes ScaI et PstI d'une part et les enzymes ScaI et HindIII d'autre part. A partir de la première digestion, on a isolé le fragment ScaI - PstI d'environ 2,250 kb. A partir de la deuxième digestion, on a isolé le fragment ScaI-HindIII d'environ 2,42 kb. La ligation de ces 2 fragments avec le segment HindIII-PstI de 1,67 kb qui porte la double mutation permet d'obtenir un nouveau plasmide appelé pEpM3b qui ne diffère de pEp3b que par la double mutation.

Le fragment SphI portant la double mutation, appelé fragment E-M, a été préparé à partir d'une digestion SphI de pEpM3b suivie d'une séparation des 2 fragments d'ADN sur gel d'agarose 0,8 % et extraction du fragment de 3,7 kb muté dans 20 µl de solution TE.

**2)** Cotransformation de <u>Cryphonectria parasitica</u> par le fragment EM et le fragment SfiI porteur d'un gène de résistance au bénomyl encadré par des séquences télomériques

Le fragment E-M purifié a été utilisé en cotransformation avec l'ADN du plasmide p578.12 digéré par SfiI.

Le plasmide p578.12 est un dérivé du plasmide pBT3 déjà décrit. L'ADN de pBT3 a été linéarisé par l'endonucléase XbaI, les extrémités ont été rendues franches par la DNA polymérase de Klenow. On y a intégré un fragment XhoI, réparé à la polymérase et qui comprend les séquences télomériques de <u>Tetrahymena</u> provenant du plasmide pPAT ura décrit par Perrot, Barreau et Béguéret Mol. Cel. Biol. (1987) 7 p. 1725 - 1730, modifié de façon à remplacer les sites BamHI par SfiI. La digestion de p578.12 par SfiI libère un fragment linéaire qui porte un gène de résistance au bénomyl et qui se termine à chacune de ses extrémités par une séquence télomérique. Perrot et Col. (réf. ci-dessus) ont montré qu'un tel fragment pouvait être maintenu à l'état de plasmide linéaire (non intégré dans le chromosome) dans le champignon filamenteux <u>Podospora anserina.</u> D'autre part, Powell et Kistler (J. bacteriology, 1990, vol 172, pp 3163-3171) ont montré que des séquences répétées de type télomérique permettaient la réplication autonome de plasmides linéaires chez <u>C. parasitica.</u> On a tiré profit de cette propriété pour construire des souches propres par cotransformation avec le fragment linéaire. L'absence d'intégration du marqueur de résistance au bénomyl dans le chromosome doit permettre de récupérer des souches bénomyl sensibles avec une fréquence très grande à partir des conidies obtenues après sporulation des transformants.

On a réalisé 2 expériences de cotransformation de la souche <u>Cryphonectria parasitica</u> SEBR 103 avec, pour chacune, environ 0,5 µg (3 µl) du fragment E-M et 0,5 µg (4 µl) du fragment SfiI du plasmide p578.12. 397 colonies résistantes au bénomyl ont été obtenues. On a repiqué 395 de ces colonies sur le milieu E (cf. section 7) pour tester la présence de halo comme décrit dans la section 7.

On a trouvé une colonie ne faisant plus de halo de coagulation de la caséine ; cette colonie, appelée colonie AJ7.272, a été transférée sur du milieu de sporulation (conidiation), suivant la méthode décrite dans la section 1. Les conidiospores ont été récoltées en masse et diluées sur le milieu de germination. On a testé 152 colonies issues de la conidiation sur du milieu caséine : aucune n'a présenté de halo de coagulation. On a également testé la résistance au bénomyl de ces colonies : 58 sur les 152 avaient perdu ce caractère de transformation. On en a repris 5 sur les 58, on les a appelées colonie AJ7272/A, colonie AJ7272/B, colonie AJ7272/J, colonie AJ7272/L et colonie AJ7272/M. On a extrait l'ADN génomique de chaque colonie et on a comparé les profils d'hybridation par la technique de Southern blot et celui de la souche SEBR 103 en utilisant diverses enzymes de restriction. Aucune différence n'a été trouvée entre la souche SEBR 103 et les 5 mutants, sondés avec le fragment A (gène de l'endothiapepsine) avec le pUC18 (séquences bactériennes) ou avec le gène de la β-tubuline de <u>Neurospora.</u> Avec le fragment A, on allume les séquences du gène de l'endothiapepsine ; le profil d'hybridation étant le même dans toutes les souches, on peut conclure qu'il n'y a pas de recombinaison "ectopique" du fragment muté (c'est-à-dire de recombinaison non homologue) dans les mutants. Avec le pUC18, on n'obtient aucune hybridation quelle que soit la souche, ce qui indique qu'il ne reste pas de séquence d'origine bactérienne intégrée dans les mutants. Avec le gène de la β-butuline, on allume une séquence dans toutes les souches, il s'agit très vraisemblablement du gène "endogène" de la β-tubuline de <u>C. parasitica.</u>

Pour s'assurer que les souches dérivées de la colonie AJ7272 étaient bien mutées par le gène de structure de l'endothiapepsine, on a vérifié qu'on pouvait complémenter leur déficience par transformation. On a utilisé chacune des colonies précédentes comme réceptrice pour une cotransformation impliquant les ADN du plasmide pBT3 et du plasmide pEp2.

Le tableau suivant montre que la majorité des transformants résistant au bénomyl obtenus de cette façon produisent à nouveau de l'endothiapepsine.

| Souche | Colonies résistantes au bénomyl (test sur caséine) | Colonies productrices de protéase endothiapepsine |
|---|---|---|
| AJ7.272/A | 2 | 2 |
| AJ7.272/B | 8 | 7 |
| AJ7.272/J | 13 | 11 |
| AJ7.272/L | 17 | 16 |
| AJ7.272/M | 10 | 10 |

Contrôle : la transformation des ségrégants avec le plasmide pBT3 seul ne conne aucun transformant producteur de protéase.

L'analyse des surnageants de culture par électrophorèse sur gel d'acrylamide a confirmé que les souches mutées ne produisaient pas de quantité détectable d'endothiapepsine avant transformation.

On a donc construit une souche AJ7.272 qui, après sporulation, a donné naissance à 5 colonies qui ne diffèrent de la souche SEBR 103 que par une ou des mutations affectant le gène de structure de l'endothiapepsine. Une de ces colonies a été retenue. Elle est appelée SEBR 3700. Elle est déficiente dans la production d'endothiapepsine et dépourvue de marqueur de sélection dominant.

Section 11 : Vérification de la fonctionnalité des fragments D et F par complémentation de la souche SEBR 3700 déficiente dans la production d'endothiapepsine

Il a été démontré dans la section 7, paragraphe 3, que le fragment C contenu dans le plasmide pEp1 porte tous les signaux nécessaires à l'expression de l'endothiapepsine. On a vérifié à l'aide de la souche SEBR 3700 déficiente pour la production d'endothiapepsine obtenue dans la section 10 la fonctionnalité des fragments D et F contenus dans les plasmides pEp2 et pEp4.

### 1) Transformation des protoplastes de la souche SEBR 3700 par les plasmides pEp2 et pBT3

Le plasmide pEp2 porte à la fois le gène de résistance au bénomyl et le fragment D et peut donc servir à transformer directement. Le plasmide pBT3 qui ne porte pas de gène codant pour l'endothiapepsine est utilisé en témoin négatif. La souche SEBR 3700 a été transformée suivant un protocole identique à celui décrit pour la souche SEBR 103 (cf. section 6) avec environ 1 µg du plasmide pEp2 et 1 µg du plasmide pBT3.

### 2) Cotransformation des protoplastes de la souche SEBR 3700 par les plasmides pEp1 et pBT3 et les plasmides pEp4 et pBT3

Les plasmides pEp1 et pEp4 ne portent pas de marqueur de sélection dominant. Le plasmide pEp1 est utilisé en témoin positif de complémentation de la souche SEBR 3700.

La souche SEBR 3700 a été cotransformée avec les mélanges des plasmides suivants : 4 µg de pEp1 et 1 µg de pBT3, 4 µg de pEp4 et 1 µg de pBT3.

3) **Détection des transformants produisant l'endothiapepsine**

Des implants mycéliens de plusieurs transformants sélectionnés pour chacune des transformations et cotransformation décrites ci-dessus ont été repiqués sur le milieu E, milieu gélosé contenant de la caséine. Après incubation, les colonies présentant un halo de précipitation caractéristique de la secrétion d'endothiapepsine ont été repérées. Les résultats obtenus sont les suivants :

- transformants obtenus avec le plasmide pBT3 : aucun clone n'a donné de halo de précipitation

- transformants obtenus soit avec les plasmides pEp1 et pBT3, soit avec les plasmides pEp4 et pBT3 : une proportion d'environ 30 % des clones ont donné des halos de précipitation

- transformants obtenus avec le plasmide pEp2 : 92 % des clones testés ont donné des halos de précipitation.

Ces résultats montrent que les plasmides pEp1, pEp2 et pEp4 complémentent la souche SEBR 3700 déficiente dans la production d'endothiapepsine et donc que les fragments C, D et F contenus dans ces plasmides portent tous un promoteur fonctionnel de l'endothiapepsine. Cependant, on ne peut pas déduire de cet essai qualitatif la force relative du promoteur présent sur chacun des fragments.

On peut déduire de ces résultats que le segment SphI-HindIII du fragment F, dont la séquence a été déterminée dans la section 5, possède des signaux impliqués dans l'activation du promoteur du gène codant pour la préproendothiapepsine.

Section 12 : Sélection de transformants surproducteurs d'endothiapepsine, dépourvus de marqueur de sélection dominant

1) **Protocole de sélection**

Les différentes étapes du protocole sont représentées dans le tableau ci-après. Le principe en est le suivant :

Les protoplastes de C. parasitica SEBR 103 sont cotransformés, suivant un protocole identique à celui décrit dans la section 6, par un mélange composé de 0,5 à 2 µg de fragment C, D ou F, préalablement purifié sur gel d'agarose après digestion des plasmides pEp1 ou pEp2 et extrait, et de 0,5 µg du plasmide de sélection pBT3 ou pBT6 soit sous forme circulaire, soit sous forme linéaire.

Après régénération des protoplastes sur le milieu C, les transformants obtenus qui sont dénommés transformants initiaux sont repiqués simultanément sur le milieu B rendu gélosé par l'addition de 20 g/l d'agar (milieu non sélectif) et sur le milieu E, milieu gélosé contenant de la caséine additionné éventuellement de 5 µg/ml de pepstatine. Les clones présentant un rapport entre le diamètre de halo de précipitation et le diamètre de colonie significativement plus élevé que celui de la souche témoin non transformée sont dénommés transformants initiaux surproducteurs et sont mis à sporuler. Une préparation de conidiospores de ces souches est effectuée selon la méthode utilisée dans la section 1.1 et une dilution de chaque suspension de conidiospores est étalée sur le milieu G de manière à obtenir des colonies isolées. Des implants mycéliens provenant d'une cinquantaine de colonies sont repiqués pour chaque transformant initial surproducteur simultanément sur milieu b rendu gélosé par l'addition de 20 g/l d'agar additionné éventuellement de 0,5 mg/l de benlate (test de sensibilité au bénomyl) et sur milieu E (test sur milieu caséine). A ce stade, les clones qui sont sensibles au bénomyl et surproducteurs d'endothiapepsine sont dénommés ségrégant bénomyl (s) surproducteur et sont mis à sporuler. Une préparation de conidiospores est ensuite effectuée pour vérifier la surproduction d'endothiapepsine après culture en fiole, vérifier l'intégration d'une ou plusieurs copies du fragment C, D ou F par hybridation de l'ADN génomique avec une sonde constituée du fragment A, et s'assurer de l'absence d'ADN hétérologue en utilisant une sonde constituée par tout ou partie du plasmide de sélection. La souche répondant positivement à ces trois critères est dénommée souche propre surproductrice. Un nouveau cycle de cotransformation-sélection peut alors être effectué à partir de cette souche propre surproductrice pour amplifier à nouveau le fragment C, D ou F.

L'avantage de ce procédé, en plus de celui obtenu par l'amplification du fragment d'intérêt, est de construire des transformants dépourvus de marqueur de sélection dominant, plus acceptables sur un plan réglementaire.

| Protocole de sélection de transformants suproducteurs dépourvus de marqueur de sélection dominant | |
| --- | --- |
| Durée (semaines) | SEBR 103 |
| | PROTOPLASTES<br>cotransformation : |

(suite)

| Protocole de sélection de transformants suproducteurs dépourvus de marqueur de sélection dominant | |
|---|---|
| Durée (semaines) | SEBR 103 |
| 1 | plasmide de sélection + fragment C, D ou F |
| | TRANSFORMANTS |
| | repiquage sur milieu non sélectif |
| | test sur milieu caséine |
| 2 | TRANSFORMANTS INITIAUX SURPRODUCTEURS |
| | étape de sporulation |
| | test de sensibilité au bénomyl |
| | test sur milieu caséine |
| 5 à 6 | SEGREGANTS BENOMYL(S) SURPRODUCTEURS |
| | étape de sporulation |
| | test de production en erlens |
| | Analyse de l'intégration par Southern |
| | Test en fermenteur 2 litres |
| 5 à 6 | SOUCHE PROPRE SURPRODUCTRICE |
| | étape de sporulation |
| 3 à 4 | PREPARATION DE LOT DE SEMENCES PRIMAIRE |
| 16 à 19 | test en fermenteur 20 litres |
| | analyse du produit |
| | étude de stabilité du transformant |
| | autres études... |
| | CHOIX D'UNE SOUCHE DE PRODUCTION |

**2) Sélection de transformants surproducteurs dépourvus de marqueur de sélection dominant après cotransformation de SEBR 103 par le fragment C, D ou F et le plasmide pBT3**

Le tableau ci-dessous résume les résultats obtenus

| cotransformation | fragment F | fragment C | fragment D |
|---|---|---|---|
| quantité | 0,5 µg | 2 µg | 0,5 µg |
| plasmide de sélection | circulaire | circulaire | linéaire |
| transformants initiaux | 4 | 137 | 106 |
| transformants initiaux surproducteurs | 1 | 26 | 33 |
| ségrégants bénomyl(s) | 1 | 4* | 8** |
| ségrégants bénomyl(s) surproducteurs | 0 | 2 | 7 |
| souches propres surproductrices | 0 | 1 | 4 |
| Aucune souche propre surproductrice n'a pu être obtenue avec le fragment C, ce qui n'est pas surprenant compte tenu du nombre peu élevé de transformants initiaux. | | | |

\* sélection effectuée sur la descendance de 12 transformants initiaux surproducteurs

\*\* sélection effectuée sur la descendance de 21 transformants initiaux surproducteurs.

La souche propre surproductrice obtenue avec le fragment D a été testée en fermenteur 2 litres et a produit une activité coagulante de 1,9 g/l, la souche témoin ne produisant qu'une activité coagulante de 1,2 g/l (le facteur de surproduction est donc de +1,58). L'analyse de l'ADN génomique par Southern de la souche SEBR 3574 a montré qu'elle avait intégré au moins 3 copies supplémentaires du fragment D dont 2 copies en tandem.

Parmi les quatre souches propres surproductrices obtenues avec le fragment F, la souche SEBR 3912 a été testée en fermenteur 2 litres et a produit une activité coagulante de 2,4 g/l (le facteur de surproduction par rapport à la souche témoin est donc de +2). L'analyse de l'ADN génomique par Southern blot a montré qu'elle avait intégré au moins 5 copies supplémentaires du fragment F, en tandem selon une orientation tête-queue.

Dans les souches surproductrices obtenues, aucune intégration du plasmide de sélection n'a été détectée. Ces souches peuvent donc être soumises à un nouveau cycle de cotransformation-sélection. D'autre part, si on compare le facteur de surproduction observé pour ces souches avec le nombre présumé de copies intégrées, on constate que le rapport obtenu est voisin de 0,2 alors qu'il est d'environ 1 pour les transformants 30Pn qui ont intégré deux copies supplémentaires du plasmide pEp1 contenant le fragment C. On peut donc penser qu'en amont du site BglII situé à l'extrémité 5' du fragment D jusqu'au site BamHI, il existe des séquences régulatrices importantes pour une forte expression du gène de l'endothiapepsine.

Section 13 : Construction du plasmide pEMR713 vecteur d'expression de la préproendothiapepsine dans C. parasitica qui comprend la région promotrice du gène codant pour la glycéraldéhyde-3-phosphate déshydrogénase d'Aspergillus nidulans

Le plasmide pAN52 (Punt et al, 1987, Gene, 56, 117-124) porte en plus du gène codant pour la résistance à l'ampicilline et l'origine de réplication de pUC18, la région promotrice du gène codant pour la glycéraldéhyde-3-phosphate déshydrogénase (gpd) d'Aspergillus nidulans [Punt et al, gene, 93, (1990) 101-109] et la région terminatrice du gène trpC d'Aspergillus nidulans, Mullaney et al, Mol. Gen. Genet. (1985) 189 ; 37-45. La région promotrice et la région terminatrice sont séparées par une séquence d'ADN qui comprend des séquences nucléotidiques reconnues par les enzymes de restriction NcoI et MluI, lesquelles sont uniques sur le plasmide pAN52. La séquence du site NcoI : CCATGG est particulièrement intéressante dans la mesure où elle comprend le codon ATG qui code pour une méthionine qui est le codon d'initiation de la majorité des protéines.

Le but de cette expérience est d'exprimer le gène codant pour l'endothiapepsine en utilisant les signaux d'expression décrits ci-dessus. L'intégration du gène codant pour l'endothiapepsine se fait par la technique du P.C.R. (Polymérase Chaîne Réaction) qui est décrite en détail dans la section 15 ci-après.

1 - Description des amorces utilisées pour le PCR

L'amplification génique par PCR permet la modification de la séquence à amplifier. Cette propriété est utilisée afin d'introduire à l'extrémité 5' de la séquence prépro du gène codant pour l'endothiapepsine, un site NcoI et à l'extrémité 3' non codante du gène codant pour l'endothiapepsine, un site MluI. Les séquences des deux amorces sont donc les suivantes :

- Amorce 5' qui porte le site NcoI :

```
5'-ACGTCCATGG CTTCCCCTCT CAAGAACGCC-3'
```

Cette amorce est principalement constituée de :

a) la séquence reconnue par l'enzyme de restriction NcoI : CCATGG
b) la séquence modifiée de l'extrémité 5' du peptide signal de l'endothiapepsine.

La modification consiste dans le changement du premier codon, après la méthionine ; en effet, le codon TCT codant pour une sérine est remplacé par le codon GCT codant pour une alanine. Cette modification est sans effet sur l'efficacité du peptide signal.

- Amorce 3' qui porte le site MluI :

```
5' -ACGTACGCGT CCACGCCTAC CCAACAAGAC-3'
```

Cette amorce est principalement constituée de :

a) la séquence reconnue par l'enzyme de restriction MluI : ACGCGT
b) la séquence de l'extrémité 3' non codante de l'endothiapepsine, qui est la séquence complémentaire de la séquence située entre les nucléotides 1921 et 1940 de la figure 2.

2 - Obtention du fragment amplifié contenant le gène codant pour l'endothiapepsine modifiée

On utilise comme matrice le plasmide p472, dont l'obtention est décrite dans la section 1.

a) la réaction PCR

100 ng de plasmide p472, préalablement purifiés sur colonne P10 sont mélangés à 100 ng de l'amorce 5', 100 ng de l'amorce 3', 2 mM $MgCl_2$, 0,2 mM de dNTP, 5 µl de mélange réactionnel concentré 10 fois (quantité finale : 67 mM Tris HCl pH 8,8, 16,6 mM $(NH_4)_2$ $2 SO_4$, 1 mM β-mercaptoéthanol, 6,7 mM EDTA, 0,15 % Triton x100, 200 g/ml de gélatine).

Le volume du mélange est ensuite porté à 50 µl en ajoutant de l'eau.

Le mélange réactionnel ainsi obtenu est incubé pendant 4 min à 94°C puis porté à la température de 50°C, qui est maintenue pendant 4 min.

On ajoute alors 0,5 µl, soit 2,5 unités, de polymérase Taq (Boehringer Mannheim réf. 1146-165). On couvre alors le mélange réactionnel avec de la paraffine afin d'éviter l'évaporation de la solution aqueuse.

L'amplification se fait au cours de 18 cycles réactionnels dont les étapes sont les suivantes :

$$2 \text{ min à } 92°C \rightarrow \text{dénaturation}$$

$$2 \text{ min à } 50°C \rightarrow \text{hybridation}$$

$$2 \text{ min à } 72°C \rightarrow \text{polymérisation.}$$

Après les 18 cycles, la réaction enzymatique est arrêtée par l'addition d'une solution 20 mM EDTA.

Le fragment d'ADN ainsi amplifié, qui présente la taille attendue d'environ 1620 pb, est ensuite isolé et purifié sur gel d'agarose 1 %, dialysé sur colonne P10 (Pharmacia) puis hydrolysé simultanément par les enzymes Ncol et Mlul. Après hydrolyse, le fragment est purifié sur colonne P10.

b) Obtention du plasmide pEMR713

L'ADN de plasmide pAN52 est hydrolysé par les enzymes de restriction Ncol et Mlul. Le fragment portant la région promotrice du gène gpd, l'origine de réplication de E. coli, le gène cocant pour la résistance à l'ampicilline et le terminateur trpC est purifié. 100 ng de ce fragment sont ligués, en présence d'ADN ligase à 100 ng du fragment ampfifié portant le gène de l'endothiapepsine (cf. paragraphe 2 ci-dessus). Le mélange de ligation est ensuite utilisé pour transformer la souche RRI. Le plasmide résultant est le pEMR713 dans lequel le gène codant pour l'endothiapepsine modifié est placé sous le contrôle de la région promotrice du gpd et la région terminatrice de trpC.

Préparation de protoplastes

cf. section 6

Cotransformation des protoplastes de la souche SEBR 3700 par le plasmide pEMR713 et le plasmide pBT3

cf. section 6

Environ 2 000 transformants obtenus sont capables de se développer sur le milieu B gélosé contenant 0,5 mg/l de Benlate, ce qui indique que toutes ces colonies portent au moins un plasmide pBT3.

Sélection de souches transformées, productrices d'endothiapepsine

## A) **Méthode générale**

a) Sélection sur milieu gélosé contenant de la caséine.

Des implants mycéliens des 2 000 colonies résistantes au Bénomyl sont repiqués sur un milieu gélosé contenant de la caséine, appelé milieu E, dont la composition est précisée dans le tableau B précédent. Sur ce milieu, les colonies de Cryphonectria parasitica qui produisent la protéase donnent naissance à un halo de précipitation dont la surface est proportionnelle à la quantité d'endothiapepsine sécrétée.

Les souches productrices sont retenues sur la base de la présence du halo de précipitation. Une préparation de conidiospores de ces souches productrices est effectuée selon la méthode utilisée dans la section 1.1). De plus, on a vérifié par l'addition au milieu E de 5 $\mu$g/ml de pepstatine, substance inhibitrice spécifique des aspartyl-protéases, que l'accroissement des halos observé chez les souches surproductrices était réduit. Ce résultat indique qu'il s'agit bien d'une surproduction d'une aspartyl-protéase.

On a isolé 3 transformants capables de produire un halo de coagulation. Des expériences de contrôle montrent que la taille du halo des clones recombinants transformés par les plasmides pBT3 et pEMR713 appelés clone 1, clone 2 et clone 3, est comparable à celle obtenue pour la souche SEBR 103.

b) Sélection en milieu liquide par étude en fiole

Pour confirmer ce résultat, des essais de production en fioles ont été réalisés de façon suivante : ensemencement des fioles de 250 ml contenant 40 ml de milieu F (cf. section 7). On incube ensuite les fioles à 28°C sur un agitateur rotatif excentrique réglé à 220 tr/min pendant 48 h. Pour chaque souche, les cultures ont été réalisées dans 3 fioles différentes et la moyenne des résultats de dosage d'activité coagulante pour les 3 fioles a été calculée. Le témoin est constitué par la souche Cryphonectria parasitica SEBR 103 non transformée. Le dosage de l'activité coagulante est réalisé selon la méthode officielle de détermination de la teneur en enzyme des solutions coagulantes publiée au Journal Officiel de la République Française du 20 mars 1981 (paragraphe C), résumée dans la section 7.

c) Analyse de l'enzyme sécrétée par les clones 1, 2 et 3

Le moût de fermentation obtenu après culture des transformants producteurs et celui de la souche témoin C. parasitica SEBR 103 non transformée ont été soumis à une centrifugation de façon à éliminer la phase mycélienne. Après dénaturation des protéines du surnageant en présence de SDS pendant 5 min à 100°C, on a effectué une électrophorèse sur gel de polyacrylamide en présence de SDS. On observe, après coloration au bleu de Coomassie, une bande majoritaire de masse moléculaire voisine de 36 kDa, qui correspond à la masse moléculaire de l'endothiapepsine mature, déduite de la séquence (cf. figure 1), de même intensité pour les transformants producteurs que pour la souche témoin, et des bandes minoritaires identiques pour les transformants surproducteurs et la souche témoin.

On a vérifié, d'autre part, par réaction antigène-anticorps (kit Rennetest, France Biochem) sur les surnageants de culture des transformants surproducteurs et de la souche témoin non transformée, que l'enzyme sécrétée est identique à celle de Cryphonectria parasitica conformément à la méthode d'identification décrite dans le Journal Officiel de la République Française du 20 mars 1981.

De plus, on a évalué le rapport entre l'activité coagulante et l'activité protéolytique de l'enzyme sécrétée. L'activité coagulante exprimée en g/l est mesurée à l'aide du réactif TNBS, décrit par R. Fields, Biochem. J. (1971) 124 : 581-590, par dosage des groupements aminés apparus après protéolyse de la diméthyl-caseine.

Ce rapport est compris entre 0,040 et 0,045 pour les transformants surproducteurs, ce qui est très proche de celui obtenu avec la souche témoin non transformée. On peut conclure de ces trois études que c'est bien l'endothiapepsine qui est spécifiquement produite par les clones 1, 2 et 3.

Cette expérience montre qu'il est possible d'exprimer dans C. parasitica le gène codant pour l'endothiapepsine, en utilisant une région promotrice et une région terminatrice hétérologues (n'appartenant pas à C. parasitica).

Section 15 : Amplification par la technique PCR de l'ADN complémentaire codant pour le précurseur de l'endothiapepsine

1) Isolement des ARN messagers de Cryphonectria parasitica

La souche de Cryphonectria parasitica SEBR 103 a été cultivée dans des conditions de production de l'endothiapepsine. Le mycélium a été récupéré par filtration sur gaze, lavé à l'eau et congelé dans l'azote liquide.

15 g de mycélium (poids humide) congelés sont mis en suspension dans 45 ml de tampon de lyse puis repris dans un même volume de billes (0,45 μm de diamètre). Le tampon de lyse est constitué de thiocyanate de guanidine 4M, Tris HCL 10 mM pH 7,6, EDTA 10 mM, β-mercaptoéthanol 50 ml/l. La suspension mycélienne est broyée pendant 5 min.

Le broyat est récupéré et les billes décantées. Environ 45 ml de surnageant sont prélevés, additionnés de 3 M final de chlorure de lithium et conservés à 0°C.

Après deux jours, on centrifuge la solution précédente pendant 60 min à 10 000 tr/min. On prélève le surnageant et on reprend le culot dans 40 ml de LiCl 3M. La suspension obtenue est recentrifugée à 10 000 tr/min pendant 1 h 30. On ajoute de la protéinase K (SIGMA) 40 μg/ml, du SDS (0,1 % p/v) et de l'EDTA 20 mM. On incube à 37°C pendant 3 h. On précipite avec 2 volumes d'éthanol, puis on effectue un lavage à l'éthanol 70 %. On reprend le culot dans 0,5 ml de tampon TE (Tris HCl 10 mM, EDTA 1 mM pH 7,5), on extrait 2 fois au chloroforme, on précipite à l'éthanol. Les ARN sont conservés à -80°C dans l'éthanol.

### 2) Purification de la fraction poly A+ des ARN

Environ 1 mg d'ARN est précipité 20 min à 4°C (15 000 tr/min), puis lavé avec de l'éthanol 70 %, puis séché. Le culot est repris dans 1 ml de tampon TE et mis en suspension par agitation au vortex. L'oligo dT-cellulose type 3 (commercialisé par Collaborative Research Inc, Biomedicals Product Division) est préparé suivant les recommandations du fabricant. L'ARN est déposé sur l'oligo dT, agité doucement pour remettre en suspension les billes, puis chauffé pendant 1 min à 65°C.

La suspension est ajustée à 0,5 M NaCl puis mise à agiter doucement pendant 10 min. La suspension est alors centrifugée pendant 1 min à 1 000 tr/min, le surnageant est éliminé, le culot est lavé 2 fois avec 1 ml de tampon TE contenant 0,5 M NaCl. Les surnageants sont éliminés. L'élution de la fraction polyadénylée des ARN (constituée des ARN messagers) est obtenue par suspension des billes dans 1 ml de tampon TE, puis chauffage de cette suspension à 60°C pendant 1 min, suivie d'une agitation pendant 10 min sur plaque basculante. On centrifuge ensuite 1 min à 1 000 tr/min, ce qui permet de récupérer d'une part le surnageant contenant des ARNm libres en solution et d'autre part le culot de billes de cellulose. L'ensemble des opérations ci-dessus (à partir de l'élution) est répété. Les surnageants ainsi obtenus sont rassemblés, on élimine l'excès de billes par centrifugation et on précipite le surnageant à l'éthanol contenant du NaCl suivant les techniques habituelles. (Maniatis : op. cit.).

### 3) Description de la technique de la réaction polymérase en chaîne (Polymerase chain reaction - PCR)

La technique de la réaction de polymérase en chaîne (PCR) est une méthode bien connue de l'homme de l'art qui permet de copier simultanément les deux brins d'une séquence d'ADN préalable-ment dénaturé en utilisant deux oligonucléotides comme amorces (cf. notamment l'ouvrage de H.A. Erlich : "PCR Technology : Principles and Applications for DNA amplification" publié en 1989 par les éditions Macmillan Publishers Ltd, Royaume-Uni). Le principe de cette technique est résumé ci-après.

De nombreux cycles, dont chacun est constitué de trois étapes, provoquent l'amplification des brins d' ADN d'intérêt ; ces trois étapes sont :

a) dénaturation de la matrice
b) hybridation des amorces sur la matrice
c) extension des amorces

Après quelques heures de cycles, des centaines de milliers de copies de la matrice originale ont été produites à l'aide d'une ADN polymérase thermostable de Thermus aquaticus, habituellement appelée polymérase Taq.

La technique du PCR est basée sur la répétition de trois étapes.

a) Dénaturation de la matrice :

L'ADN double brin est dénaturé en ADN simple brin par incubation à haute température (de 92°C à 96°C) pendant approximativement 2 min.

b) Hybridation des amorces :

Ces amorces sont une paire d'oligonucléotides synthétiques qui s'hybrident avec les extrémités de la région à amplifier. Les deux amorces s'hybrident avec les brins opposés. Les amorces sont ajoutées en excès, de façon que la formation du complexe amorce-matrice soit favorisée.

c) Extension des amorces :

L'étape au cours de laquelle la polymérase Taq assure l'extension du complexe amorce-matrice de 5' vers 3' est effectuée à 72°C.

Dans la technique du PCR, le produit d'intérêt apparaît au troisième cycle et il est alors amplifié de manière significative. Au cours du déroulement des cycles, le produit d'amplification devient rapidement la matrice majeure avec laquelle les amorces s'hybrident.

4) <u>Description des amorces utilisées</u>

On a préparé deux oligonucléotides synthétiques à partir de la séquence du fragment A (cf. figure 2).
Le premier oligonucléotide, appelé amorce 1, dont la séquence est la suivante :

```
5' AGAAAGCTTG GAGGAGCGAG GGCCC 3'
     région 1          région 2
```

possède deux régions distinctes : la région 1, qui porte un site de clonage AAGCTT correspondant au site de reconnaissance de l'endonucléase HindIII et la région 2, qui est une région destinée à s'hybrider avec la région non codante du brin codant du fragment A, située en 3' de la séquence codant pour la préproendothiapepsine (cf. figure 2 - position 1870-1881).

Le deuxième oligonucléotide, appelé amorce 2, dont la séquence est la suivante :

```
5'  GCAGAATTCA CATATG TCTTCCCCTC TCAAG   3'
      région 1          région 2
```

est également constitué de deux régions distinctes : la région 1 qui porte un site de clonage CATATG correspondant au site de reconnaissance de l'endonucléase NdeI, dans lequel est comprise la séquence du codon initiateur ATG, et la région 2 qui porte une séquence nucléotidique identique à celle codant pour les cinq premiers acides aminés de la préproendothiapepsine, qui suivent la méthionine initiale. Cette région est destinée à s'hybrider avec le brin non codant du fragment A.

5) <u>Obtention du fragment amplifié représentant l'ADN complémentaire de l'endothiapepsine.</u>

On utilise comme matrice un pool d'ARN messagers, dont on sait qu'il contient l'ARN messager codant pour l'endothiapepsine ; une réaction enzymatique utilisant la transcriptase inverse est effectuée sur l'ARN messager avant l'amplification.

a) Mise en évidence de la présence de l'ARN messager codant pour l'endothiapepsine dans la préparation d'ARN total.

α) <u>Le Northern Blot</u> :

La technique du northern est utilisée (Maniatis). Elle consiste essentiellement à séparer, par électrophorèse, sur gel d'agarose 1,0 % en conditions dénaturantes (MOPS 20 mM pH 7, acétate de sodium 5 mM, EDTA ImM, formaldéhyde 6,6 %) approximativement 10 μg d'ARN total. Les ARN ainsi séparés sont transférés sur une feuille de nitrocellulose (Maniatis op. cit.). On prépare ainsi deux filtres de nitrocellulose différents qui sont hybridés l'un avec la sonde radiomarquée 1 et l'autre avec la sonde radiomarquée 2 (cf. section 1.2) et 3) pour la préparation des sondes et leur marquage au $^{32}$P).

β) <u>Hybridation avec la sonde radiomarquée 1 et la sonde radiomarquée 2</u> :

Les conditions d'hybridation sont les mêmes que celles décrites dans la section 1.5). Après l'hybridation, chacun des filtres est lavé individuellement dans une solution contenant 0,5 x SSC à 42°C. Les filtres sont ensuite exposés à un film photographique (Kodak XAR5) pendant la nuit.

L'analyse des films montre qu'une population d'ARN répond de manière spécifique aux deux sondes, ce qui indique ainsi que l'ARN messager codant pour l'endothiapepsine est présent dans la préparation.

b) La réaction utilisant la transcriptase inverse.

On effectue la réaction de la transcriptage inverse avec 1 µg d'ARN messager en présence de dithiothréitol DTT 10 mM, de RNasine (inhibiteur de RNAse Genofit) 0,0040 U/µl, de mélange des quatre désoxyribonucléotidyltriphosphates dNTP 10 mM, du tampon de composition : 50 mM Tris HCl pH 8,3, 20 mM KCl et 10 mM MgCl$_2$, de 0,7 unité de transcriptase inverse (Stratagene) et de 0,1 ng de l'amorce 1 ainsi que 0,1 ng de l'amorce 2 pour un volume final de 10 µl. Après une demi-heure d'incubation à 46°C, la réaction est arrêtée en ajoutant 20 mM d'EDTA puis le mélange est incubé pendant 5 min à 65°C.

c) La réaction de PCR.

Le mélange préalablement décrit est soumis à une chromatographie sur colonne de gel de polyacrylamide P10 afin d'éliminer les molécules de petite taille (nucléotides, EDTA, etc.). La solution alors obtenue est incubée pendant 2 min à 92°C afin de dénaturer la matrice composée d'un brin d'ADN complémentaire et d'un brin d'ARN messager. Dans le tube, on ajoute alors 100 ng de l'amorce 1, 100 ng de l'amorce 2, 2 mM MgCl$_2$, 0,2 mM de dNTP, 5 µl de mélange réactionnel concentré 10 fois (quantité finale : 67 mM Tris HCl pH 8,8, 16,6 mM (NH$_4$)$_2$ SO$_4$, 1 mM β mercaptoéthanol, 6,7 mM EDTA, 0,15 % Triton X100, 200 g/ml de gélatine).

Le volume du mélange est ensuite porté à 50 µl en ajoutant de l'eau.

Le mélange réactionnel ainsi obtenu est incubé 4 min à 94°C puis amené à la température de 41°C qui est maintenue pendant 4 min. La température de 41°C correspond à la température de demi-dénaturation de l'oligonucléotide diminuée de 5 degrés, calculée avec une formule empirique bien connue de l'homme de l'art.

On ajoute alors 0,5 µl soit 2,5 unités de polymérase Taq (Boehringer Mannheim réf. 1146-165). On coupe alors le mélange réactionnel avec de la paraffine afin d'éviter l'évaporation de la solution aqueuse.

L'amplification se fait au cours de 30 cycles réactionnels dont les étapes d'un cycle sont les suivantes :

2 min à 92°C → dénaturation

2 min à 41°C → hybridation

2 min à 72°C → polymérisation.

Après les 30 cycles, la réaction enzymatique est arrêtée par l'addition de 20 mM EDTA.

Le fragment d'ADN ainsi amplifié, qui présente la taille attendue d'environ 1 300 pb, est ensuite isolé et purifié sur gel d'agarose 1 %, dialysé sur colonne p10 puis hydrolysé simultanément par les enzymes NdeI et HindIII selon les techniques habituelles bien connues de l'homme de l'art (Maniatis, op. cit.) afin de former les extrémités cohésives NdeI et HindIII. Après hydrolyse, le fragment est purifié sur colonne p10.

Section 16 : Construction du plasmide p572, vecteur de clonage et d'expression de l'ADN complémentaire codant pour le précurseur de l'endothiapepsine, dans E. coli. Détermination de la séquence de cet ADN complémentaire et expression de ce dernier.

1) Construction du plasmide p572

Le plasmide p572 a été préparé à partir du plasmide p466, vecteur de clonage et d'expression de l'ADN complémentaire de l'urate oxydase d'Aspergillus flavus dans E. coli, décrit dans la demande de brevet PCT-FR-90-00532, qui comprend un fragment du plasmide pBR327 incluant l'origine de réplication et le gène de résistance à l'ampicilline, un promoteur synthétique d'E. coli (R. RODRIGUEZ et M. CHAMBERLIN "Promoters-Structure and function (1982) Preager), une séquence de Shine-Dalgarno, suivie d'un polylinker présentant les sites uniques NdeI et HindIII, un terminateur de transcription (dérivé du phage fd) et le gène lac i.

a) Construction du plasmide p466.

On a préparé le plasmide p466, vecteur d'expression dans E. coli. Ce dernier comprend un fragment de pBR327 incluant l'origine de réplication et le gène de résistance à l'ampicilline, il comprend également un promoteur synthétique d'E. coli (R. RODRIGUEZ et M. CHAMBERLIN "Promoters-Structure and function (1982) Preager), une séquence de Shine-Dalgarno, suivi d'un polylinker présentant les sites uniques NdeI et KpnI, un terminateur de transcription (dérivé du phage fd) et le gène lac i.

Ce plasmide a été construit à partir d'un plasmide d'expression de l'hGH dans E. coli (p462) par substitution d'un fragment portant le gène de l'hGH par l'ADNc de l'urate oxydase.

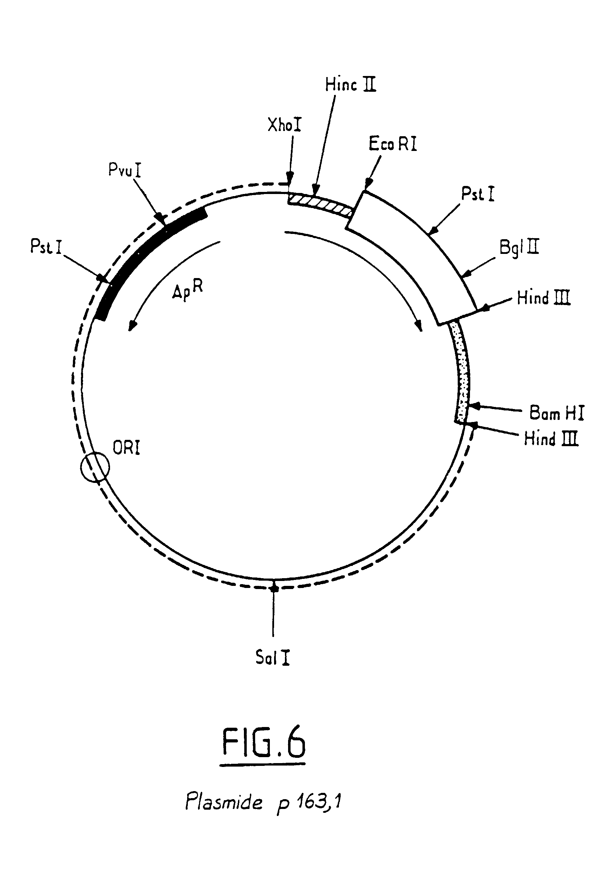

La construction du plasmide p466 va maintenant être décrite plus en détail dans l'exposé ci-après dans lequel il sera fait référence aux figures 6, 7, 8, 9, 10.

b) Construction du plasmide p373,2

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par T. MANIATIS EF, FRITSCH et J. SAMBROOK, Cold Spring Harbor Laboratory (1982). La synthèse des oligonucléotides est réalisée à l'aide d'un synthétiseur d'ADN Biosearch 4 600.

On a soumis le plasmide p163,1 (figure 6), décrit dans la demande de brevet EP-A-0245138 et déposée à la CNCM sous la référence I-530 le 17 février 1986, à une digestion par les enzymes PvuI et BamHI. Ce plasmide contient le gène codant pour l'hGH. Le fragment PvuI-BamHI - ci-après fragment 1 - contenant le site d'action de l'enzyme de restriction XhoI, représenté sur la figure 6, a été purifié.

De même, on a soumis le plasmide pBR327, bien connu de l'homme de l'art, (cf. SOBERON, X et al., Gene, 9 (1980) 287-305) à une digestion par les enzymes PvuI et BamHI. Le fragment PvuI-BamHI - ci-après fragment 2 -, contenant l'origine de réplication, a été purifié.

Puis on a préparé le fragment 3 qui est un fragment synthétique BamHI(1)-BamHI(2) contenant le gène lac i et son promoteur dont la séquence est la suivante sur laquelle les deux extrémités du brin sont repérées par les nombres 1 et 2 pour préciser l'orientation du fragment dans les plasmides décrits aux figures 7 et 8.

FRAGMENT 3

BamHI(1)

```
5'        GATCC GCGGAAGCAT AAAGTGTAAA GCCTGGGGTG CCTAATGAGT
          GAGCTAACTT ACATTAATTG CGTTGCGCTC ACTGCCCGCT TTCCAGTCGG
          GAAACCTGTC GTGCCAGCTG CATTAATGAA TCGGCCAACG CGCGGGGAGA
          GGCGGTTTGC GTATTGGGCG CCAGGGTGGT TTTTCTTTTC ACCAGTGAGA
          CGGGCAACAG CTGATTGCCC TTCACCGCCT GGCCCTGAGA GAGTTGCAGC
          AAGCGGTCCA CGCTGGTTTG CCCCACCACC CGAAAATCCT GTTTGATGGT
          GGTTAACGGC GGGATATAAC ATGAGCTGTC TTCGGTATCG TCGTATCCCA
          CTACCGAGAT ATCCGCACCA ACGCGCAGCC CGGACTCGGT AATGGCGCGC
          ATTGCGCCCA GCGCCATCTG ATCGTTGGCA ACCAGCATCG CAGTGGGAAC
          GATGCCCTCA TTCAGCATTT GCATGGTTTG TTGAAAACCG GACATGGCAC
          TCCAGTCGCC TTCCCGTTCC GCTATCGGCT GAATTTGATT GCGAGTGAGA
          TATTTATGCC AGCCAGCCAG ACGCAGACGC GCCGAGACAG AACTTAATGG
          GCCCGCTAAC AGCGCGATTT GCTGGTGACC CAATGCGACC AGATGCTCCA
          CGCCCAGTCG CGTACCGTCT TCATGGGAGA AAATAATACT GTTGATGGGT
          GTCTGGTCAG AGACATCAAG AAATAACGCC GGAACATTAG TGCAGGCAGC
          TTCCACAGCA ATGGCATCCT GGTCATCCAG CGGATAGTTA ATGATCAGCC
          CACTGACGCG TTGCGCGAGA AGATTGTGCA CCGCCGCTTT ACAGGCTTCG
          ACGCCGCTTC GTTCTACCAT CGACACCACC ACGCTGGCAC CCAGTTGATC
          GGCGCGAGAT TTAATCGCCG CGACAATTTG CGACGGCGCG TGCAGGGCCA
          GACTGGAGGT GGCAACGCCA ATCAGCAACG ACTGTTTGCC CGCCAGTTGT
          TGTGCCACGC GGTTGGGAAT GTAATTCAGC TCCGCCATCG CCGCTTCCAC
          TTTTTCCCGC GTTTTCGCAG AAACGTGGCT GGCCTGGTTC ACCACGCGGG
          AAACGGTCTG ATAACAGACA CCGGCATACT CTGCGACATC GTATAACGTT
          ACTGGTTTCA CATTCACCAC CCTGAATTGA CTCTCTTCCG GGCGCTATCA
          TGCCATACCG CGAAAGGTTT TGCGCCATTC GATGGTGTCC G                3'
                                                    BamHI(2)
```

Les fragments 1, 2 et 3 ont été alors ligués de manière à obtenir le plasmide p160 représenté sur la figure 7.
Ce plasmide a été soumis à une digestion partielle par les enzymes de restriction HincII et PstI. Le grand fragment HincII-PstI, contenant l'origine de réplication et représenté sur la figure 7, a été ensuite ligué au fragment 4 représenté ci-après, qui est un fragment synthétique d'ADN portant une séquence codant pour les 44 premiers acides aminés d'un précurseur naturel de l'hGH et en amont de cette séquence des signaux de régulation.

FRAGMENT 4

```
                                                                    ClaI
                                                                     ▼
              5'        TCGAGCTGACTGACCTGTTGCTTATATTACATCGA
                        ------------------------------------

                        AGCTCGACTGACTGGACAACGAATATAATGTAGCT
                                                           ▲
                                                          NdeI
                                                           ▼
   TAGCGTATAATGTGTGGAATTGTGAGCGATAACAATTTCACACAGTTTAACTTTAAGAAGGAGATATACAT
   ----------------------------------------------------------------------

   ATCGATATTACACACCTTAACACTCGCCTATTGTTAAAGTGTGTCAAATTGAAATTCTTCCTCTATATGTA


   ATG GCT ACC GGA TCC CGG ACT AGT CTG CTC CTG GCT TTT GGC CTG CTC TGC CTG
   ------------------------------------------------------------------------


   TAC CGA TGG CCT AGG GCC TGA TCA GAC GAG GAC CGA AAA CCG GAC GAC ACG GAC
   ▲
   M   A   T   G   S   R   T   S   L   L   L   A   F   G   L   L   C   L
  -26


                                                            XbaI
                                                             ▼
   CCC TGG CTT CAA GAG GGC AGT GCC TTC CCA ACC ATT CCC TTA TCT AGA CTT TTT
       ----------------------------------------------------------------

   GGG ACC GAA GTT CTC CCG TCA CGG AAG GGT TGG TAA GGG AAT AGA TCT GAA AAA
   P   W   L   Q   E   G   S   A   F   P   T   I   P   L   S   R▲  L   F
                                           -1   1


   GAC AAC GCT ATG CTC CGC GCC CAT CGT CTG CAC CAG CTG GCC TTT GAC ACC TAC
   ------------------------------------------------------------------------

   CTG TTG CGA TAC GAG GCG CGG GTA GCA GAC GTG GTC GAC CGG AAA CTG TGG ATC
   D   N   A   M   L   R   A   H   R   L   H   Q   L   A   F   L   T   Y
                                                                      PstI
   CAG GAG TTT GAA GAA GCC TAT ATC CCA AAG GAA CAG AAG TAT TCA TTC CTG CA
   ----------------------------------------------------------------------

   GTC CTC AAA CTT CTT CGG ATA TAG GGT TTC CTT GTC TTC ATA AGT AAG G
   Q   E   F   E   E   A   Y   I   P   K   E   Q   K   Y   S   F
                                                                   44
```

Dans ce fragment, les acides aminés sont désignés par des lettres selon le code suivant :

| | |
|---|---|
| A = Alanine | M = Méthionine |
| C = Cystéine | N = Asparagine |
| D = Acide aspartique | P = Proline |
| E = Acide glutamique | Q = Glutamine |
| F = Phénylalanine | R = Arginine |
| G = Glycine | S = Sérine |
| H = Histidine | T = Thréonine |
| I = Isoleucine | V = Valine |
| K = Lysine | W = Tryptophane |
| L = Leucine | Y = Tyrosine |

Sont successivement soulignées dans ce fragment les séquences -35 (TTGCTT) et -10 (TATAAT) de la séquence promotrice, et la séquence de Shine et Dalgarno bien connue de l'homme de l'art.

Le plasmide p380,1 a été ainsi obtenu.

Le plasmide p380,1 a été ensuite soumis à une digestion par les enzymes de restriction ClaI et NdeI de manière à en éliminer le petit fragment ClaI-NdeI du fragment 4 ci-dessus et à lui substituer le fragment ClaI-NdeI ci-après :

```
                    ClaI

        5'          CGATAGCGTATAATGTGTGGAATTGTGAGCGGATAACA

                    TATCGCATATTACACACCTTAACACTCGCCTATTGT



                                                NdeI

        ATTTCACACAGTTTTTCGCGAAGAAGGAGATATACA

        TAAAGTGTGTCAAAAAGCGCTTCTTCCTCTATATGTAT          5'
```

Le plasmide résultant est le plasmide p373,2 (figure 8).

2) Construction du plasmide p466 :

Le plasmide p373,2 a été soumis à une double digestion par les enzymes BglII et HindIII. Le grand fragment issu de cette digestion a été purifié et ligué avec un fragment d'ADN synthétique, dont la séquence donnée ci-après est destinée à reconstituer la fin du gène de l'hGH suivie en 3' des sites de clonage KpnI et SnaBI.

```
                    B

                    g

                    l

                    I

                    I

                  GATCTTCAAGCAGACCTACAGCAAGTTCGACACAAACTCACACAACGAT

                  ----+---------+---------+---------+---------+

                  AAGTTCGTCTGGATGTCGTTCAAGCTGTGTTTGAGTGTGTTGCTA
GACGCACTACTCAAGAACTACGGGCTGCTCTACTGCTTCAGGAAGGACATGGACAAGGTC

---------+---------+---------+---------+---------+---------+

CTGCGTGATGAGTTCTTGATGCCCGACGAGATGACGAAGTCCTTCCTGTACCTGTTCCAG


                    F

                    s

                    p

                    I

GAGACATTCCTGCGCATCGTGCAGTGCCGCTCTGTGGAGGGCAGCTGTGGCTTCTAGTAA

---------+---------+---------+---------+---------+---------+

CTCTGTAAGGACGCGTAGCACGTCACGGCGAGACACCTCCCGTCGACACCGAAGATCATT


                    H

                    i

          S         n

     K    n         d

     p    a         I

     n    B         I

     I    I         I

GGTACCCTGCCCTACGTACCA

---------+---------+-----

CCATGGGACGGGATGCATGGTTCGA
```

Ce fragment comprend les extrémités cohésives BglII et HindIII. Le nouveau plasmide ainsi formé p462 (Cf. Fig. 9) comprend ainsi un site KpnI et un site NdeI qui vont servir au clonage du fragment portant l'ADNc de l'urate oxydase dans le vecteur d'expression.

Le plasmide hybride dérivé de pTZ19R portant l'ADNc d'environ 1,2 Kb (clone 9C), de l'urate oxydase comprend un site KpnI unique. Ce site est localisé quelques paires de bases en aval du site de clonage de l'ADNc. D'autre part l'ADNc de l'urate oxydase contient un site AccI situé à proximité de l'extrémité 5'.

On a donc isolé et purifié le fragment AccI-KpnI comprenant la plus grande partie de cet ADNc. D'autre part on a synthétisé deux oligonucléotides complémentaires dont la séquence donnée ci-après:

```
5'-TATGTCTGCG GTAAAAGCAG CGCGCTACGG CAAGGACAAT GTTCGCGT
       ACAGACGCCA TTTTCGTCGC GCGATGCCGT TCCTGTTACA AGCGCAGA-5'
```

est destinée à reconstituer l'extrémité 5' de l'ADNc. Ce fragment synthétique ainsi obtenu possède une extrémité NdeI et une autre AccII. Le fragment et la séquence synthétique ont été ligués avec le vecteur d'expression coupé par KpnI et par NdeI. Cette ligation à trois fragments permet d'obtenir le vecteur d'expression nommé p466, de l'urate oxydase pour E. coli (Cf. figure 10). Ce plasmide a été soumis à une série d'hydrolyses enzymatiques par des enzymes de restriction, qui a permis de vérifier la présence des sites de restriction attendus, en particulier ceux portés par le gène codant pour l'urate oxydase.

Le plasmide p466 contient donc par construction un gène codant pour l'urate oxydase de séquence ci-après :

```
ATGTCTGCGG TAAAAGCAGC GCGCTACGGC AAGGACAATG TTCGCGTCTA
CAAGGTTCAC AAGGACGAGA AGACCGGTGT CCAGACGGTG TACGAGATGA
CCGTCTGTGT GCTTCTGGAG GGTGAGATTG AGACCTCTTA CACCAAGGCC
GACAACAGCG TCATTGTCGC AACCGACTCC ATTAAGAACA CCATTTACAT
CACCGCCAAG CAGAACCCCG TTACTCCTCC CGAGCTGTTC GGCTCCATCC
TGGGCACACA CTTCATTGAG AAGTACAACC ACATCCATGC CGCTCACGTC
AACATTGTCT GCCACCGCTG GACCCGGATG GACATTGACG GCAAGCCACA
CCCTCACTCC TTCATCCGCG ACAGCGAGGA GAAGCGGAAT GTGCAGGTGG
ACGTGGTCGA GGGCAAGGGC ATCGATATCA AGTCGTCTCT GTCCGGCCTG
ACCGTGCTGA AGAGCACCAA CTCGCAGTTC TGGGGCTTCC TGCGTGACGA
GTACACCACA CTTAAGGAGA CCTGGGACCG TATCCTGAGC ACCGACGTCG
ATGCCACTTG GCAGTGGAAG AATTTCAGTG GACTCCAGGA GGTCCGCTCG
CACGTGCCTA AGTTCGATGC TACCTGGGCC ACTGCTCGCG AGGTCACTCT
GAAGACTTTT GCTGAAGATA ACAGTGCCAG CGTGCAGGCC ACTATGTACA
AGATGGCAGA GCAAATCCTG GCGCGCCAGC AGCTGATCGA GACTGTCGAG
TACTCGTTGC CTAACAAGCA CTATTTCGAA ATCGACCTGA GCTGGCACAA
GGGCCTCCAA AACACCGGCA AGAACGCCGA GGTCTTCGCT CCTCAGTCGG
ACCCCAACGG TCTGATCAAG TGTACCGTCG GCCGGTCCTC TCTGAAGTCT
AAATTG.
```

(Les nucléotides différents des nucléotides de l'ADNc isolé d'A. flavus sont soulignés dans la séquence ci-dessus. Ces différences ont été introduites sur le fragment synthétique AccI-KpnI de façon à avoir en aval de l'ATG une séquence nucléotidique plus conforme à celles habituellement rencontrées dans un gène procaryote).

Le plasmide p466 a été hydrolysé par les enzymes NdeI et HindIII et le fragment portant le gène lac i, l'origine de réplication et le gène codant pour la résistance à l'ampicilline a été purifié selon les techniques connues de l'homme de l'art (Maniatis, op. cit.).

Ce fragment a été ligué au fragment d'ADN complémentaire amplifié préalablement hydrolysé par les endonucléases NdeI et HindIII. Le produit de cette ligation a servi à transformer dans la souche E. coli K12 RRI (Gibco-BRL - réf. : 520-8261 SA). On a retenu un transformant, appelé clone 512, qui contient le plasmide appelé p572.

2) Détermination de la séquence de l'ADN complémentaire

Le plasmide p572 a été hydrolysé d'une part par les endonucléases ClaI et KpnI, d'autre part par les enzymes KpnI et HindIII. (L'endonucléase KpnI coupe la séquence codante du fragment A). Le fragment ClaI-KpnI portant l'extrémité 5' de l'ADN codant pour la protéine et le fragment KpnI-HindIII portant l'extrémité 3' de l'ADN codant pour la protéine ont été clonés dans le phage M13mp19 (Pharmacia) et séquencés par la technique du cyclone ("Cyclone I Biosystem" de IBI).

La séquence nucléotidique de l'ADN complémentaire ainsi obtenue est représentée sur la figure 11. On constate que la séquence codante de l'ADN complémentaire est exactement identique à celle de l'ADN génomique, à la différence près que cette dernière est interrompue par trois introns, lesquels ont été correctement localisés (cf. section 1.7)).

3) Expression de l'ADN complémentaire de la préproendothiapepsine

La souche E. coli K12 RRI (Gibco BRL-Réf. : 520-8261A) a été transformée pour la résistance à l'ampicilline avec le plasmide p572 portant l'ADN complémentaire de la préproendothiapepsine, dont on a déterminé la séquence en 2), et avec un plasmide témoin négatif pBR322.

Des colonies résistantes à l'ampicilline ont été obtenues dans les deux cas.

1 colonie de chaque type a été mise en culture dans du milieu LB liquide (de composition précisée dans le tableau 4, mais sans agar) additionné d'ampicilline 100 $\mu$g/ml. Après une nuit à 37°C sous agitation, les deux cultures ont été diluées 100 fois dans du milieu liquide LB additionné d'ampicilline 100 $\mu$g/ml. Après 1 h de culture, on ajoute de l'IPTG (Isopropyl $\beta$-DThiogalactoside) jusqu'à 1 mM pendant 3 h.

Immunodétection de la préproendothiapepsine par Western Blot :

a) Mode opératoire

On prélève du milieu de culture obtenu après 3 h d'induction à l'IPTG une fraction aliquote correspondant à 0,2 ml à D0 = 1. On centrifuge cette dernière et on élimine le surnageant. On soumet ensuite le culot à un Western Blot, technique bien connue de l'homme de l'art, qui comprend les étapes suivantes :

- Solubilisation du culot par ébullition pendant 10 min dans un tampon dénommé tampon de charge constitué de Tris HCl 0,125 M pH 6,8 SDS 4 %, bleu de bromophénol 0,002 %, glycérol 20 %, $\beta$-mercaptoéthanol 10 % (selon le protocole décrit par LAEMMLI (U.K. LAEMMLI, Nature, 227 (1970), 680-685)),

- Séparation électrophorétique des différentes protéines contenues dans le solubilisat selon le protocole décrit par LAEMMLI (U.K. LAEMMLI, Nature, 227, (1970), 680-685),

- Transfert desdites protéines contenues dans le gel sur un filtre de nitrocellulose (selon la technique de H. TOWBIN et al. Proc. Natl. Acad. Sci. USA 76 (1979) 4350-4354),

- Immunodétection, réalisée selon la technique de BURNETTE (W.W. BURNETTE Ana. Biochem. 112 (1981) 195-203), elle implique successivement :

  . Le rinçage du filtre de nitrocellulose pendant 10 min avec un tampon de composition Tris-HCl 10 mM, NaCl 170 mM, KCl 1 mM.
  . La mise en contact du filtre de nitrocellulose pendant 30 min à 37°C avec du tampon A additionné de sérum albumine bovine à raison de 3 g pour 100 ml.
  . La mise en contact du filtre de nitrocellulose pendant 1 h à 37°C avec les anticorps polyclonaux du kit Rennetest France Biochem, conformément à la méthode d'identification de l'endothiapepsine décrite dans le Journal Officiel de la République Française du 20 mars 1981.
  . Le rinçage du filtre de nitrocellulose avec le tampon A, additionné de 3 g/100 ml de sérum albumine bovine.
  . La mise en contact du filtre de nitrocellulose pendant 1 h à 37°C avec une solution de protéine G marquée à l'iode 125 à 0,1 microcurie/ml.
  . Le rinçage du filtre avec le tampon A.
  . Le séchage du filtre entre deux feuilles absorbantes.
  . La mise en contact d'un film radiographique.
  . La révélation du film.

b) Résultats :

On constate que la souche transformée par le plasmide p572 surproduit une protéine de poids moléculaire apparent d'environ 43 kDa correspondant à la masse moléculaire attendue de la préproendothiapepsine, qui est reconnue par les anticorps dirigés contre l'endothiapepsine qui est absente dans la souche témoin.

**Revendications**

1. Cassette d'expression d'un précurseur de l'endothiapepsine dans Cryphonectria parasitica, caractérisée en ce qu'elle comprend le promoteur du gène codant pour la préproendothiapepsine ou une partie fonctionnelle de ce promoteur, ledit promoteur étant en amont d'une séquence codant pour le précurseur de l'endothiapepsine, laquelle a la séquence (P1) suivante :

```
Ser Thr Gly Ser Ala Thr Thr Thr Pro Ile Asp Ser Leu Asp Asp Ala Tyr
Ile Thr Pro Val Gln Ile Gly Thr Pro Ala Gln Thr Leu Asn Leu Asp Phe
Asp Thr Gly Ser Ser Asp Leu Trp Val Phe Ser Ser Glu Thr Thr Ala Ser
Glu Val Asp Gly Gln Thr Ile Tyr Thr Pro Ser Lys Ser Thr Thr Ala Lys
Leu Leu Ser Gly Ala Thr Trp Ser Ile Ser Tyr Gly Asp Gly Ser Ser Ser
Ser Gly Asp Val Tyr Thr Asp Thr Val Ser Val Gly Gly Leu Thr Val Thr
Gly Gln Ala Val Glu Ser Ala Lys Lys Val Ser Ser Ser Phe Thr Glu Asp
Ser Thr Ile Asp Gly Leu Leu Gly Leu Ala Phe Ser Thr Leu Asn Thr Val
Ser Pro Thr Gln Gln Lys Thr Phe Phe Asp Asn Ala Lys Ala Ser Leu Asp
Ser Pro Val Phe Thr Ala Asp Leu Gly Tyr His Ala Pro Gly Thr Tyr Asn
Phe Gly Phe Ile Asp Thr Thr Ala Tyr Thr Gly Ser Ile Thr Tyr Thr Ala
Val Ser Thr Lys Gln Gly Phe Trp Glu Trp Thr Ser Thr Gly Tyr Ala Val
Gly Ser Gly Thr Phe Lys Ser Thr Ser Ile Asp Gly Ile Ala Asp Thr Gly
Thr Thr Leu Leu Tyr Leu Pro Ala Thr Val Val Ser Ala Tyr Trp Ala Gln
Val Ser Gly Ala Lys Ser Ser Ser Ser Val Gly Gly Tyr Val Phe Pro Cys
Ser Ala Thr Leu Pro Ser Phe Thr Phe Gly Val Gly Ser Ala Arg Ile Val
Ile Pro Gly Asp Tyr Ile Asp Phe Gly Pro Ile Ser Thr Gly Ser Ser Ser
Cys Phe Gly Gly Ile Gln Ser Ser Ala Gly Ile Gly Ile Asn Ile Phe Gly
Asp Val Ala Leu Lys Ala Ala Phe Val Val Phe Asn Gly Ala Thr Thr Pro
Thr Leu Gly Phe Ala Ser Lys,
```

le précurseur de l'endothiapepsine étant la préproendothiapepsine de séquence (P4) suivante :

```
Met Ser Ser Pro Leu Lys Asn Ala Leu Val Thr Ala Met Leu Ala Gly Gly
Ala Leu Ser Ser Pro Thr Lys Gln His Val Gly Ile Pro Val Asn Ala Ser
Pro Glu Val Gly Pro Gly Lys Tyr Ser Phe Lys Gln Val Arg Asn Pro Asn
Tyr Lys Phe Asn Gly Pro Leu Ser Val Lys Lys Thr Tyr Leu Lys Tyr Gly
Val Pro Ile Pro Ala Trp Leu Glu Asp Ala Val Gln Asn Ser Thr Ser Gly
Leu Ala Glu Arg Ser Thr Gly Ser Ala Thr Thr Thr Pro Ile Asp Ser Leu
Asp Asp Ala Tyr Ile Thr Pro Val Gln Ile Gly Thr Pro Ala Gln Thr Leu
Asn Leu Asp Phe Asp Thr Gly Ser Ser Asp Leu Trp Val Phe Ser Ser Glu
Thr Thr Ala Ser Glu Val Asp Gly Gln Thr Ile Tyr Thr Pro Ser Lys Ser
Thr Thr Ala Lys Leu Leu Ser Gly Ala Thr Trp Ser Ile Ser Tyr Gly Asp
Gly Ser Ser Ser Ser Gly Asp Val Tyr Thr Asp Thr Val Ser Val Gly Gly
Leu Thr Val Thr Gly Gln Ala Val Glu Ser Ala Lys Lys Val Ser Ser Ser
Phe Thr Glu Asp Ser Thr Ile Asp Gly Leu Leu Gly Leu Ala Phe Ser Thr
Leu Asn Thr Val Ser Pro Thr Gln Gln Lys Thr Phe Phe Asp Asn Ala Lys
Ala Ser Leu Asp Ser Pro Val Phe Thr Ala Asp Leu Gly Tyr His Ala Pro
Gly Thr Tyr Asn Phe Gly Phe Ile Asp Thr Thr Ala Tyr Thr Gly Ser Ile
Thr Tyr Thr Ala Val Ser Thr Lys Gln Gly Phe Trp Glu Trp Thr Ser Thr
Gly Tyr Ala Val Gly Ser Gly Thr Phe Lys Ser Thr Ser Ile Asp Gly Ile
Ala Asp Thr Gly Thr Thr Leu Leu Tyr Leu Pro Ala Thr Val Val Ser Ala
Tyr Trp Ala Gln Val Ser Gly Ala Lys Ser Ser Ser Ser Val Gly Gly Tyr
Val Phe Pro Cys Ser Ala Thr Leu Pro Ser Phe Thr Phe Gly Val Gly Ser
Ala Arg Ile Val Ile Pro Gly Asp Tyr Ile Asp Phe Gly Pro Ile Ser Thr
Gly Ser Ser Ser Cys Phe Gly Gly Ile Gln Ser Ser Ala Gly Ile Gly Ile
Asn Ile Phe Gly Asp Val Ala Leu Lys Ala Ala Phe Val Val Phe Asn Gly
Ala Thr Thr Pro Thr Leu Gly Phe Ala Ser Lys.
```

**2.** Cassette selon la revendication 1, caractérisée en ce que la séquence codant pour la préproendothiapepsine comprend la séquence (N4a) suivante :

```
ATGTCTT CCCCTCTCAA GAACGCCTTG GTGACCGCCA TGTTGGCTGG

TGGTGCTCTC AGCTCGCCTA CAAAGCAACA CGTTGGAATT CCCGTCAACG

CCTCTCCTGA AGTTGGCCCC GGAAAGTACT CGTTCAAGCA AGTCCGGAAC

CCCAACTACA AGTTCAACGG GCCTCTGTCG GTCAAGAAGA CGTACCTCAA

GTACGGCGTG CCGATCCCAG CCTGGCTGGA GGATGCTGTC CAGAACTCTA

CCTCGGGCCT GGCTGAGCGC TCGACCGGTT CTGCGACCAC AACTCCCATC

GACAGCCTCG ATGATGCTTA CATCACTCCG GTTCAGATCG GCACCCCTGC

GCAGACTCTG AACCTGGACT TTGACACTGG ATCTTCGGAT CTGTGGGTCT

TCAGCAGCGA GACTACAGCC AGCGAGGTCG ATGGGCAGAC CATCTACACC

CCCAGCAAGA GCACCACCGC CAAGCTGCTG TCGGCGCTAC CTGGTCCATC

TCCTACGGAG ACGGTAGCTC TTCCAGCGGC GATGTCTACA CTGACACCGT

CTCGGTTGGA GGCCTTACCG TGACGGGCCA GGCTGTCGAG TCGGCCAAGA

AGGTTTCTTC CAGCTTCACC GAGGACTCGA CCATTGACGG TCTCCTGGGC

CTGGCCTTCA GCACCCTGAA CACTGTGTCG CCTACCCAGC AAAAGACTTT

CTTCGACAAT GCGAAGGCGT CCTTGGACTC GCCTGTGTTC ACGGCTGATC

TTGGCTACCA TGCCCCTGGT ACCTACAACT TCGGCTTCAT CGATACCACT

GCCTACACGG GCTCCATCAC CTACACCGCT GTCTCGACCA AGCAAGGGTT

CTGGGAGTGG ACTTCGACCG GCTACGCCGT CGGCTCCGGC ACCTTCAAGT

CGACTTCCAT CGACGGCATC GCTGACACTG GCACGACCCT CCTGTACCTC

CCTGCCACCG TCGTGTCGGC CTACTGGGCC CAGGTCTCGG GCGCCAAGTC

CAGCTCTTCC GTCGGCGGCT ACGTCTTCCC CTGCAGCGCG ACCCTGCCTT

CCTTCACCTT CGGCGTTGGC TCAGCTCGCA TTGTGATTCC TGGCGACTAC

ATTGATTTCG GCCCCATCTC CACTGGAAGC TCGTCTTGCT TTGGCGGCAT

CCAGTCCAGC GCTGGTATCG GCATCAACAT CTTCGGTGAT GTCGCTCTGA

AGGCTTTGTC GTCTTCAACG GGGCTACAAC TCCCACTCTT GGCTTTGCTT

CCAAG
```

**3.** Cassette selon la revendication 1, caractérisée en ce que la séquence codant pour la préproendothiapepsine comprend la séquence (N4a) interrompue par au moins un intron.

**4.** Cassette selon la revendication 3, caractérisée en ce que la séquence codant pour la préproendothiapepsine est la séquence (N4b) suivante :

```
                                       AT GTCTTCCCCT CTCAAGAACG
          CCTTGGTGAC CGCCATGTTG GCTGGTGGTG CTCTCAGCTC GCCTACAAAG
          CAACACGTTG GAATTCCCGT CAACGCCTCT CCTGAAGTTG GCCCCGGAAA
          GTACTCGTTC AAGCAAGGTG AGTAGAGCTG CTTCTGTGTG TTGCAACAGA
          AGACCAACGC AAAAAGAAGA GGTCAAGCCA AGACGGATAT TTTACTGACA
          ATTATACTTT TGAAGTCCGG AACCCCAACT ACAAGTTCAA CGGGCCTCTG
          TCGGTCAAGA AGACGTACCT CAAGTACGGC GTGCCGATCC CAGCCTGGCT
          GGAGGATGCT GTCCAGAACT CTACCTCGGG CCTGGCTGAG CGCTCGACCG
          GTTCTGCGAC CACAACTCCC ATCGACAGCC TCGATGATGC TTACATCACT
          CCGGTTCAGA TCGGCACCCC TGCGCAGACT CTGAACCTGG ACTTTGACAC
          TGGATCTTCG GATCTGTGGG TCTTCAGCAG CGAGACTACA GCCAGCGAGG
          TTGGTCAACC CTCGCCCGCA TTTTATTGCA TACATTTTTA GTTTTTTTGG
          TAATCAGAAT ACTAACATTG GGAATTTCCC AACTGTAGGT CGATGGGCAG
          ACCATCTACA CCCCCAGCAA GAGCACCACC GCCAAGCTGC TGTCGGGCGC
          TACCTGGTCC ATCTCCTACG GAGACGGTAG CTCTTCCAGC GGCGATGTCT
          ACACTGACAC CGTCTCGGTT GGAGGCCTTA CCGTGACGGG CCAGGCTGTC
          GAGTCGGCCA AGAAGGTTTC TTCCAGCTTC ACCGAGGACT CGACCATTGA
          CGGTCTCCTG GGCCTGGCCT TCAGCACCCT GAACACTGTG TCGCCTACCC
          AGCAAAAGAC TTTCTTCGAC AATGCGAAGG CGTCCTTGGA CTCGCCTGTG
          TTCACGGCTG ATCTTGGCTA CCATGCCCGT GAGTGACCCC TCTTGATACA
          TATACTTTTT GATGAATCTT GTTGGAGAAG CATTCCCCAC TAATATGGAA
          ATTGTTTGTA TCTACAGCTG GTACCTACAA CTTCGGCTTC ATCGATACCA
          CTGCCTACAC GGGCTCCATC ACCTACACCG CTGTCTCGAC CAAGCAAGGG
          TTCTGGGAGT GGACTTCGAC CGGCTACGCC GTCGGCTCCG GCACCTTCAA
          GTCGACTTCC ATCGACGGCA TCGCTGACAC TGGCACGACC CTCCTGTACC
          TCCCTGCCAC CGTCGTGTCG GCCTACTGGG CCCAGGTCTC GGGCGCCAAG
          TCCAGCTCTT CCGTCGGCGG CTACGTCTTC CCCTGCAGCG CGACCCTGCC
          TTCCTTCACC TTCGGCGTTG GCTCAGCTCG CATTGTGATT CCTGGCGACT
          ACATTGATTT CGGCCCCATC TCCACTGGAA GCTCGTCTTG CTTTGGCGGC
          ATCCAGTCCA GCGCTGGTAT CGGCATCAAC ATCTTCGGTG ATGTCGCTCT
          GAAGGCCGCC TTTGTCGTCT TCAACGGGGC TACAACTCCC ACTCTTGGCT
          TTGCTTCCAA G
```

**5.** Cassette selon la revendication 1, caractérisée en ce que la partie fonctionnelle du promoteur du gène codant pour la préproendothiapepsine est le segment BglII-ScaI du fragment C, ledit fragment C étant une partie de l'ADN génomique de <u>Cryphonectria parasitica</u> contenue dans la souche <u>d'E.coli</u> déposée à la CNCM le 31/08/90 sous le n° I-998.

**6.** Cassette selon la revendication 1, caractérisée en ce que la partie fonctionnelle du promoteur du gène codant pour la préproendothiapepsine est le segment BamHI-ScaI du fragment C, ledit fragment C étant une partie de l'ADN génomique de <u>Cryphonectria parasitica</u> contenue dans la souche <u>d'E.coli</u> déposée à la CNCM le 31/08/90 sous le n° I-998.

**7.** Cassette selon la revendication 1, caractérisée en ce que le promoteur comprend une partie portant la boîte TATA de la séquence (N5) suivante :

```
AAGCTTATCC GCCGCCGGCG GGGGAATTCT ATTGAACTTG TTCGAATCAT
TGGTCCGTGG TCTTTTCGTC CATGCGGGCT CCGCTGGCGG ATGAATGACC
TTCTGGCTTC TAGCCTGGCG AAGCGATGTT ACTCTGTTGT CTATACTATA
CGATATGGTC AAGAGAGCAC ATGTGCCGCC AGATGAAGAC ATGTATATAA
AAGGAGTGGC CTCGACGGTT GCTCAACCAT CTTCTGTCTG TCCCAACGCC
ATCGACTCTT CAACTTCTCC TTCGTGTTCC ACCACCATCA CCTTGCTCCA
GACTTAGGAC TTTCAGCAAC CTTCAAAG
```

et en amont de la séquence (N5), un segment X du fragment C compris entre l'extrémité 5' du fragment A ct l'extrémité 5' du fragment C, choisi de façon que le segment X contienne une région activatrice.

**8.** Cassette selon la revendication 7, caractérisée en ce que le segment X du fragment C est le fragment de séquence suivante :

```
GCATGCTTGG CTCTTTAACG TCCTGCCCAT TCAGGGCCTT CAGCCGGCAC
TGGTCCTTCA TCAAGGGGGA CCTCATGACC ATGAACTAAT CTGTGATATC
TGATATATTC TAGAAGGCTT GGCTCCTCAA AGTTTCCAGC TAATGAATCA
GCGGCCCGCC GCCCTTAAAC CGCATCAGGC AAGTCGTTTG GTGTTGCCAG
GCGATGGCGA CAGGAGAGTG GTGTTGATGG GACAAGGGGA GGGAGGCTTA
GCCGACTTCA TCCATAGCAC CCACCTGCTT GGCGCCGATA AGTCTGACGA
TCCGCTTGAG CTGCAAAACG GCTCCTTGAC CTTTGTTTGG TCGACCGAGG
GAAATAGTCT CTTTTTGCGT GATCGTGCGC GCTTCGTATA GCAATAGCAG
CCAGCACCAG CAGGACGGGC CGTTGTCACG GTCACATCGT TCGCAACATG
CCGAGCGTAG GGATGAACGA ATGACTCGAG CCTTGCCTGA CAGTCTGGCA
ATCAATCTAT GGTCACGCAC GATCACAAGC CAATCGCTGT GACTGCGTTA
CTAGCCCAAT AATCCCTTGT TCGATCAGAG TGTTCTACAG ACTTCAAGTG
AGGTTCAC
```

**9.** Cassette selon l'une des revendications 1 à 5, caractérisée en ce que le promoteur provient d'un gène exprimé dans <u>Cryphonectria parasitica</u> ou dans un autre champignon filamenteux du groupe des ascomycètes.

**10.** Cassette selon la revendication 9, caractérisée en ce que le promoteur provient du gène codant pour la glycéraldéhyde-3-phosphate déshydrogénase <u>d'Aspergillus nidulans.</u>

**11.** Souche de <u>Cryphonectria parasitica</u> productrice d'endothiapepsine, caractérisée en ce qu'elle est transformée par une cassette selon l'une des revendications 1 à 10 et elle surproduit l'endothiapepsine par rapport à la souche non transformée.

12. Souche selon la revendication 11, caractérisée en ce qu'elle est dépourvue de marqueur de sélection dominant.

13. Souche selon l'une des revendications 11 et 12, caractérisée en ce que la souche hôte est la souche SEBR 103, déposée à la CNCM le 31.08.1990 sous le No I-997.

14. Souche selon la revendication 13, caractérisée en ce qu'elle surproduit l'endothiapepsine par rapport à la souche SEBR 103, avec un facteur de surproduction au moins égal à 2.

15. Procédé de préparation de l'endothiapepsine, caractérisé en ce qu'il comprend l'étape de culture d'une souche selon l'une des revendications 11 à 14, suivie d'une étape d'isolement et de purification de cette protéine.

16. Procédé pour obtenir une souche selon la revendication 15, caractérisé en ce qu'il comprend au moins un cycle comportant une étape de cotransformation par une cassette selon l'une des revendications 1 à 10, et un marqueur de sélection dominant, suivie d'une étape de purification par sporulation permettant d'éliminer le marqueur de sélection dominant.


**Patentansprüche**

1. Expressionskassette eines Vorläufers von Endothiapepsin in Cryphonectria parasitica, dadurch gekennzeichnet, daß sie den Promotor des Gens, welches das Präproendothiapepsin codiert, oder einen funktionellen Teil dieses Promotors umfaßt, wobei dieser Promotor stromauf einer Sequenz angeordnet ist, die den Vorläufer des Endothiapepsins codiert, das folgende Sequenz (P1) aufweist:

```
Ser Thr Gly Ser Ala Thr Thr Thr Pro Ile Asp Ser Leu Asp Asp Ala Tyr
Ile Thr Pro Val Gln Ile Gly Thr Pro Ala Gln Thr Leu Asn Leu Asp Phe
Asp Thr Gly Ser Ser Asp Leu Trp Val Phe Ser Ser Glu Thr Thr Ala Ser
Glu Val Asp Gly Gln Thr Ile Tyr Thr Pro Ser Lys Ser Thr Thr Ala Lys
Leu Leu Ser Gly Ala Thr Trp Ser Ile Ser Tyr Gly Asp Gly Ser Ser Ser
Ser Gly Asp Val Tyr Thr Asp Thr Val Ser Val Gly Gly Leu Thr Val Thr
Gly Gln Ala Val Glu Ser Ala Lys Lys Val Ser Ser Ser Phe Thr Glu Asp
Ser Thr Ile Asp Gly Leu Leu Gly Leu Ala Phe Ser Thr Leu Asn Thr Val
Ser Pro Thr Gln Gln Lys Thr Phe Phe Asp Asn Ala Lys Ala Ser Leu Asp
Ser Pro Val Phe Thr Ala Asp Leu Gly Tyr His Ala Pro Gly Thr Tyr Asn
Phe Gly Phe Ile Asp Thr Thr Ala Tyr Thr Gly Ser Ile Thr Tyr Thr Ala
Val Ser Thr Lys Gln Gly Phe Trp Glu Trp Thr Ser Thr Gly Tyr Ala Val
Gly Ser Gly Thr Phe Lys Ser Thr Ser Ile Asp Gly Ile Ala Asp Thr Gly
Thr Thr Leu Leu Tyr Leu Pro Ala Thr Val Val Ser Ala Tyr Trp Ala Gln
Val Ser Gly Ala Lys Ser Ser Ser Ser Val Gly Gly Tyr Val Phe Pro Cys
Ser Ala Thr Leu Pro Ser Phe Thr Phe Gly Val Gly Ser Ala Arg Ile Val
Ile Pro Gly Asp Tyr Ile Asp Phe Gly Pro Ile Ser Thr Gly Ser Ser Ser
Cys Phe Gly Gly Ile Gln Ser Ser Ala Gly Ile Gly Ile Asn Ile Phe Gly
Asp Val Ala Leu Lys Ala Ala Phe Val Val Phe Asn Gly Ala Thr Thr Pro
Thr Leu Gly Phe Ala Ser Lys,
```

wobei der Vorläufer des Endothiapepsins Präproendothiapepsin der folgenden Sequenz (P4) ist:

```
Met Ser Ser Pro Leu Lys Asn Ala Leu Val Thr Ala Met Leu Ala Gly Gly

Ala Leu Ser Ser Pro Thr Lys Gln His Val Gly Ile Pro Val Asn Ala Ser

Pro Glu Val Gly Pro Gly Lys Tyr Ser Phe Lys Gln Val Arg Asn Pro Asn

Tyr Lys Phe Asn Gly Pro Leu Ser Val Lys Lys Thr Tyr Leu Lys Tyr Gly

Val Pro Ile Pro Ala Trp Leu Glu Asp Ala Val Gln Asn Ser Thr Ser Gly

Leu Ala Glu Arg Ser Thr Gly Ser Ala Thr Thr Thr Pro Ile Asp Ser Leu

Asp Asp Ala Tyr Ile Thr Pro Val Gln Ile Gly Thr Pro Ala Gln Thr Leu

Asn Leu Asp Phe Asp Thr Gly Ser Ser Asp Leu Trp Val Phe Ser Ser Glu

Thr Thr Ala Ser Glu Val Asp Gly Gln Thr Ile Tyr Thr Pro Ser Lys Ser

Thr Thr Ala Lys Leu Leu Ser Gly Ala Thr Trp Ser Ile Ser Tyr Gly Asp

Gly Ser Ser Ser Ser Gly Asp Val Tyr Thr Asp Thr Val Ser Val Gly Gly

Leu Thr Val Thr Gly Gln Ala Val Glu Ser Ala Lys Lys Val Ser Ser Ser

Phe Thr Glu Asp Ser Thr Ile Asp Gly Leu Leu Gly Leu Ala Phe Ser Thr

Leu Asn Thr Val Ser Pro Thr Gln Gln Lys Thr Phe Phe Asp Asn Ala Lys

Ala Ser Leu Asp Ser Pro Val Phe Thr Ala Asp Leu Gly Tyr His Ala Pro

Gly Thr Tyr Asn Phe Gly Phe Ile Asp Thr Thr Ala Tyr Thr Gly Ser Ile

Thr Tyr Thr Ala Val Ser Thr Lys Gln Gly Phe Trp Glu Trp Thr Ser Thr

Gly Tyr Ala Val Gly Ser Gly Thr Phe Lys Ser Thr Ser Ile Asp Gly Ile

Ala Asp Thr Gly Thr Thr Leu Leu Tyr Leu Pro Ala Thr Val Val Ser Ala

Tyr Trp Ala Gln Val Ser Gly Ala Lys Ser Ser Ser Ser Val Gly Gly Tyr

Val Phe Pro Cys Ser Ala Thr Leu Pro Ser Phe Thr Phe Gly Val Gly Ser

Ala Arg Ile Val Ile Pro Gly Asp Tyr Ile Asp Phe Gly Pro Ile Ser Thr

Gly Ser Ser Ser Cys Phe Gly Gly Ile Gln Ser Ser Ala Gly Ile Gly Ile

Asn Ile Phe Gly Asp Val Ala Leu Lys Ala Ala Phe Val Val Phe Asn Gly

Ala Thr Thr Pro Thr Leu Gly Phe Ala Ser Lys.
```

2. Expressionskassette nach Anspruch 1, dadurch gekennzeichnet, daß die Sequenz, die das Präproendothiapepsin codiert, die folgende Sequenz (N4a) enthält:

```
          ATGTCTT CCCCTCTCAA GAACGCCTTG GTGACCGCCA TGTTGGCTGG
          TGGTGCTCTC AGCTCGCCTA CAAAGCAACA CGTTGGAATT CCCGTCAACG
          CCTCTCCTGA AGTTGGCCCC GGAAAGTACT CGTTCAAGCA AGTCCGGAAC
          CCCAACTACA AGTTCAACGG GCCTCTGTCG GTCAAGAAGA CGTACCTCAA
          GTACGGCGTG CCGATCCCAG CCTGGCTGGA GGATGCTGTC CAGAACTCTA
          CCTCGGGCCT GGCTGAGCGC TCGACCGGTT CTGCGACCAC AACTCCCATC
          GACAGCCTCG ATGATGCTTA CATCACTCCG GTTCAGATCG GCACCCCTGC
          GCAGACTCTG AACCTGGACT TTGACACTGG ATCTTCGGAT CTGTGGGTCT
          TCAGCAGCGA GACTACAGCC AGCGAGGTCG ATGGGCAGAC CATCTACACC
          CCCAGCAAGA GCACCACCGC CAAGCTGCTG TCGGCGCTAC CTGGTCCATC
          TCCTACGGAG ACGGTAGCTC TTCCAGCGGC GATGTCTACA CTGACACCGT
          CTCGGTTGGA GGCCTTACCG TGACGGGCCA GGCTGTCGAG TCGGCCAAGA
          AGGTTTCTTC CAGCTTCACC GAGGACTCGA CCATTGACGG TCTCCTGGGC
          CTGGCCTTCA GCACCCTGAA CACTGTGTCG CCTACCCAGC AAAAGACTTT
          CTTCGACAAT GCGAAGGCGT CCTTGGACTC GCCTGTGTTC ACGGCTGATC
          TTGGCTACCA TGCCCCTGGT ACCTACAACT TCGGCTTCAT CGATACCACT
          GCCTACACGG GCTCCATCAC CTACACCGCT GTCTCGACCA AGCAAGGGTT
          CTGGGAGTGG ACTTCGACCG GCTACGCCGT CGGCTCCGGC ACCTTCAAGT
          CGACTTCCAT CGACGGCATC GCTGACACTG GCACGACCCT CCTGTACCTC
          CCTGCCACCG TCGTGTCGGC CTACTGGGCC CAGGTCTCGG GCGCCAAGTC
          CAGCTCTTCC GTCGGCGGCT ACGTCTTCCC CTGCAGCGCG ACCCTGCCTT
          CCTTCACCTT CGGCGTTGGC TCAGCTCGCA TTGTGATTCC TGGCGACTAC
          ATTGATTTCG GCCCCATCTC CACTGGAAGC TCGTCTTGCT TTGGCGGCAT
          CCAGTCCAGC GCTGGTATCG GCATCAACAT CTTCGGTGAT GTCGCTCTGA
          AGGCTTTGTC GTCTTCAACG GGGCTACAAC TCCCACTCTT GGCTTTGCTT
          CCAAG
```

3. Expressionskassette nach Anspruch 1, dadurch gekennzeichnet, daß die Sequenz, die das Präproendothiapepsin codiert, die Sequenz (N4a) enthält, die von mindestens einem Intron unterbrochen ist.

4. Expressionskassette nach Anspruch 3, dadurch gekennzeichnet, daß die Sequenz, die das Präproendothiapepsin codiert, die folgende Sequenz (N4b) ist:

```
                                         AT GTCTTCCCCT CTCAAGAACG
        CCTTGGTGAC CGCCATGTTG GCTGGTGGTG CTCTCAGCTC GCCTACAAAG
        CAACACGTTG GAATTCCCGT CAACGCCTCT CCTGAAGTTG GCCCCGGAAA
        GTACTCGTTC AAGCAAGGTG AGTAGAGCTG CTTCTGTGTG TTGCAACAGA
        AGACCAACGC AAAAAGAAGA GGTCAAGGCA AGACGGATAT TTTACTGACA
        ATTATACTTT TGAAGTCCGG AACCCCAACT ACAAGTTCAA CGGGCCTCTG
        TCGGTCAAGA AGACGTACCT CAAGTACGGC GTGCCGATCC CAGCCTGGCT
        GGAGGATGCT GTCCAGAACT CTACCTCGGG CCTGGCTGAG CGCTCGACCG
        GTTCTGCGAC CACAACTCCC ATCGACAGCC TCGATGATGC TTACATCACT
        CCGGTTCAGA TCGGCACCCC TGCGCAGACT CTGAACCTGG ACTTTGACAC
        TGGATCTTCG GATCTGTGGG TCTTCAGCAG CGAGACTACA GCCAGCGAGG
        TTGGTCAACC CTCGCCCGCA TTTTATTGCA TACATTTTTA GTTTTTTTGG
        TAATCAGAAT ACTAACATTG GGAATTTCCC AACTGTAGGT CGATGGGCAG
        ACCATCTACA CCCCCAGCAA GAGCACCACC GCCAAGCTGC TGTCGGGCGC
        TACCTGGTCC ATCTCCTACG GAGACGGTAG CTCTTCCAGC GGCGATGTCT
        ACACTGACAC CGTCTCGGTT GGAGGCCTTA CCGTGACGGG CCAGGCTGTC
        GAGTCGGCCA AGAAGGTTTC TTCCAGCTTC ACCGAGGACT CGACCATTGA
        CGGTCTCCTG GGCCTGGCCT TCAGCACCCT GAACACTGTG TCGCCTACCC
        AGCAAAAGAC TTTCTTCGAC AATGCGAAGG CGTCCTTGGA CTCGCCTGTG
        TTCACGGCTG ATCTTGGCTA CCATGCCCGT GAGTGACCCC TCTTGATACA
        TATACTTTTT GATGAATCTT GTTGGAGAAG CATTCCCCAC TAATATGGAA
        ATTGTTTGTA TCTACAGCTG GTACCTACAA CTTCGGCTTC ATCGATACCA
        CTGCCTACAC GGGCTCCATC ACCTACACCG CTGTCTCGAC CAAGCAAGGG
        TTCTGGGAGT GGACTTCGAC CGGCTACGCC GTCGGCTCCG GCACCTTCAA
        GTCGACTTCC ATCGACGGCA TCGCTGACAC TGGCACGACC CTCCTGTACC
        TCCCTGCCAC CGTCGTGTCG GCCTACTGGG CCCAGGTCTC GGGCGCCAAG
        TCCAGCTCTT CCGTCGGCGG CTACGTCTTC CCCTGCAGCG CGACCCTGCC
        TTCCTTCACC TTCGGCGTTG GCTCAGCTCG CATTGTGATT CCTGGCGACT
        ACATTGATTT CGGCCCCATC TCCACTGGAA GCTCGTCTTG CTTTGGCGGC
        ATCCAGTCCA GCGCTGGTAT CGGCATCAAC ATCTTCGGTG ATGTCGCTCT
        GAAGGCCGCC TTTGTCGTCT TCAACGGGGC TACAACTCCC ACTCTTGGCT
        TTGCTTCCAA G
```

**5.** Expressionskassette nach Anspruch 1, dadurch gekennzeichnet, daß der funktionelle Teil des Promotors des Gens, welches das Präproendothiapepsin codiert, das BglII-Scal-Bruchstück des Fragments C ist, wobei das Fragment C ein Teil der genomischen DNA von Cryphonectria parasitica ist, der in dem E.coli-Stamm enthalten ist, der bei CNCM am 31.08.1990 unter der Nummer I998 hinterlegt wurde.

6. Expressionskassette nach Anspruch 1, dadurch gekennzeichnet, daß der funktionelle Teil des Promotors des Gens, welches das Präproendothiapepsin codiert, das BamHI-Scal-Bruchstück des Fragments C ist, wobei das Fragment C ein Teil der genomischen DNA von Cryphonectria parasitica ist, der in dem E.coli-Stamm enthalten ist, der bei CNCM am 31.08.1990 unter der Nummer I-998 hinterlegt wurde.

7. Expressionskassette nach Anspruch 1, dadurch gekennzeichnet, daß der Promotor einen Teil, der die TATA-Box der folgenden Sequenz (N5) trägt:

```
AAGCTTATCC GCCGCCGGCG GGGGAATTCT ATTGAACTTG TTCGAATCAT

TGGTCCGTGG TCTTTTCGTC CATGCGGGCT CCGCTGGCGG ATGAATGACC

TTCTGGCTTC TAGCCTGGCG AAGCGATGTT ACTCTGTTGT CTATACTATA

CGATATGGTC AAGAGAGCAC ATGTGCCGCC AGATGAAGAC ATGTATATAA

AAGGAGTGGC CTCGACGGTT GCTCAACCAT CTTCTGTCTG TCCCAACGCC

ATCGACTCTT CAACTTCTCC TTCGTGTTCC ACCACCATCA CCTTGCTCCA

GACTTAGGAC TTTCAGCAAC CTTCAAAG
```

sowie stromauf der Sequenz (N5) ein Bruchstück X des Fragments C, das zwischen dem 5'-Ende des Fragments A und dem 5'-Ende des Fragments C vorliegt und so ausgewählt ist, daß das Bruchstück X eine Aktivatorregion enthält, aufweist.

8. Expressionskassette nach Anspruch 7, dadurch gekennzeichnet, daß das Bruchstück X des Fragments C das Fragment der folgenden Sequenz ist:

```
GCATGCTTGG CTCTTTAACG TCCTGCCCAT TCAGGGCCTT CAGCCGGCAC

TGGTCCTTCA TCAAGGGGGA CCTCATGACC ATGAACTAAT CTGTGATATC

TGATATATTC TAGAAGGCTT GGCTCCTCAA AGTTTCCAGC TAATGAATCA

GCGGCCCGCC GCCCTTAAAC CGCATCAGGC AAGTCGTTTG GTGTTGCCAG

GCGATGGCGA CAGGAGAGTG GTGTTGATGG GACAAGGGGA GGGAGGCTTA

GCCGACTTCA TCCATAGCAC CCACCTGCTT GGCGCCGATA AGTCTGACGA

TCCGCTTGAG CTGCAAAACG GCTCCTTGAC CTTTGTTTGG TCGACCGAGG

GAAATAGTCT CTTTTTGCGT GATCGTGCGC GCTTCGTATA GCAATAGCAG

CCAGCACCAG CAGGACGGGC CGTTGTCACG GTCACATCGT TCGCAACATG

CCGAGCGTAG GGATGAACGA ATGACTCGAG CCTTGCCTGA CAGTCTGGCA

ATCAATCTAT GGTCACGCAC GATCACAAGC CAATCGCTGT GACTGCGTTA

CTAGCCCAAT AATCCCTTGT TCGATCAGAG TGTTCTACAG ACTTCAAGTG

AGGTTCAC
```

9. Expressionskassette nach einem der Ansprüche 1 bis 5, dadurch gekennzeichnet, daß der Promotor aus einem Gen stammt, das in Cryphonectria parasitica oder in einem anderen fadenförmigen Pilz der Gruppe der Ascomyceten exprimiert wird.

10. Expressionskassette nach Anspruch 9, dadurch gekennzeichnet, daß der Promotor aus dem Gen stammt, das die Glycerinaldehyd-3-phosphatdehydrogenase von Aspergillus nidulans codiert.

11. Cryphonectria-parasitica-Stamm, der Endothiapepsin produziert, dadurch gekennzeichnet, daß er mit einer Expressionskassette nach einem der Ansprüche 1 bis 10 transformiert ist und, bezogen auf einen nicht transformierten Stamm, eine Überproduktion an Endothiapepsin aufweist.

12. Stamm nach Anspruch 11, dadurch gekennzeichnet, daß er keinen dominanten Selektionsmarker enthält.

13. Stamm nach Anspruch 11 oder 12, dadurch gekennzeichnet, daß der Wirtsstamm der Stamm SEBR 103 ist, der bei CNCM am 31.08.1990 unter der Nummer I-997 hinterlegt wurde.

14. Stamm nach Anspruch 13, dadurch gekennzeichnet, daß er, bezogen auf den Stamm SEBR 103, eine Überproduktion an Endothiapepsin aufweist, wobei der Überproduktionsfaktor mindestens gleich 2 ist.

15. Verfahren zur Herstellung von Endothiapepsin, gekennzeichnet durch folgende Stufen: Kultivierung eines Stamms nach einem der Ansprüche 11 bis 14 und anschließende Isolierung und Reinigung dieses Proteins.

16. Verfahren zur Erzeugung eines Stamms nach Anspruch 15, dadurch gekennzeichnet, daß es mindestens einen Zyklus, der eine Stufe der Co-Transformation mit einer Kassette nach einem der Ansprüche 1 bis 10 und einem dominanten Selektionsmarker umfaßt, und eine daran anschließende Stufe der Reinigung durch Sporenbildung umfaßt, durch welche der dominante Selektionsmarker entfernt werden kann.

## Claims

1. Cassette for the expression of an endothiapepsin precursor in <u>Cryphonectria parasitica,</u> characterised in that it comprises the promoter of the gene coding for preproendothiapepsin or a functional portion of said promoter, said promoter being upstream of a sequence coding for the endothiapepsin precursor, endothiapepsin having the following sequence (P1) :

```
Ser Thr Gly Ser Ala Thr Thr Thr Pro Ile Asp Ser Leu Asp Asp Ala Tyr
Ile Thr Pro Val Gln Ile Gly Thr Pro Ala Gln Thr Leu Asn Leu Asp Phe
Asp Thr Gly Ser Ser Asp Leu Trp Val Phe Ser Ser Glu Thr Thr Ala Ser
Glu Val Asp Gly Gln Thr Ile Tyr Thr Pro Ser Lys Ser Thr Thr Ala Lys
Leu Leu Ser Gly Ala Thr Trp Ser Ile Ser Tyr Gly Asp Gly Ser Ser Ser
Ser Gly Asp Val Tyr Thr Asp Thr Val Ser Val Gly Gly Leu Thr Val Thr
Gly Gln Ala Val Glu Ser Ala Lys Lys Val Ser Ser Ser Phe Thr Glu Asp
Ser Thr Ile Asp Gly Leu Leu Gly Leu Ala Phe Ser Thr Leu Asn Thr Val
Ser Pro Thr Gln Gln Lys Thr Phe Phe Asp Asn Ala Lys Ala Ser Leu Asp
Ser Pro Val Phe Thr Ala Asp Leu Gly Tyr His Ala Pro Gly Thr Tyr Asn
Phe Gly Phe Ile Asp Thr Thr Ala Tyr Thr Gly Ser Ile Thr Tyr Thr Ala
Val Ser Thr Lys Gln Gly Phe Trp Glu Trp Thr Ser Thr Gly Tyr Ala Val
Gly Ser Gly Thr Phe Lys Ser Thr Ser Ile Asp Gly Ile Ala Asp Thr Gly
Thr Thr Leu Leu Tyr Leu Pro Ala Thr Val Val Ser Ala Tyr Trp Ala Gln
Val Ser Gly Ala Lys Ser Ser Ser Ser Val Gly Gly Tyr Val Phe Pro Cys
Ser Ala Thr Leu Pro Ser Phe Thr Phe Gly Val Gly Ser Ala Arg Ile Val
Ile Pro Gly Asp Tyr Ile Asp Phe Gly Pro Ile Ser Thr Gly Ser Ser Ser
Cys Phe Gly Gly Ile Gln Ser Ser Ala Gly Ile Gly Ile Asn Ile Phe Gly
Asp Val Ala Leu Lys Ala Ala Phe Val Val Phe Asn Gly Ala Thr Thr Pro
Thr Leu Gly Phe Ala Ser Lys,
```

the precursor of the endothiapepsin being preproendothiapepsin of the following sequence (P4) ;

```
Met Ser Ser Pro Leu Lys Asn Ala Leu Val Thr Ala Met Leu Ala Gly Gly
Ala Leu Ser Ser Pro Thr Lys Gln His Val Gly Ile Pro Val Asn Ala Ser
Pro Glu Val Gly Pro Gly Lys Tyr Ser Phe Lys Gln Val Arg Asn Pro Asn
Tyr Lys Phe Asn Gly Pro Leu Ser Val Lys Lys Thr Tyr Leu Lys Tyr Gly
Val Pro Ile Pro Ala Trp Leu Glu Asp Ala Val Gln Asn Ser Thr Ser Gly
Leu Ala Glu Arg Ser Thr Gly Ser Ala Thr Thr Thr Pro Ile Asp Ser Leu
Asp Asp Ala Tyr Ile Thr Pro Val Gln Ile Gly Thr Pro Ala Gln Thr Leu
Asn Leu Asp Phe Asp Thr Gly Ser Ser Asp Leu Trp Val Phe Ser Ser Glu
Thr Thr Ala Ser Glu Val Asp Gly Gln Thr Ile Tyr Thr Pro Ser Lys Ser
Thr Thr Ala Lys Leu Leu Ser Gly Ala Thr Trp Ser Ile Ser Tyr Gly Asp
Gly Ser Ser Ser Ser Gly Asp Val Tyr Thr Asp Thr Val Ser Val Gly Gly
Leu Thr Val Thr Gly Gln Ala Val Glu Ser Ala Lys Lys Val Ser Ser Ser
Phe Thr Glu Asp Ser Thr Ile Asp Gly Leu Leu Gly Leu Ala Phe Ser Thr
Leu Asn Thr Val Ser Pro Thr Gln Gln Lys Thr Phe Phe Asp Asn Ala Lys
Ala Ser Leu Asp Ser Pro Val Phe Thr Ala Asp Leu Gly Tyr His Ala Pro
Gly Thr Tyr Asn Phe Gly Phe Ile Asp Thr Thr Ala Tyr Thr Gly Ser Ile
Thr Tyr Thr Ala Val Ser Thr Lys Gln Gly Phe Trp Glu Trp Thr Ser Thr
Gly Tyr Ala Val Gly Ser Gly Thr Phe Lys Ser Thr Ser Ile Asp Gly Ile
Ala Asp Thr Gly Thr Thr Leu Leu Tyr Leu Pro Ala Thr Val Val Ser Ala
Tyr Trp Ala Gln Val Ser Gly Ala Lys Ser Ser Ser Ser Val Gly Gly Tyr
Val Phe Pro Cys Ser Ala Thr Leu Pro Ser Phe Thr Phe Gly Val Gly Ser
Ala Arg Ile Val Ile Pro Gly Asp Tyr Ile Asp Phe Gly Pro Ile Ser Thr
Gly Ser Ser Ser Cys Phe Gly Gly Ile Gln Ser Ser Ala Gly Ile Gly Ile
Asn Ile Phe Gly Asp Val Ala Leu Lys Ala Ala Phe Val Val Phe Asn Gly
Ala Thr Thr Pro Thr Leu Gly Phe Ala Ser Lys.
```

2. Cassette according to claim 1, characterised in that the sequence coding for prepoendothiapepsin comprises the following sequence (N4a) :

```
ATGTCTT CCCCTCTCAA GAACGCCTTG GTGACCGCCA TGTTGGCTGG
TGGTGCTCTC AGCTCGCCTA CAAAGCAACA CGTTGGAATT CCCGTCAACG
CCTCTCCTGA AGTTGGCCCC GGAAAGTACT CGTTCAAGCA AGTCCGGAAC
CCCAACTACA AGTTCAACGG GCCTCTGTCG GTCAAGAAGA CGTACCTCAA
GTACGGCGTG CCGATCCCAG CCTGGCTGGA GGATGCTGTC CAGAACTCTA
CCTCGGGCCT GGCTGAGCGC TCGACCGGTT CTGCGACCAC AACTCCCATC
GACAGCCTCG ATGATGCTTA CATCACTCCG GTTCAGATCG GCACCCCTGC
GCAGACTCTG AACCTGGACT TTGACACTGG ATCTTCGGAT CTGTGGGTCT
TCAGCAGCGA GACTACAGCC AGCGAGGTCG ATGGGCAGAC CATCTACACC
CCCAGCAAGA GCACCACCGC CAAGCTGCTG TCGGCGCTAC CTGGTCCATC
TCCTACGGAG ACGGTAGCTC TTCCAGCGGC GATGTCTACA CTGACACCGT
CTCGGTTGGA GGCCTTACCG TGACGGGCCA GGCTGTCGAG TCGGCCAAGA
AGGTTTCTTC CAGCTTCACC GAGGACTCGA CCATTGACGG TCTCCTGGGC
CTGGCCTTCA GCACCCTGAA CACTGTGTCG CCTACCCAGC AAAAGACTTT
CTTCGACAAT GCGAAGGCGT CCTTGGACTC GCCTGTGTTC ACGGCTGATC
TTGGCTACCA TGCCCCTGGT ACCTACAACT TCGGCTTCAT CGATACCACT
GCCTACACGG GCTCCATCAC CTACACCGCT GTCTCGACCA AGCAAGGGTT
CTGGGAGTGG ACTTCGACCG GCTACGCCGT CGGCTCCGGC ACCTTCAAGT
CGACTTCCAT CGACGGCATC GCTGACACTG GCACGACCCT CCTGTACCTC
CCTGCCACCG TCGTGTCGGC CTACTGGGCC CAGGTCTCGG GCGCCAAGTC
CAGCTCTTCC GTCGGCGGCT ACGTCTTCCC CTGCAGCGCG ACCCTGCCTT
CCTTCACCTT CGGCGTTGGC TCAGCTCGCA TTGTGATTCC TGGCGACTAC
ATTGATTTCG GCCCCATCTC CACTGGAAGC TCGTCTTGCT TTGGCGGCAT
CCAGTCCAGC GCTGGTATCG GCATCAACAT CTTCGGTGAT GTCGCTCTGA
AGGCTTTGTC GTCTTCAACG GGGCTACAAC TCCCACTCTT GGCTTTGCTT
CCAAG
```

3. Cassette according to Claim 1, characterised in that the sequence coding for preproendothiapepsin comprises the sequence (N4a) interrupted by at least one intron.

4. Cassette according to Claim 3, characterised in that the sequence coding for preproendothiapepsin is the following sequence (N4b) :

```
                                 AT GTCTTCCCCT CTCAAGAACG
CCTTGGTGAC CGCCATGTTG GCTGGTGGTG CTCTCAGCTC GCCTACAAAG
CAACACGTTG GAATTCCCGT CAACGCCTCT CCTGAAGTTG GCCCCGGAAA
GTACTCGTTC AAGCAAGGTG AGTAGAGCTG CTTCTGTGTG TTGCAACAGA
AGACCAACGC AAAAAGAAGA GGTCAAGCCA AGACGGATAT TTTACTGACA
ATTATACTTT TGAAGTCCGG AACCCCAACT ACAAGTTCAA CGGGCCTCTG
TCGGTCAAGA AGACGTACCT CAAGTACGGC GTGCCGATCC CAGCCTGGCT
GGAGGATGCT GTCCAGAACT CTACCTCGGG CCTGGCTGAG CGCTCGACCG
GTTCTGCGAC CACAACTCCC ATCGACAGCC TCGATGATGC TTACATCACT
CCGGTTCAGA TCGGCACCCC TGCGCAGACT CTGAACCTGG ACTTTGACAC
TGGATCTTCG GATCTGTGGG TCTTCAGCAG CGAGACTACA GCCAGCGAGG
TTGGTCAACC CTCGCCCGCA TTTTATTGCA TACATTTTTA GTTTTTTTGG
TAATCAGAAT ACTAACATTG GGAATTTCCC AACTGTAGGT CGATGGGCAG
ACCATCTACA CCCCCAGCAA GAGCACCACC GCCAAGCTGC TGTCGGGCGC
TACCTGGTCC ATCTCCTACG GAGACGGTAG CTCTTCCAGC GGCGATGTCT
ACACTGACAC CGTCTCGGTT GGAGGCCTTA CCGTGACGGG CCAGGCTGTC
GAGTCGGCCA AGAAGGTTTC TTCCAGCTTC ACCGAGGACT CGACCATTGA
CGGTCTCCTG GGCCTGGCCT TCAGCACCCT GAACACTGTG TCGCCTACCC
AGCAAAAGAC TTTCTTCGAC AATGCGAAGG CGTCCTTGGA CTCGCCTGTG
TTCACGGCTG ATCTTGGCTA CCATGCCCGT GAGTGACCCC TCTTGATACA
TATACTTTTT GATGAATCTT GTTGGAGAAG CATTCCCCAC TAATATGGAA
ATTGTTTGTA TCTACAGCTG GTACCTACAA CTTCGGCTTC ATCGATACCA
CTGCCTACAC GGGCTCCATC ACCTACACCG CTGTCTCGAC CAAGCAAGGG
TTCTGGGAGT GGACTTCGAC CGGCTACGCC GTCGGCTCCG GCACCTTCAA
GTCGACTTCC ATCGACGGCA TCGCTGACAC TGGCACGACC CTCCTGTACC
TCCCTGCCAC CGTCGTGTCG GCCTACTGGG CCCAGGTCTC GGGCGCCAAG
TCCAGCTCTT CCGTCGGCGG CTACGTCTTC CCCTGCAGCG CGACCCTGCC
TTCCTTCACC TTCGGCGTTG GCTCAGCTCG CATTGTGATT CCTGGCGACT
ACATTGATTT CGGCCCCATC TCCACTGGAA GCTCGTCTTG CTTTGGCGGC
ATCCAGTCCA GCGCTGGTAT CGGCATCAAC ATCTTCGGTG ATGTCGCTCT
GAAGGCCGCC TTTGTCGTCT TCAACGGGGC TACAACTCCC ACTCTTGGCT
TTGCTTCCAA G
```

5.  Cassette according to Claim 1, characterised in that the functional portion of the promoter of the gene coding for preproendothiapepsin is the BglII-ScaI segment of the fragment C, said fragment C being a portion of the genomic DNA of <u>Cryphonectria parasitica</u> contained in the <u>E. Coli</u> strain deposited with the CNCM on 31.08.1990 under No. I-998.

**6.** Cassette according to Claim 1, characterised in that the functional portion of the promoter of the gene coding for preproendothiapepsin is the BamHI-Scal segment of the fragment C, said fragment C being a portion of the genomic DNA of <u>Cryphonectria parasitica</u> contained in the <u>E, Coli</u> strain deposited with the CNCM on 31.08.1990 under No. I-998.

**7.** Cassette according to Claim 1, characterised in that the promoter comprises a portion carrying the TATA box of the following sequence (N5) :

```
AAGCTTATCC GCCGCCGGCG GGGGAATTCT ATTGAACTTG TTCGAATCAT

TGGTCCGTGG TCTTTTCGTC CATGCGGGCT CCGCTGGCGG ATGAATGACC

TTCTGGCTTC TAGCCTGGCG AAGCGATGTT ACTCTGTTGT CTATACTATA

CGATATGGTC AAGAGAGCAC ATGTGCCGCC AGATGAAGAC ATGTATATAA

AAGGAGTGGC CTCGACGGTT GCTCAACCAT CTTCTGTCTG TCCCAACGCC

ATCGACTCTT CAACTTCTCC TTCGTGTTCC ACCACCATCA CCTTGCTCCA

GACTTAGGAC TTTCAGCAAC CTTCAAAG
```

and, upstream of the sequence (N5), a segment X of the fragment C bounded by the 5' end of the fragment A and the 5' end of the fragment C, chosen so that the segment X contains an activator region.

**8.** Cassette according to Claim 7, characterised in that the segment X of the fragment C is the fragment of the following sequence :

```
GCATGCTTGG CTCTTTAACG TCCTGCCCAT TCAGGGCCTT CAGCCGGCAC

TGGTCCTTCA TCAAGGGGGA CCTCATGACC ATGAACTAAT CTGTGATATC

TGATATATTC TAGAAGGCTT GGCTCCTCAA AGTTTCCAGC TAATGAATCA

GCGGCCCGCC GCCCTTAAAC CGCATCAGGC AAGTCGTTTG GTGTTGCCAG

GCGATGGCGA CAGGAGAGTG GTGTTGATGG GACAAGGGGA GGGAGGCTTA

GCCGACTTCA TCCATAGCAC CCACCTGCTT GGCGCCGATA AGTCTGACGA

TCCGCTTGAG CTGCAAAACG GCTCCTTGAC CTTTGTTTGG TCGACCGAGG

GAAATAGTCT CTTTTTGCGT GATCGTGCGC GCTTCGTATA GCAATAGCAG

CCAGCACCAG CAGGACGGGC CGTTGTCACG GTCACATCGT TCGCAACATG

CCGAGCGTAG GGATGAACGA ATGACTCGAG CCTTGCCTGA CAGTCTGGCA

ATCAATCTAT GGTCACGCAC GATCACAAGC CAATCGCTGT GACTGCGTTA

CTAGCCCAAT AATCCCTTGT TCGATCAGAG TGTTCTACAG ACTTCAAGTG

AGGTTCAC
```

**9.** Cassette according to one of Claims 1 to 5, characterised in that the promoter originates from a gene expressed in <u>Cryphonectria parasitica</u> or in another filamentous fungus of the Ascomycetes group.

**10.** Cassette according to Claim 9, characterised in that the promoter originates from the gene coding for glyceraldehyde-3-phosphate dehydrogenase of <u>Aspergillus nidulans</u>.

**11.** <u>Cryphonectria parasitica</u> strain productive of endothiapepsin, characterised in that it is transformed with a cassette according to one of Claims 1 to 10 and overproduces endothiapepsin compared with the untransformed strain.

12. Strain according to Claim 1, characterised in that it is devoid of a dominant selection marker.

13. Strain according to one of Claims 11 and 12, characterised in that the host strain is the strain SEBR103 deposited with the CNCM on 31.08.1990 under No. I-997.

14. Strain according to Claim 13, characterised in that it overproduces endothiapepsin compared with the strain SEBR 103, with an overproduction factor equal to at least 2.

15. Process for preparing endothiapepsin, characterised in that it comprises a step of culturing of a strain according to one of Claims 11 to 14, followed by a step of isolation and purification of this protein.

16. Process for obtaining a strain according to Claim 15, characterised in that it comprises at least one cycle entailing a step of cotransformation with a cassette according to one of Claims 1 to 10, and a dominant selection marker, followed by a step of purification by sporulation enabling the dominant selection marker to be removed.

SerThrGlySerAlaThrThrThrProIleAsp

SerLeuAspAspAlaTyrIleThrProValGlnIleGlyThrProAlaGlnThrLeuAsn

LeuAspPheAspThrGlySerSerAspLeuTrpValPheSerSerGluThrThrAlaSer

GluValAspGlyGlnThrIleTyrThrProSerLysSerThrThrAlaLysLeuLeuSer

GlyAlaThrTrpSerIleSerTyrGlyAspGlySerSerSerSerGlyAspValTyrThr

AspThrValSerValGlyGlyLeuThrValThrGlyGlnAlaValGluSerAlaLysLys

ValSerSerSerPheThrGluAspSerThrIleAspGlyLeuLeuGlyLeuAlaPheSer

ThrLeuAsnThrValSerProThrGlnGlnLysThrPhePheAspAsnAlaLysAlaSer

LeuAspSerProValPheThrAlaAspLeuGlyTyrHisAlaProGlyThrTyrAsnPhe

GlyPheIleAspThrThrAlaTyrThrGlySerIleThrTyrThrAlaValSerThrLys

GlnGlyPheTrpGluTrpThrSerThrGlyTyrAlaValGlySerGlyThrPheLysSer

ThrSerIleAspGlyIleAlaAspThrGlyThrThrLeuLeuTyrLeuProAlaThrVal

ValSerAlaTyrTrpAlaGlnValSerGlyAlaLysSerSerSerSerValGlyGlyTyr

ValPheProCysSerAlaThrLeuProSerPheThrPheGlyValGlySerAlaArgIle

ValIleProGlyAspTyrIleAspPheGlyProIleSerThrGlySerSerSerCysPhe

GlyGlyIleGlnSerSerAlaGlyIleGlyIleAsnIlePheGlyAspValAlaLeuLys

AlaAlaPheValValPheAsnGlyAlaThrThrProThrLeuGlyPheAlaSerLys

# FIG.1 :   séquence d'acides aminés de l'endothiapepsine

```
     AAGCTTATCCGCCGCCGGCGGGGGAATTCTATTGAACTTGTTCGAATCATTGGTCCGTGG
  1  ---------+---------+---------+---------+---------+---------+  60

     TCTTTTCGTCCATGCGGGCTCCGCTGGCGGATGAATGACCTTCTGGCTTCTAGCCTGGCG
 61  ---------+---------+---------+---------+---------+---------+  120

     AAGCGATGTTACTCTGTTGTCTATACTATCGATATGGTCAAGAGAGCACATGTGCCGCC
121  ---------+---------+---------+---------+---------+---------+  180

     AGATGAAGACATGTATATAAAAGGAGTGGCCTCGACGGTTGCTCAACCATCTTCTGTCTG
181  ---------+---------+---------+---------+---------+---------+  240

     TCCCAACGCCATCGACTCTTCAACTTCTCCTTCGTGTTCCACCACCATCACCTTGCTCCA
241  ---------+---------+---------+---------+---------+---------+  300
                                                        BstEII
     GACTTAGGACTTTCAGCAACCTTCAAAGATGTCTTCCCCTCTCAAGAACGCCTTGGTGAC
301  ---------+---------+---------+---------+---------+----J----+  360
                               MetSerSerProLeuLysAsnAlaLeuValTh

     CGCCATGTTGGCTGGTGGTGCTCTCAGCTCGCCTACAAAGCAACACGTTGGAATTCCCGT
361  ---------+---------+---------+---------+---------+---------+  420
     rAlaMetLeuAlaGlyGlyAlaLeuSerSerProThrLysGlnHisValGlyIleProVa

     CAACGCCTCTCCTGAAGTTGGCCCCGGAAAGTACTCGTTCAAGCAAGGTGAGTAGAGCTG
421  ---------+---------+---------+---------+---------+---------+  480
     lAsnAlaSerProGluValGlyProGlyLysTyrSerPheLysGlnV

     CTTCTGTGTGTTGCAACAGAAGACCAACGCAAAAAGAAGAGGTCAAGGCAAGACGGATAT
481  ---------+---------+---------+---------+---------+---------+  540

     TTTACTGACAATTATACTTTTGAAGTCCGGAACCCCAACTACAAGTTCAACGGGCCTCTG
541  ---------+---------+---------+---------+---------+---------+  600
                         alArgAsnProAsnTyrLysPheAsnGlyProLeu

     TCGGTCAAGAAGACGTACCTCAAGTACGGCGTGCCGATCCCAGCCTGGCTGGAGGATGCT
601  ---------+---------+---------+---------+---------+---------+  660
     SerValLysLysThrTyrLeuLysTyrGlyValProIleProAlaTrpLeuGluAspAla

     GTCCAGAACTCTACCTCGGGCCTGGCTGAGCGCTCGACCGGTTCTGCGACCACAACTCCC
661  ---------+---------+---------+---------+---------+---------+  720
     ValGlnAsnSerThrSerGlyLeuAlaGluArgSerThrGlySerAlaThrThrThrPro

     ATCGACAGCCTCGATGATGCTTACATCACTCCGGTTCAGATCGGCACCCCTGCGCAGACT
721  ---------+---------+---------+---------+---------+---------+  780
     IleAspSerLeuAspAspAlaTyrIleThrProValGlnIleGlyThrProAlaGlnThr

     CTGAACCTGGACTTTGACACTGGATCTTCGGATCTGTGGGTCTTCAGCAGCGAGACTACA
781  ---------+---------+---------+---------+---------+---------+  840
     LeuAsnLeuAspPheAspThrGlySerSerAspLeuTrpValPheSerSerGluThrThr

     GCCAGCGAGGTTGGTCAACCCTCGCCCGCATTTTATTGCATACATTTTTAGTTTTTTTGG
841  ---------+---------+---------+---------+---------+---------+  900
     AlaSerGlu

     TAATCAGAATACTAACATTGGGAATTTCCCAACTGTAGGTCGATGGGCAGACCATCTACA
901  ---------+---------+---------+---------+---------+---------+  960
                                          ValAspGlyGlnThrIleTyrT

     CCCCCAGCAAGAGCACCACCGCCAAGCTGCTGTCGGGCGCTACCTGGTCCATCTCCTACG
961  ---------+---------+---------+---------+---------+---------+  1020
     hrProSerLysSerThrThrAlaLysLeuLeuSerGlyAlaThrTrpSerIleSerTyrG

     GAGACGGTAGCTCTTCCAGCGGCGATGTCTACACTGACACCGTCTCGGTTGGAGGCCTTA
1021 ---------+---------+---------+---------+---------+---------+  1080
     lyAspGlySerSerSerSerGlyAspValTyrThrAspThrValSerValGlyGlyLeuT
```

## FIG.2

```
      CCGTGACGGGCCAGGCTGTCGAGTCGGCCAAGAAGGTTTCTTCCAGCTTCACCGAGGACT
1081  --------+---------+---------+---------+---------+---------+ 1140
      hrValThrGlyGlnAlaValGluSerAlaLysLysValSerSerSerPheThrGluAspS

      CGACCATTGACGGTCTCCTGGGCCTGGCCTTCAGCACCCTGAACACTGTGTCGCCTACCC
1141  --------+---------+---------+---------+---------+---------+ 1200
      erThrIleAspGlyLeuLeuGlyLeuAlaPheSerThrLeuAsnThrValSerProThrG

      AGCAAAAGACTTTCTTCGACAATGCGAAGGCGTCCTTGGACTCGCCTGTGTTCACGGCTG
1201  --------+---------+---------+---------+---------+---------+ 1260
      lnGlnLysThrPhePheAspAsnAlaLysAlaSerLeuAspSerProValPheThrAlaA

      ATCTTGGCTACCATGCCCGTGAGTGACCCCTCTTGATACATATACTTTTTGATGAATCTT
1261  --------+---------+---------+---------+---------+---------+ 1320
      spLeuGlyTyrHisAlaP

      GTTGGAGAAGCATTCCCCACTAATATGGAAATTGTTTGTATCTACAGCTGGTACCTACAA
1321  --------+---------+---------+---------+---------+---------+ 1380
                                                      roGlyThrTyrAs

      CTTCGGCTTCATCGATACCACTGCCTACACGGGCTCCATCACCTACACCGCTGTCTCGAC
1381  --------+---------+---------+---------+---------+---------+ 1440
      nPheGlyPheIleAspThrThrAlaTyrThrGlySerIleThrTyrThrAlaValSerTh

      CAAGCAAGGGTTCTGGGAGTGGACTTCGACCGGCTACGCCGTCGGCTCCGGCACCTTCAA
1441  --------+---------+---------+---------+---------+---------+ 1500
      rLysGlnGlyPheTrpGluTrpThrSerThrGlyTyrAlaValGlySerGlyThrPheLy

      GTCGACTTCCATCGACGGCATCGCTGACACTGGCACGACCCTCCTGTACCTCCCTGCCAC
1501  --------+---------+---------+---------+---------+---------+ 1560
      sSerThrSerIleAspGlyIleAlaAspThrGlyThrThrLeuLeuTyrLeuProAlaTh

      CGTCGTGTCGGCCTACTGGGCCCAGGTCTCGGGCGCCAAGTCCAGCTCTTCCGTCGGCGG
1561  --------+---------+---------+---------+---------+---------+ 1620
      rValValSerAlaTyrTrpAlaGlnValSerGlyAlaLysSerSerSerSerValGlyGl

      CTACGTCTTCCCCTGCAGCGCGACCCTGCCTTCCTTCACCTTCGGCGTTGGCTCAGCTCG
1621  --------+---------+---------+---------+---------+---------+ 1680
      yTyrValPheProCysSerAlaThrLeuProSerPheThrPheGlyValGlySerAlaAr

      CATTGTGATTCCTGGCGACTACATTGATTTCGGCCCCATCTCCACTGGAAGCTCGTCTTG
1681  --------+---------+---------+---------+---------+---------+ 1740
      gIleValIleProGlyAspTyrIleAspPheGlyProIleSerThrGlySerSerSerCy

      CTTTGGCGGCATCCAGTCCAGCGCTGGTATCGGCATCAACATCTTCGGTGATGTCGCTCT
1741  --------+---------+---------+---------+---------+---------+ 1800
      sPheGlyGlyIleGlnSerSerAlaGlyIleGlyIleAsnIlePheGlyAspValAlaLe

      GAAGGCCGCCTTTGTCGTCTTCAACGGGGCTACAACTCCCACTCTTGGCTTTGCTTCCAA
1801  --------+---------+---------+---------+---------+---------+ 1860
      uLysAlaAlaPheValValPheAsnGlyAlaThrThrProThrLeuGlyPheAlaSerLy

      GTAAATTAAGGGCCCTCGCTCCTCCATAGCTGCGATAAATGAGGCAGGCTCAAGTGGAAA
1861  --------+---------+---------+---------+---------+---------+ 1920
      s

      GTCTTGTTGGGTAGGCGTGGATACGTATTGTCTACTTAATTAATTAATGCCAAAGCAGAC
1921  --------+---------+---------+---------+---------+---------+ 1980

      CTGAAGATAGCTTTAGTAATTAATTCAATAAGCACATGGAGATCCTTCGGATCAATATGC
1981  --------+---------+---------+---------+---------+---------+ 2040

      TAACTCGGTCTTCATCTCTAAACGAATGTGTTGTTGCTTGAGTTTCAGATGAATTTCCTG
2041  --------+---------+---------+---------+---------+---------+ 2100

      CTGTGATATCCCTCTAAGGTGTAGTATGGACAGTAAGCTT
2101  --------+---------+---------+---------+ 2140
```

# FIG. 2 (suite)

MetSerSerProLeuLysAsnAlaLeuValThrAlaMetLeuAlaGlyGlyAlaLeuSer

SerProThrLysGlnHisValGlyIleProValAsnAlaSerProGluValGlyProGly

LysTyrSerPheLysGlnValArgAsnProAsnTyrLysPheAsnGlyProLeuSerVal

LysLysThrTyrLeuLysTyrGlyValProIleProAlaTrpLeuGluAspAlaValGln

AsnSerThrSerGlyLeuAlaGluArgSerThrGlySerAlaThrThrThrProIleAsp

SerLeuAspAspAlaTyrIleThrProValGlnIleGlyThrProAlaGlnThrLeuAsn

LeuAspPheAspThrGlySerSerAspLeuTrpValPheSerSerGluThrThrAlaSer

GluValAspGlyGlnThrIleTyrThrProSerLysSerThrThrAlaLysLeuLeuSer

GlyAlaThrTrpSerIleSerTyrGlyAspGlySerSerSerSerGlyAspValTyrThr

AspThrValSerValGlyGlyLeuThrValThrGlyGlnAlaValGluSerAlaLysLys

ValSerSerSerPheThrGluAspSerThrIleAspGlyLeuLeuGlyLeuAlaPheSer

ThrLeuAsnThrValSerProThrGlnGlnLysThrPhePheAspAsnAlaLysAlaSer

LeuAspSerProValPheThrAlaAspLeuGlyTyrHisAlaProGlyThrTyrAsnPhe

GlyPheIleAspThrThrAlaTyrThrGlySerIleThrTyrThrAlaValSerThrLys

GlnGlyPheTrpGluTrpThrSerThrGlyTyrAlaValGlySerGlyThrPheLysSer

ThrSerIleAspGlyIleAlaAspThrGlyThrThrLeuLeuTyrLeuProAlaThrVal

ValSerAlaTyrTrpAlaGlnValSerGlyAlaLysSerSerSerSerValGlyGlyTyr

ValPheProCysSerAlaThrLeuProSerPheThrPheGlyValGlySerAlaArgIle

ValIleProGlyAspTyrIleAspPheGlyProIleSerThrGlySerSerSerCysPhe

GlyGlyIleGlnSerSerAlaGlyIleGlyIleAsnIlePheGlyAspValAlaLeuLys

AlaAlaPheValValPheAsnGlyAlaThrThrProThrLeuGlyPheAlaSerLys

# FIG.3 : Séquence d'acides aminés de la préproendothiapepsine

FIG 4 : Carte de restriction du Fragment C

Sp: Sph I
B : BamHI
C : SacI
G : BglII
H : HindIII
P : PstI
S : ScaI

L : codon d'initiation présumé

EP 0 475 842 B1

EP 0 475 842 B1

```
   1  ATGTCTTCCC CTCTCAAGAA CGCCTTGGTG ACCGCCATGT TGGCTGGTGG
  51  TGCTCTCAGC TCGCCTACAA AGCAACACGT TGGAATTCCC GTCAACGCCT
 101  CTCCTGAAGT TGGCCCCGGA AAGTACTCGT TCAAGCAAGG TGAGTAGAGC
 151  TGCTTCTGTG TGTTGCAACA GAAGACCAAC GCAAAAAGAA GAGGTCAAGG
 201  CAAGACGGAT ATTTTACTGA CAATTATACT TTTGAAGTCC GGAACCCCAA
 251  CTACAAGTTC AACGGGCCTC TGTCGGTCAA GAAGACGTAC CTCAAGTACG
 301  GCGTGCCGAT CCCAGCCTGG CTGGAGGATG CTGTCCAGAA CTCTACCTCG
 351  GGCCTGGCTG AGCGCTCGAC CGGTTCTGCG ACCACAACTC CCATCGACAG
 401  CCTCGATGAT GCTTACATCA CTCCGGTTCA GATCGGCACC CCTGCGCAGA
 451  CTCTGAACCT GGACTTTGAC ACTGGATCTT CGGATCTGTG GGTCTTCAGC
 501  AGCGAGACTA CAGCCAGCGA GGTTGGTCAA CCCTCGCCCG CATTTTATTG
 551  CATACATTTT TAGTTTTTTT GGTAATCAGA ATACTAACAT TGGGAATTTC
 601  CCAACTGTAG GTCGATGGGC AGACCATCTA CACCCCCAGC AAGAGCACCA
 651  CCGCCAAGCT GCTGTCGGGC GCTACCTGGT CCATCTCCTA CGGAGACGGT
 701  AGCTCTTCCA GCGGCGATGT CTACACTGAC ACCGTCTCGG TTGGAGGCCT
 751  TACCGTGACG GGCCAGGCTG TCGAGTCGGC CAAGAAGGTT TCTTCCAGCT
 801  TCACCGAGGA CTCGACCATT GACGGTCTCC TGGGCCTGGC CTTCAGCACC
 851  CTGAACACTG TGTCGCCTAC CCAGCAAAAG ACTTTCTTCG ACAATGCGAA
 901  GGCGTCCTTG GACTCGCCTG TGTTCACGGC TGATCTTGGC TACCATGCCC
 951  GTGAGTGACC CCTCTTGATA CATATACTTT TTGATGAATC TTGTTGGAGA
1001  AGCATTCCCC ACTAATATGG AAATTGTTTG TATCTACAGC TGGTACCTAC
1051  AACTTCGGCT TCATCGATAC CACTGCCTAC ACGGGCTCCA TCACCTACAC
1101  CGCTGTCTCG ACCAAGCAAG GGTTCTGGGA GTGGACTTCG ACCGGCTACG
1151  CCGTCGGCTC CGGCACCTTC AAGTCGACTT CCATCGACGG CATCGCTGAC
1201  ACTGGCACGA CCCTCCTGTA CCTCCCTGCC ACCGTCGTGT CGGCCTACTG
1251  GGCCCAGGTC TCGGGCGCCA AGTCCAGCTC TTCCGTCGGC GGCTACGTCT
1301  TCCCCTGCAG CGCGACCCTG CCTTCCTTCA CCTTCGGCGT TGGCTCAGCT
1351  CGCATTGTGA TTCCTGGCGA CTACATTGAT TTCGGCCCCA TCTCCACTGG
1401  AAGCTCGTCT TGCTTTGGCG GCATCCAGTC CAGCGCTGGT ATCGGCATCA
1451  ACATCTTCGG TGATGTCGCT CTGAAGGCCG CCTTTGTCGT CTTCAACGGG
1501  GCTACAACTC CCACTCTTGG CTTTGCTTCC AAG
```

FIG.5 : Séquence de l'ADN génomique codant pour la préproendothiapepsine interrompue par trois introns.

66

# FIG.6

Plasmide  p 163,1

# FIG.7

*Plasmide p 160*

FIG.8

Plasmide p 373,2

# FIG.9

*Plasmide p 462*

# FIG.10

*Plasmide p 466*

```
ATGTCTT CCCCTCTCAA GAACGCCTTG GTGACCGCCA TGTTGGCTGG

TGGTGCTCTC AGCTCGCCTA CAAAGCAACA CGTTGGAATT CCCGTCAACG

CCTCTCCTGA AGTTGGCCCC GGAAAGTACT CGTTCAAGCA AGTCCGGAAC

CCCAACTACA AGTTCAACGG GCCTCTGTCG GTCAAGAAGA CGTACCTCAA

GTACGGCGTG CCGATCCCAG CCTGGCTGGA GGATGCTGTC CAGAACTCTA

CCTCGGGCCT GGCTGAGCGC TCGACCGGTT CTGCGACCAC AACTCCCATC

GACAGCCTCG ATGATGCTTA CATCACTCCG GTTCAGATCG GCACCCCTGC

GCAGACTCTG AACCTGGACT TTGACACTGG ATCTTCGGAT CTGTGGGTCT

TCAGCAGCGA GACTACAGCC AGCGAGGTCG ATGGGCAGAC CATCTACACC

CCCAGCAAGA GCACCACCGC CAAGCTGCTG TCGGCGCTAC CTGGTCCATC

TCCTACGGAG ACGGTAGCTC TTCCAGCGGC GATGTCTACA CTGACACCGT

CTCGGTTGGA GGCCTTACCG TGACGGGCCA GGCTGTCGAG TCGGCCAAGA

AGGTTTCTTC CAGCTTCACC GAGGACTCGA CCATTGACGG TCTCCTGGGC

CTGGCCTTCA GCACCCTGAA CACTGTGTCG CCTACCCAGC AAAAGACTTT

CTTCGACAAT GCGAAGGCGT CCTTGGACTC GCCTGTGTTC ACGGCTGATC

TTGGCTACCA TGCCCCTGGT ACCTACAACT TCGGCTTCAT CGATACCACT

GCCTACACGG GCTCCATCAC CTACACCGCT GTCTCGACCA AGCAAGGGTT

CTGGGAGTGG ACTTCGACCG GCTACGCCGT CGGCTCCGGC ACCTTCAAGT

CGACTTCCAT CGACGGCATC GCTGACACTG GCACGACCCT CCTGTACCTC

CCTGCCACCG TCGTGTCGGC CTACTGGGCC CAGGTCTCGG GCGCCAAGTC

CAGCTCTTCC GTCGGCGGCT ACGTCTTCCC CTGCAGCGCG ACCCTGCCTT

CCTTCACCTT CGGCGTTGGC TCAGCTCGCA TTGTGATTCC TGGCGACTAC

ATTGATTTCG GCCCCATCTC CACTGGAAGC TCGTCTTGCT TTGGCGGCAT

CCAGTCCAGC GCTGGTATCG GCATCAACAT CTTCGGTGAT GTCGCTCTGA

AGGCTTTGTC GTCTTCAACG GGGCTACAAC TCCCACTCTT GGCTTTGCTT

CCAAG
```

# FIG.11 : Séquence de l'ADN complémentaire codant pour la préproendothiapepsine

GCATGCTTGG CTCTTTAACG TCCTGCCCAT TCAGGGCCTT CAGCCGGCAC

TGGTCCTTCA TCAAGGGGGA CCTCATGACC ATGAACTAAT CTGTGATATC

TGATATATTC TAGAAGGCTT GGCTCCTCAA AGTTTCCAGC TAATGAATCA

GCGGCCCGCC GCCCTTAAAC CGCATCAGGC AAGTCGTTTG GTGTTGCCAG

GCGATGGCGA CAGGAGAGTG GTGTTGATGG GACAAGGGGA GGGAGGCTTA

GCCGACTTCA TCCATAGCAC CCACCTGCTT GGCGCCGATA AGTCTGACGA

TCCGCTTGAG CTGCAAAACG GCTCCTTGAC CTTTGTTTGG TCGACCGAGG

GAAATAGTCT CTTTTTGCGT GATCGTGCGC GCTTCGTATA GCAATAGCAG

CCAGCACCAG CAGGACGGGC CGTTGTCACG GTCACATCGT TCGCAACATG

CCGAGCGTAG GGATGAACGA ATGACTCGAG CCTTGCCTGA CAGTCTGGCA

ATCAATCTAT GGTCACGCAC GATCACAAGC CAATCGCTGT GACTGCGTTA

CTAGCCCAAT AATCCCTTGT TCGATCAGAG TGTTCTACAG ACTTCAAGTG

AGGTTCAC

# FIG.12 : Séquence du segment X du fragment F